# EUROPÄISCHE PATENTSCHRIFT

(11) **EP 3 129 394 B1**
(45) Veröffentlichungstag und Bekanntmachung des Hinweises auf die Patenterteilung: **08.01.2020**
(21) Anmeldenummer: 15738237.5
(22) Anmeldetag: 10.04.2015
(51) Int. Cl.: C07K 14/415, C12N 15/82

(54) **TONOPLASTIDÄRE PROTONEN/ZUCKER-ANTIPORTER-PROTEINE UND DEREN VERWENDUNG ZUR ERHÖHUNG DER SACCHAROSEKONZENTRATION EINES SACCHAROSESPEICHERORGANS VON PFLANZEN**
TONOPLAST PROTON/SUGAR ANTIPORTER PROTEINS AND THE USE THEREOF TO INCREASE THE SACCHAROSE CONCENTRATION IN A SACCHAROSE STORAGE ORGAN OF PLANTS
PROTÉINES TONOPLASTIQUES ANTIPORTEURS SUCRE-PROTONS, ET LEUR UTILISATIONS POUR AUGMENTER LA CONCENTRATION DE SACCHAROSE D'UN ORGANE DE STOCKAGE DE SACCHAROSE CHEZ LES VÉGÉTAUX

(30) Priorität: 11.04.2014 DE 102014005337
(43) Veröffentlichungstag der Anmeldung: 15.02.2017
(73) Patentinhaber: KWS SAAT SE & Co. KGaA, 37574 Einbeck (DE); Südzucker AG, 68165 Mannheim (DE)
(72) Erfinder: KOCH, Wolfgang, 37574 Einbeck (DE); SAUER, Norbert, 91058 Erlangen (DE); WIRSCHING, Petra, 90765 Fürth (DE); POMMERRENIG, Benjamin, 38820 Halberstadt (DE); NEUHAUS, Ekkehard, 67659 Kaiserslautern (DE); JUNG, Benjamin, 67693 Fischbach (DE); FLÜGGE, Ulf-Ingo, 50674 Köln (DE); LUDEWIG, Frank, 91052 Erlangen (DE); WÖSTEFELD, Nicole, 52353 Düren (DE); MARTEN, Irene, 97082 Würzburg (DE); HEDRICH, Rainer, 97082 Würzburg (DE); SCHULZ, Alexander, 97262 Hausen bei Würzburg (DE)
(86) Internationale Anmeldenummer: PCT/DE2015/000170
(87) Internationale Veröffentlichungsnummer: WO 2015/154741

(56) Entgegenhaltungen:
- WO-A1-2011/120549
- KR-A- 20110 085 729
- KR-A- 20110 085 732
- DATABASE EMBL [Online] 17. Oktober 2012 (2012-10-17), "TSA: Beta vulgaris Locus_1975_Transcript_18/21_Confidence_0.2 69_Length_3084 mRNA sequence.", XP002743849, gefunden im EBI accession no. EM_TSA:JP489061 Database accession no. JP489061
- ALEXANDER SCHULZ ET AL: "Proton-driven sucrose symport and antiport are provided by the vacuolar transporters SUC4 and TMT1/2", THE PLANT JOURNAL, Bd. 68, Nr. 1, 27. Oktober 2011 (2011-10-27), Seiten 129-136, XP055210435, ISSN: 0960-7412, DOI: 10.1111/j.1365-313X.2011.04672.x
- WINGENTER KARINA ET AL: "Increased Activity of the Vacuolar Monosaccharide Transporter TMT1 Alters Cellular Sugar Partitioning, Sugar Signaling, and Seed Yield in Arabidopsis", PLANT PHYSIOLOGY, Bd. 154, Nr. 2, 1. Oktober 2010 (2010-10-01), Seiten 665-677, XP009145890, AMERICAN SOCIETY OF PLANT PHYSIOLOGISTS, ROCKVILLE, MD, US ISSN: 0032-0889, DOI: 10.1104/PP.110.162040
- ENDLER ANNE ET AL: "Identification of a vacuolar sucrose transporter in barley and arabidopsis mesophyll cells by a tonoplast proteomic approach", PLANT PHYSIOLOGY (ROCKVILLE), Bd. 141, Nr. 1, Mai 2006 (2006-05), Seiten 196-207, XP002743850, ISSN: 0032-0889
- KUHN C ET AL: "Sucrose transporters of higher plants", CURRENT OPINION IN PLANT BIOLOGY, Bd. 13, Nr. 3, 1. Juni 2010 (2010-06-01), Seiten 287-297, XP027081073, QUADRANT SUBSCRIPTION SERVICES, GB ISSN: 1369-5266, DOI: 10.1016/J.PBI.2010.02.001 [gefunden am 2010-03-18]
- LUDEWIG FRANK ET AL: "Role of metabolite transporters in source-sink carbon allocation", FRONTIERS IN PLANT SCIENCE, Bd. 4, 231, 2. Juli 2013 (2013-07-02), Seiten 1-16, XP002743851, DOI: 10.3389/fpls.2013.00231
- DATABASE Geneseq [Online] 5 March 2009 (2009-03-05), "Arabidopsis thaliana protein, SEQ ID 390.", retrieved from EBI accession no. GSP:AUZ31616 Database accession no. AUZ31616 -& WO 2008/070179 A2 (MONSANTO TECHNOLOGY LLC [US]; ABAD MARK [US]; CHITTOOR JAISHREE [US];) 12 June 2008 (2008-06-12)

## Beschreibung

Die vorliegende Erfindung liegt auf dem Gebiet der industriellen Zuckergewinnung aus Nutzpflanzen und betrifft die Steigerung des Saccharoseertrags beim landwirtschaftlichen Anbau von Nutzpflanzen. Insbesondere betrifft die Erfindung tonoplastidäre Protonen/Zucker-Antiporter-Proteine, insbesondere tonoplastidäre Protonen/Saccharose-Antiporter-Proteine, und die sie kodierenden Nukleinsäuren sowie deren Verwendung zur Erhöhung der Saccharosekonzentration eines Saccharosespeicherorgans von Nutzpflanzen.

Zucker ist einerseits ein Sammelbegriff für alle süß schmeckenden Mono- und Disaccharide, andererseits auch die im Handel gebräuchliche Bezeichnung für das Disaccharid Saccharose. Saccharose ist der gewöhnliche Haushalts- oder Kristallzucker und wird auch als Sucrose bezeichnet. Saccharose ist ein Dimer aus je einem Molekül α-D-Glucose und β-D-Fructose, die über eine α,β-1,2-glycosidische Bindung miteinander verbunden sind.

Saccharose wird in Pflanzen mittels Photosynthese gebildet. Die Biosynthese von Saccharose erfolgt im Cytoplasma der Pflanzenzellen. Dazu werden die beiden Triosephosphate Glycerinaldehyd-3-phosphat und Dihydroxyacetonphosphat, die als Nettogewinn bei der Kohlenstoffassimilation der Photosynthese (Calvin-Zyklus) entstehen, aus dem Chloroplasten in das Cytosol exportiert. Im Cytosol der Pflanzenzelle werden die Monosaccharide UDP-Glucose und Fructose-6-phosphat aus den Triosephosphaten gebildet. Dazu wird zuerst Fructose-1,6-bisphosphat durch eine Kondensationsreaktion zwischen Glycerinaldehyd-3-phosphat und Dihydroxyacetonphosphat gebildet. Fructose-1,6-bisphosphat wird dann durch Dephosphorylierung zu Fructose-6-Phosphat umgesetzt. Aus Fructose-6-Phosphat kann durch Isomerisierung auch Glucose-6-phosphat gebildet werden, das nach voriger Umisomerisierung zu Glucose-1-phosphat mit Uridintriphosphat (UTP) zu Uridindiphosphat-Glucose (*UDP-Glucose*) reagiert. Die nachfolgende Kondensation von UDP-Glucose und Fructose-6-phosphat zu Saccharose-6-phosphat wird von dem Enzym Saccharose-phosphat-Synthase katalysiert. Die dafür nötige Energie wird durch die Abspaltung von Uridindiphosphat (UDP) bereitgestellt. Zuletzt wird der Phosphatrest von Saccharose-6-Phosphat in einer irreversiblen Reaktion durch das Enzym Saccharose-Phosphat-Phosphatase abgespalten, so dass Saccharose entsteht.

Saccharose ist ein nicht-reduzierendes Disaccharid und daher der wichtigste Transportzucker in Pflanzen. Saccharose wird in den Blättern der Pflanzen neu synthetisiert und über das Phloem in deren Speicherorgane transportiert, wo sie in den Vakuolen der dortigen Pflanzenzellen als Nährstoff- und Energiequelle akkumuliert wird.

Für die industrielle Gewinnung von Saccharose sind vor allem Zuckerrüben (*Beta vulgaris* subsp. *vulgaris*), Zuckerrohr (*Saccharum officinarum*) und Zuckerpalme (*Arenga pinnata,* Syn.: *Arenga saccharifera* Labil., vornehmlich in Indonesien) von Bedeutung. In kleineren Mengen wird Saccharose auch aus dem Saft des Zuckerahorns (*Acer saccharum*) gewonnen. Diese Pflanzen werden wegen ihres außergewöhnlich hohen Saccharosegehalts zur Gewinnung von Saccharose genutzt.

Im Zuckerrohr finden sich Zucker - überwiegend Saccharose - mit einem Anteil von gewöhnlich 10 bis 20 % im Mark der Pflanze (dessen Saccharosespeicherorgan). Der Rohrzucker wird durch Kristallisation und Raffination des durch Auspressen erhaltenen Pflanzensafts gewonnen.

Die Zuckerrübe ist eine zweijährige Pflanze, die im ersten Jahr einen Zuckervorrat im Rübenkörper anlegt, der im 2. Jahr der blühenden Pflanze als Nahrung dient. Die Zuckergewinnung erfolgt gewöhnlich aus Schnitzeln der Zuckerrübe in einem Extraktionsprozess mit Wasser. Der Extrakt kann danach mit Calciumoxid versetzt werden, um die Pflanzensäuren wie Oxalsäure oder Weinsäure und die Eiweißstoffe auszufällen. Der überschüssige Kalk wird durch das Einleiten von Kohlenstoffdioxid ausgeschieden. Durch das nachfolgende Abdampfen des Wassers aus der Zuckerlösung im Vakuum erhält man eine sirupartige Lösung. Der auskristallisierende Zucker wird durch Zentrifugieren vom zurückbleibenden, braunen Sirup abgetrennt. Der Rückstand, die Melasse, kommt als Viehfutter zum Einsatz oder wird für die alkoholische Gärung verwendet. Eine Reinigung des Zuckers (Raffination) erfolgt durch Umkristallisieren, Filtrieren und durch Eindampfen im Vakuum.

Durch jahrzehntelange Bemühungen in der Züchtung bei Saccharosespeichernden Pflanzen konnten erhebliche Zuwächse im Ertrag des Saccharosespeicherorgans und der Saccharosekonzentration erzielt werden. Beispielsweise liegt bei gegenwärtig für die Zuckergewinnung angebauten Rübensorten die Saccharosekonzentration des Rübenkörpers bei etwa 15 bis 20 Gew.-%, bezogen auf das Frischgewicht des Rübenkörpers. Allerdings sind die erzielten Saccharosekonzentrationen noch immer nicht zufriedenstellend.

Der vorliegenden Erfindung lag daher die Aufgabe zugrunde, Pflanzen mit einer höheren Saccharosekonzentration bereitzustellen und Verfahren zu finden, mit denen die Saccharosekonzentration von Pflanzen, insbesondere von Zuckerrohr und Zuckerrüben, erhöht werden kann.

In der unter WO 2010/072210 A1 veröffentlichten internationalen Anmeldung wird ein Verfahren zur Steigerung des Saccharoseertrags beim landwirtschaftlichen Anbau von Zuckerrüben offenbart. Bei dem Verfahren werden Zuckerrüben- oder Zuckerrohrpflanzen verwendet, deren genetische Ausstattung auf die Reduzierung der enzymatischen Aktivität einer Invertase gerichtet ist. Hierzu wird eine Nukleinsäure, die in einer Pflanzenzelle zur Reduzierung der enzymatischen Aktivität einer Invertase geeignet ist, zur Ausbildung eines Saccharosespeicherorgans einer Pflanze verwendet, bei dem die Saccharosekonzentration gegenüber der Saccharosekonzentration eines nicht veränderten Kontrollsaccharosespeicherorgans des gleichen Genotyps in vergleichbarem Entwicklungsstadium erhöht ist.

Pflanzliche Vakuolen spielen eine zentrale Rolle bei der lang- oder kurzfristigen Speicherung von Zuckern, denn die Vakuole nimmt als Organell ein Volumen von etwa 90% in einer photosynthetisch aktiven Pflanzenzelle ein (Martinola, E. et al. (2007) "Vacuolar transporters and their essential role in plant metabolism", J. Exp. Bot. 58: 83-102). Vakuolen sind daher schon aufgrund ihrer Größe für die Speicherung von Zuckern von immenser Bedeutung (Neuhaus, H.E. (2007) "Transport of primary metabolites across the plant vacuolar membrane", FEBS Lett. 581: 2223-2226). Speichergewebe wie die Pfahlwurzel der Zuckerrübe (*Beta vulgaris*) und das Mark des Zuckerrohrs (*Saccharum officinarum*) reichern große Mengen an Saccharose in den Vakuolen der Zellen ihrer Speicherorgane an, um sie als Energiequelle für ihren Pflanzenstoffwechsel nutzen zu können.

In verschiedenen monokotyledonen und dikotyledonen Pflanzen wie *Nedicago* (Identifikationsnr. AC131026), *Vitis vinifera* (Identifikationsnr. AAX47312) und Reis (*Oryza sativa;* Identifikationsnr. Os02g13560) wurden Proteine entdeckt, welche für den Zuckertransport aus dem Cytoplasma der Pflanzenzelle in deren Vakuole verantwortlich sind. In der Pflanze *Arabidopsis* wurde ein Gen identifiziert, dessen Proteinprodukt ein Zuckertransporter ist, der in der Vakuolenmembran photosynthetisch aktiver Zellen lokalisiert ist und Glucose aus dem Cytosol in die Vakuole importieren kann (Wormit, A. et al. (2006) "Molecular identification and physiological characterization of a novel monosaccharide transporter from Arabidopsis involved invacuolar sugar transport", Plant Cell 18: 3476-3490). Dieses als tonoplastidärer Monosaccharid-Transporter (TMT) benannte Transportprotein ist in der Membran der Vakuole, dem Tonoplasten, lokalisiert. Das tonoplastidäre Monosaccharid-Transporter (TMT)-Protein umfasst drei Isoformen in *Arabidopsis thaliana,* welche als *At*TMT1, *At*TMT2 und A*t*TMT3 bezeichnet werden. Die Gene für A*t*TMT1 und *At*TMT2 weisen ein gewebe- und zellartspezifisches Expressionsmuster auf, wohingegen das A*t*TMT3-Gen nur sehr schwach exprimiert wird. Über TMT-Gen-Knockouts konnte gezeigt werden, dass die so veränderten Pflanzen im Vergleich zu Wildtyp-Pflanzen deutlich weniger Glucose und Fructose in ihren Vakuolen akkumulierten. Hinsichtlich der Akkumulation von Saccharose konnten jedoch keine Unterschiede zwischen den Wildtyp-Pflanzen und den TMT-Gen-Knockouts nachgewiesen werden.

Der tonoplastidäre Monosaccharid-Transporter TMT1 aus *Arabidopsis thaliana* wurde elektrophysiologisch als von Protonen getriebener Glucose- und Saccharose-Antiporter charakterisiert, der Glucose und Saccharose mit annähernd gleicher Spezifität über die Vakuolenmembran transportiert (Schulz, A. et al. (2011) "Proton-driven sucrose symport and antiport are provided by the vacuolar transporters SUC4 and TMT1/2", The Plant Journal 68: 129-136). In demselben Artikel wird auch das Saccharose-Transportprotein SUC4 von *Arabidopsis thaliana* als Protonen/Saccharose-Symporter charakterisiert, der ebenfalls in der Vakuolenmembran lokalisiert sein soll.

In der unter WO 2011/120549 A1 veröffentlichten internationalen Anmeldung wird offenbart, dass durch Überexpression des tonoplastidären Monosaccharid-Transporters AtTMT1 in Pflanzen der Samenertrag erhöht oder der Protein- und Ölgehalt der Samen gesteigert oder das Frühwachstum von monokotyledonen oder dikotyledonen Pflanzen gefördert werden kann. Eine Anreichung von Saccharose in einem Speicherorgan wird dagegen nicht offenbart.

Vor diesem Hintergrund wurde die der vorliegenden Erfindung zugrundeliegende Aufgabe durch die Identifizierung des für den Import von Zucker in die Vakuole der Pflahlwurzelzellen von Zuckerrüben verantwortlichen Proteine, insbesonderedes für den Import von Saccharose in die Vakuolen der Pfahlwurzelzellen von Zuckerrüben verantwortlichen Proteins, welches spezifisch für Saccharose ist, gelöst. Mit der Identifizierung dieser Proteine, insbesondere mit der Identifizierung dieses ersten für Saccharose spezifischen tonoplastidären Protonen/Zucker-Antiporter-Proteins und der für diese Proteine kodierenden Nukleotidsequenzen werden züchterische und/oder molekulargenetische Verfahren zur Erhöhung der Saccharosekonzentration in Pflanzen und somit auch Pflanzen mit einer höheren Saccharosekonzentration bereitgestellt.

Gemäß einem ersten Aspekt wird ein Nukleinsäuremolekül beschrieben, das ein tonoplastidäres Protonen/Zucker-Antiporter-Protein kodiert. Vorzugsweise kodiert das Nukleinsäuremolekül ein tonoplastidäres Protonen/Zucker-Antiporter-Protein, welches spezifisch für Saccharose ist. Nachfolgend wird ein solches Protonen/Zucker-Antiporter-Protein, welches spezifisch für Saccharose ist, auch als Protonen/Saccharose-Antiporter-Protein bezeichnet.

Gemäß einem zweiten Aspekt wird ein rekombinantes Gen offenbart, umfassend ein Nukleinsäuremolekül gemäß dem ersten Aspekt oder ein Nukleinsäuremolekül mit einer Nukleotidsequenz, die für ein tonoplastidäres Protonen/Zucker-Antiporter-Protein, vorzugsweise für ein ein tonoplastidäres Protonen/Saccharose-Antiporter-Protein, kodiert. Das Nukleinsäuremolekül kann operativ mit zumindest einem regulatorischen Element verbunden sein.

Gemäß einem dritten Aspekt betrifft die Erfindung einen Vektor oder ein mobiles genetisches Element, umfassend ein Nukleinsäuremolekül gemäß dem ersten Aspekt.

Offenbart ist auch ein Vektor enthaltend ein rekombinantes Gen gemäß dem zweiten Aspekt.

Gemäß einem weiteren Aspekt betrifft die Erfindung eine eukaryontische Wirtszelle oder eine prokaryontische Wirtszelle, die einen Vektor oder mobiles genetisches Element gemäß dem dritten Aspekt umfasst.

Eine eukaryontische Wirtszelle oder eine prokaryontische Wirtszelle, die ein Nukleinsäuremolekül gemäß dem ersten Aspekt, vorzugsweise als Transgen oder ein rekombinantes Gen gemäß dem zweiten Aspekt umfasst, wird ebenfalls beschrieben.

In einem weiteren Aspekt wirdein Protein beschrieben, das als tonoplastidärer Protonen/Zucker-Antiporter, welcher vorzugsweise spezifisch für Saccharose ist, beziehungsweise vorzugsweise als tonoplastidärer Protonen/Saccharose-Antiporter funktioniert.

Gemäß einem weiteren Aspekt betrifft die Erfindung eine transgene Pflanzenzelle, die ein Nukleinsäuremolekül gemäß dem ersten Aspekt als Transgen oder einen Vektor oder mobiles genetisches Element gemäß dem dritten Aspekt umfasst, sowie eine transgene Pflanze oder Teile davon, die mindestens eine solche trangene Pflanzenzelle umfassen.

Eine transgene Pflanzenzelle enthaltend ein rekombinantes Gen gemäß dem zweiten Aspekt als Transgen wird ebenfalls beschrieben.

Gemäß einem weiteren Aspekt betrifft die Erfindung Samen einer transgenen Pflanze gemäß dem vorhergehenden Aspekt, wobei der Samen ein Nukleinsäuremolekül gemäß dem ersten Aspekt als Transgen einen Vektor oder mobiles genetisches Element gemäß dem dritten Aspekt aufweist.

Samen einer transgenen Pflanze, die ein rekombinantes Gen gemäß dem zweiten Aspekt als Transgen aufweisen, sind ebenfalls offenbart.

Gemäß einem weiteren Aspekt betrifft die Erfindung Verfahren zur Herstellung von transgenen Pflanzen.

Gemäß einem weiteren Aspekt betrifft die Erfindung Verfahren zur Erhöhung der Saccharosekonzentration eines Saccharosespeicherorgans einer Pflanze.

Gemäß einem weiteren Aspekt betrifft die Erfindung Verfahren zum Identifizieren einer Pflanze, die geeignet ist, eine erhöhte Saccharosekonzentration in einem Saccharosespeicherorgan der Pflanze zu erzeugen.

Gemäß einem weiteren Aspekt betrifft die Erfindung Oligonukleotide, die zur Verwendung als molekulare Marker für den diagnostischen Nachweis eines Nukleinsäuremoleküls gemäß dem ersten Aspekt geeignet sind.

Offenbart sind auch Antikörper die diagnostisch für ein Protein sind, das als tonoplastidärer Protonen/Zucker-Antiporter, welcher vorzugsweise spezifisch für Saccharose ist, beziehungsweise vorzugsweise als tonoplastidärer Protonen/Saccharose-Antiporter funktioniert.

Gemäß einem weiteren Aspekt betrifft die Erfindung die Verwendung von tonoplastidären Protonen/Zucker-Antiporter-Proteinen zur Erhöhung der Saccharosekonzentration eines Saccharosespeicherorgans einer Pflanze.
Figur 1 zeigt eine Tabelle, aus der die Identitäten und die Ähnlichkeiten der Aminosäuresequenzen der drei paralogen tonoplastidären Monosaccharid-Transporter (TMT)-Proteine von *Arabidopsis thaliana* mit den vier paralogen tonoplastidären Zucker-Transporter (TST)-Proteinen aus *Beta vulgaris* hervorgehen.
Figur 2 zeigt ein Cladogramm, in dem die phylogenetischen Beziehungen der drei paralogen tonoplastidären Monosaccharid-Transporter (TMT)-Proteine von *Arabidopsis thaliana* und den vier paralogen tonoplastidären Zucker-Transporter (TST)-Proteinen aus *Beta vulgaris* dargestellt sind.
Figur 3 zeigt ein Säulendiagramm, das die Saccharosekonzentration in unterschiedlich alten Pfahlwurzeln zweier Zuckerrübensorten veranschaulicht.
Figur 4 zeigt ein Säulendiagramm, aus dem die relativen mRNA-Mengen der vier paralogen TST-Gene von *Beta vulgaris* bei zwei unterschiedlichen Zuckerrübensorten an unterschiedlichen Entwicklungszeitpunkten hervorgehen.
Figur 5 zeigt ein Säulendiagramm, das die Konzentration an verschiedenen Zuckern in den Blättern der Zuckerrübensorte "Belladonna KWS" an verschiedenen Entwicklungszeitpunkten darstellt.
Figur 6 zeigt ein Säulendiagramm, aus dem die relativen mRNA-Menge für die vier paralogen BvTST-Gene in Blättern der Zuckerrübensorte "Belladonna KWS" an unterschiedlichen Entwicklungszeitpunkten hervorgehen.
Figur 7 ist ein Säulendiagramm, dass die von verschiedenen Zuckern (Sacchariden) bei Vakuolen von transient transformierten Mesophyllzellen induzierte Veränderung der Stromdichte zeigt.

Die Erfinder haben das hier als BvTST2.1 bezeichnete Protein als eines der in der Vakuolenmembran von Pfahlwurzelzellen der Zuckerrübe am mengenmäßig häufigsten vorhandenen Proteine identifiziert und überraschenderweise festgestellt, dass das Protein BvTST2.1 als tonoplastidärer Zucker-Transporter spezifisch Saccharose aus dem Cytosol in die Vakuolen von Pflanzenzellen importieren kann. Daher stellen dieses Protein sowie Proteine mit gleicher Funktion -nicht nur tonoplastidäre Zucker-Transporter (TST) dar, sondern werden nachfolgend auch als tonoplastidäre Saccharose-Transporter oder tonoplastidäre Protonen/Saccharose-Antiporter oder tonoplastidäre Protonen/Saccharose-Antiporter-Proteine bezeichnet, wobei *"Bv"* in der hier verwendeten Abkürzung für *Beta vulgaris* steht, dem Organismus, in dem dieses Protein ursprünglich identifiziert wurde. Das Protein BvTST2.1 wurde von den Erfindern als ein für Saccharose hochspezifisches Protonen/Zucker-Antiporter-Protein identifiziert und stellt den ersten bekannten Vertreter dieser pflanzlichen Zucker-Transport-Protein-Familie dar. Daneben gelang auch die Identifizierung von drei anderen paralogen Isoformen, *Bv*TST1, BvTST2.2 und *Bv*TST3, die funktionell wahrscheinlich den bekannten TMT-Proteinen aus Arabidopsis zuzuordnen sind.

Ausgehend von der Identifizierung dieses neuartigen, für Sachcharose spezifischen Antiporters haben die Erfinder auch die für das tonoplastidäre Protonen/Zucker-Antiporter-Protein sowie die für die anderen Isoformen kodierenden Nukleotidsequenzen identifiziert.

Daher werden Nukleinsäuremoleküle offenbart, die ein tonoplastidäres Protonen/Zucker-Antiporter-Protein, vorzugsweise ein tonoplastidäres Protonen/Saccharose-Antiporter-Protein kodieren.

Gemäß dem dritten Aspekt umfasst das Nukleinsäuremolekül, das ein tonoplastidäres Protonen/Saccharose-Antiporter-Protein kodiert, ein Nukleinsäuremolekül, ausgewählt aus der Gruppe:
a) ein Nukleinsäuremolekül mit einer Nukleotidsequenz gemäß SEQ ID NO: 2, oder ein Nukleinsäuremolekül mit einer Nukleotidsequenz, die eine Identität von mindestens 80% zu der Nukleotidsequenz gemäß SEQ ID NO: 2 aufweist;
b) ein Nukleinsäuremolekül mit einer Nukleotidsequenz, die komplementär zu einer der Nukleotidsequenzen gemäß a) ist und
c) ein Nukleinsäuremolekül mit einer Nukleotidsequenz, die ein Polypeptid mit einer Aminosäuresequenz gemäß SEQ ID NO: 1 kodiert, oder ein Nukleinsäuremolekül mit einer Nukleotidsequenz, die ein Polypeptid kodiert, dessen Aminosäuresequenz eine Identität von mindestens 80% zu der Aminosäuresequenz gemäß SEQ ID NO: 1 aufweist
d) .

Darüber hinaus wird ein Nukleinsäuremolekül offenbart, das ein tonoplastidäres Protonen/Saccharose-Antiporter-Protein kodiert, umfassend ein Nukleinsäuremolekül, ausgewählt aus der Gruppe:
e) ein Nukleinsäuremolekül, das mit einem Nukleinsäuremolekül gemäß a) oder b) hybridisiert; und
f) ein Nukleinsäuremolekül mit einer Nukleotidsequenz, die ein Homolog, ein Analog oder ein Ortholog des Polypeptids gemäß SEQ ID NO: 1 kodiert.

### Offenbart wird auch ein

Nukleinsäuremolekül, das ein tonoplastidäres Protonen/Saccharose-Antiporter-Protein kodiert, umfassend ein Nukleinsäuremolekül, ausgewählt aus der Gruppe:
a) ein Nukleinsäuremolekül mit einer Nukleotidsequenz gemäß SEQ ID NO: 2, oder ein Nukleinsäuremolekül mit einer Nukleotidsequenz, die eine Identität von mindestens 80% zu der Nukleotidsequenz gemäß SEQ ID NO: 2 aufweist;
b) ein Nukleinsäuremolekül mit einer Nukleotidsequenz, die komplementär zu einer der Nukleotidsequenzen gemäß a) ist;
c) ein Nukleinsäuremolekül, das mit einem Nukleinsäuremolekül gemäß a) oder b) hybridisieren;
d) ein Nukleinsäuremolekül mit einer Nukleotidsequenz, die ein Polypeptid mit einer Aminosäuresequenz gemäß SEQ ID NO: 1 kodiert, oder ein Nukleinsäuremolekül mit einer Nukleotidsequenz, die ein Polypeptid kodiert, dessen Aminosäuresequenz eine Identität von mindestens 80% zu der Aminosäuresequenz gemäß SEQ ID NO: 1 aufweist; und
e) ein Nukleinsäuremolekül mit einer Nukleotidsequenz, die ein Homolog, ein Analog oder ein Ortholog des Polypeptids gemäß SEQ ID NO: 1 kodiert.

Der Begriff "Nukleinsäuremolekül mit einer Nukleotidsequenz" umfasst nicht nur Nukleinsäuremoleküle deren Nukleotidsequenz aus der dann näher bezeichneten Nukleotidsequenz besteht, sondern auch Nukleinsäuremoleküle, die zu der dann näher bezeichneten Nukleotidsequenz zusätzlich mindestens ein Nukleotid oder Nukleotidsequenzen aufweisen.

Gemäß einer alternativen und/oder zusätzlichen Ausführungsform kodiert das Nukleinsäuremolekül für eine Aminosäuresequenz gemäß SEQ ID NO: 1, 3, 5 oder 7. Das Nukleinsäuremolekül kann aber auch für eine Aminosäuresequenz kodieren, bei der mindestens ein Aminosäurerest der Aminosäuresequenz durch eine Aminosäure, welche ähnliche chemische Eigenschaften aufweist, ausgetauscht ist (konservative oder semikonservative Aminosäureaustausch). Bei einem konservativen Aminosäureaustausch wird eine Aminosäure durch eine andere Aminosäure mit ähnlichen chemischen Eigenschaften ersetzt. Bei einem semikonservativen Aminosäureaustausch wird eine Aminosäure durch eine andere Aminosäure mit ähnlicher sterischer Konformation ersetzt. Der Austausch hat vorzugsweise keine Folgen für die Proteinfunktion. Beispiele für Aminosäureaustausche sind Asp und Glu, Leu und Ile, Ala und Val, Arg und Lys, sowie Phe und Trp.

Gemäß einer alternativen und/oder zusätzlichen Ausführungsform weisen die Nukleotidsequenzen der Nukleinsäuren und/oder die Aminosäuresequenzen, die von den Nukleotidsequenzen kodiert werden, eine Identität von mindestens 80%, mindestens 85%, vorzugsweise von mindestens 90%, besonders bevorzugt von mindestens 95%, mindestens 96%, mindestens 97% oder mindestens 98%, und ganz besonders bevorzugt von mindestens 99% zu der Nukleotidsequenz gemäß SEQ ID NO: 2, 4, 6 oder 8 beziehungsweise der Aminosäuresequenz gemäß SEQ ID NO: 1, 3, 5 oder 7 auf.

Der hier verwendete Begriff "hybridisieren" bedeutet hybridisieren unter üblichen Bedingungen, wie sie in Sambrook et al. (1989) "Molecular Cloning, A Laboratory Manual" (Cold Spring Harbor Laboratory Press, New York) beschrieben sind, bevorzugt unter stringenten Bedingungen. Stringente Hybridisierungsbedingungen sind beispielsweise: Hybridisieren in 4 x SSC bei 65 °C und anschließendes mehrfaches Waschen in 0,1 x SSC bei 65 °C für insgesamt etwa 1 Stunde. Wenig stringente Hybridisierungsbedingungen sind beispielsweise: Hybridisieren in 4 x SSC bei 37 °C und anschließendes mehrfaches Waschen in 1 x SSC bei Raumtemperatur. "Stringente Hybridisierungsbedingungen" kann auch bedeuten: Hybridisieren bei 68 °C in 0,25 M Natriumphosphat, pH 7,2, 7 % SDS, 1 mM EDTA und 1 % BSA für 16 Stunden und anschließendes zweimaliges Waschen mit 2 x SSC und 0,1 % SDS bei 68 °C.

Unter "spezifisch für Saccharose" oder "hochspezifisch für Saccharose" oder "Saccharose spezifischer Transport" oder "Saccharose hochspezifischer Transport" oder "Spezifität für Saccharose" oder Saccharosespezifität" ist zu verstehen, dass die Spezifität eines tonoplastidären Protonen/Zucker-Antiporter-Proteins für Saccharose gegenüber einem anderen Zucker mindestens 5-fach, 10-fach oder 15-fach, vorzugsweise mindestens 18-fach, 20-fach, 22-fach, 24-fach, 26-fach oder 28-fach, besonders bevorzugt mindestens 30-fach, mindestens 31-fach, mindestens 32-fach, mindestens 33-fach, mindestens 34-fach, mindestens 35-fach, mindestens 36-fach, mindestens 37-fach, mindestens 38-fach oder mindestens 39-fach, und ganz besonders bevorzugt mindestens 40-fach höher ist. Im Sinne der Erfindung ist unter "spezifische für Saccharose" zu verstehen, dass die Spezifität eines tonoplastidären Protonen/Zucker-Antiporter-Proteins für Saccharose gegenüber einem anderen Zucker mindestens 5-fach höher ist. Weiterhin kann dies auch bedeuten, dass die Spezifität eines tonoplastidären Protonen/Zucker-Antiporter-Proteins für Saccharose gegenüber einem Monosaccharid wie Glucose oder Fructose mindestens 5-fach, 10-fach oder 15-fach, vorzugsweise mindestens 18-fach, 20-fach, 22-fach, 24-fach, 26-fach oder 28-fach, besonders bevorzugt mindestens 30-fach, mindestens 31-fach, mindestens 32-fach, mindestens 33-fach, mindestens 34-fach, mindestens 35-fach, mindestens 36-fach, mindestens 37-fach, mindestens 38-fach oder mindestens 39-fach, und ganz besonders bevorzugt mindestens 40-fach höher ist.

Im Sinne der Erfindung wird unter einem "Homolog" ein Protein gleichen phylogenetischen Ursprungs verstanden, unter einem "Analog" ein Protein verstanden, welches die gleiche Funktion ausübt, jedoch einen anderen phylogenetischen Ursprung hat, und unter einem "Ortholog" ein Protein aus einer anderen Spezies verstanden, das die gleiche Funktion ausübt.

Offenbart wird auch ein rekombinantes Gen, umfassend ein Nukleinsäuremolekül gemäß dem ersten Aspekt oder ein Nukleinsäuremolekül mit einer Nukleotidsequenz, die vorzugsweise für ein tonoplastidäres Protonen/Saccharose-Antiporter-Protein kodiert. Das Nukleinsäuremolekül kann operativ mit zumindest einem regulatorischen Element verbunden sein.

Unter einem "regulatorischen Element" werden Nukleotidsequenzen verstanden, die nicht Teil der proteinkodierenden Nukleotidsequenz sind, aber die Expression der proteinkodierenden Nukleotidsequenz vermitteln. Regulatorische Elemente sind beispielsweise Promotoren, cis-regulatorische Elemente, Enhancer, Introns oder Terminatoren. Abhängig von der Art des regulatorischen Elements ist dieses auf dem Nukleinsäuremolekül vor (also 5' von) oder hinter (also 3' von) der proteinkodierenden Nukleotidsequenz angeordnet. Die regulatorischen Elemente sind in einer lebenden Pflanzenzelle funktionsfähig.

Der Begriff "operativ verbunden" bedeutet, dass ein regulatorisches Element in der Weise mit der proteinkodierenden Nukleotidsequenz verbunden ist, also derart zu der proteinkodierenden Nukleotidsequenz auf beispielsweise einem Nukleinsäuremolekül positioniert ist, dass eine Expression der proteinkodierenden Nukleotidsequenz unter Kontrolle des regulatorischen Elements in einer lebenden Zelle erfolgen kann.

Im Sinne der vorliegenden Erfindung ist ein "Promotor" eine die Expression eines Gens regulierende Nukleotidsequenz, die üblicherweise am 5'-Ende eines Gens liegt und über Wechselwirkung mit bestimmten DNA-bindenden Proteinen den Start der Transkription durch RNA-Polymerase vermittelt. Beispiele für Promotoren, die in pflanzlichen Zellen funktionell sind, umfassen konstitutive Promotoren wie virale Promotoren, zum Beispiel den CaM35S-Promotor, einen doppelten CaM35S-Promotor, oder wie pflanzliche Promotoren, beispielsweise Ubiquitin-Promotoren wie in der EP 0 305 668 und US 6,528,701 beschrieben. Weiterhin können auch Promotoren eingesetzt werden, welche beispielsweise spezifische Aktivität zu bestimmten Entwicklungsphasen oder Induzierbarkeit durch Umweltfaktoren wie biotischer oder abiotischer Stress oder eine Gewebespezifität aufweisen. Insbesondere sind solche Promotoren einsetzbar, welche eine erhöhte Spezifität für das Saccharosespeicherorgan oder Teile davon zeigen, also insbesondere in diesem Saccharosespeicherorgan oder in Teilen davon aktiv sind. Für die Zuckerrübe kann der Promotor beispielweise ein wurzelspezifischer bzw. pfahlwurzelspezischer Promotor sein. Solche sind dem Fachmann aus dem Stand der Technik hinreichend bekannt: WO 02/40687, Oltmanns, H. et al. (2006) "Taproot promoters cause tissue specific gene expression within the storage root of sugar beet", Planta 224: 485-495, Noh, Seol Ah, et al. (2012) "A sweetpotato SRD1 promoter confers strong root-, taproot-, and tuber-specific expression in Arabidopsis, carrot, and potato", Transgenic research 21: 265-278. Für Zuckerrohr können vorzugsweise Halm-spezifische Promotoren verwendet werden, wie sie beispielsweise aus Goshu Abraha, Tsion. "Isolation and characterisation of a culm-specific promoter element from sugarcane", Diss. Stellenbosch: University of Stellenbosch, 2005; Govender, C. "Stem specific promoters from sorghum and maize for use in sugarcane". Diss. Stellenbosch: Stellenbosch University, 2008; und Mudge, S.R. et al. (2013) "Mature-stem expression of a silencing-resistant sucrose isomerase gene drives isomaltulose accumulation to high levels in sugarcane", Plant Biotechnology Journal 1: 502-509) bekannt sind.

Als geeignete Promotoren haben sich weiterhin auch synthetische Promotoren erwiesen. Hierbei handelt es sich um durch molekularbiologische Arbeitstechniken erstellte Promotoren, die in dieser Ausführung nicht in der Natur gefunden werden. Ein synthetischer Promotor ist ein minimalistischer Promotor, der neben einem Minimalpromotor nur noch ein oder mehrere ausgewählte, definierte cis-Elemente enthält. Diese cis-Elemente sind Bindungsstellen für DNA-Bindungsproteine wie Transkriptionsfaktoren und werden aus natürlichen Promotoren isoliert, aus bereits isolierten cis-Elementen abgeleitet oder durch zufallsorientierte Rekombinationstechniken technisch erzeugt und durch geeignete Verfahren ausgewählt, im Vergleich zu einem natürlichen Promotor wird ein synthetischer Promotor aufgrund seines weniger komplexen Aufbaus nur durch wenige exogene und endogene Faktoren aktiviert und ist damit spezifischer reguliert.

Der "Minimalpromoter" oder "Core"-Promoter ist eine Nukleotidsequenz, welche die Bindungsstellen für den basalen Transkriptionsfaktorkomplex enthält und die akkurate Initiation der Transkription durch die RNA-Polymerase II ermöglicht. Charakteristische Sequenzmotive des Minimalpromotors sind die TATA-Box, das Initiatorelement (Inr), das "TFBII recognition element"(BRE) und das "downstream core promoter element" (OPE). Diese Elemente können im Minimalpromotor einzeln oder in Kombination vorkommen. Erhältlich ist der Minimalpromotor oder sind seine Sequenzmotive zum Beispiel von einem beliebigen pflanzlichen, bakteriellen, pilzlichen oder viralen Gen.

"Cis-Elemente" sind Nukleotidsequenzen, die auf demselben Nukleinsäuremolekül liegen wie die proteinkodierende Nukleotidsequenz, die zu exprimieren ist. Cis-Elemente müssen weder RNA noch Protein kodieren und können in Transkriptionsrichtung vor oder hinter der zu exprimierenden proteinkodierenden Nukleotidsequenz liegen. Cis-Elemente stromaufwärts vor einer zu exprimierenden proteinkodierenden Nukleotidsequenz stellen oft notwendige Bindungsmotive für insbesondere Transkriptionsfaktoren dar, die hier als *transwirkende Elemente* (von lat. *trans* 'jenseits') molekular gesehen von anderer Seite in die Regulierung der Transkription dieses Gens eingreifen. Führen cis-Elemente darüber zu einer Hemmung der Transkription, werden sie als Silencer bezeichnet. Cis-Elemente, die zu einer Verstärkung der Transkription führen, werden Enhancer genannt. Die Gesamtheit der cis-/trans-Aktivitäten im Promotor bestimmt schließlich die Intensität, mit der die RNA-Polymerase die Transkription durchführt.

Weiterhin kann es sich bei einem Promotor auch um einen chimären Promotor und/oder um einen Promotor, der durch cis-Elemente modifiziert wurde, handeln. Die Modifikation eines Promotors kann dabei auch das zusätzliche Einbringen eines cis Elements in den Promotor bedeuten, welcher beispielsweise natürlicherweise bereits ein cis-Element aufweist. Ferner umfasst die Modifikation auch eine Multimerisierung eines cis-Elements, insbesondere eine Multimerisierung eines natürlicherweise vorhandenen cis-Elements. Ein solcher modifizierter Promotor kann im Vergleich mit der nativen Version geänderte Eigenschaften hinsichtlich beispielsweise Spezifität, Expressionslevel oder Hintergrundaktivität aufweisen.

Terminatoren sind Nukleotidsequenzen auf der DNA, die gewöhnlich das Ende eines Gens markieren und zur Beendigung der Transkription führen.

Gemäß einer alternativen und/oder zusätzlichen Ausführungsform sind die für das tonoplastidäre Protonen/Zucker-Antiporter-Protein kodierende Nukleotidsequenz, insbesondere die für das tonoplastidäre Protonen/Saccharose-Antiporter-Protein kodierende Nukleotidsequenz, und die Nukleotidsequenz des mindestens einen regulatorischen Elements heterolog. Das bedeutet, dass sie aus unterschiedlichen Spezies stammen oder natürlicherweise nicht in der vorgesehenen Kombination in einer Spezies vorkommen.

Gemäß einem dritten Aspekt betrifft die Erfindung einen Vektor oder ein mobiles genetisches Element, umfassend ein Nukleinsäuremolekül mit einer Nukleotidsequenz gemäß dem ersten Aspekt.

Hierbei wird unter einem Vektor ein Transportvehikel für ein Nukleinsäuremolekül gemäß dem ersten Aspekt oder ein rekombinantes Gen gemäß dem zweiten Aspekt verstanden, insbesondere für die Übertragung einer Fremd-Nukleinsäure in eine lebende Empfängerzelle. Bei der lebenden Empfängerzelle kann es sich um eine eukaryontische Zelle oder eine prokaryontische Zelle handeln. Zu den Vektoren zählen beispielsweise Plasmide, Cosmide, künstliche Hefechromosomen (YACs), künstliche Bakterienchromsomen (BACs) oder künstliche P1 Chromosomen (PACs) aber auch modifizierte Viren wie Adenoviren, Retroviren und Phagen.

Mobile genetische Elemente sind Nukleotidsequenzen, deren Position im Genom eines Organismus veränderlich ist. Zu den mobilen genetischen Elementen zählen beispielsweise eigennützige Nukleotidsequenzen wie Transposons, Retroelemente, Insertionssequenzen und Inteine, aber auch Gruppe-II-Introns, insertierende Plasmide und manche Bakteriophagen, wie der Mu-Phage.

Gemäß einem weiteren Aspekt betrifft die Erfindung eine eukaryontische Wirtszelle oder prokaryontische Wirtszelle, die einen Vektor oder mobiles genetisches Element gemäß dem dritten Aspekt als Transgen umfasst. Das bedeutet, dass das der Vektor oder das mobile genetische Element in die Wirtszelle eingeschleust wurde, beispielsweise mittels Transformation oder Transfektion. Beispiele für prokaryontische Wirtszellen sind Bakterien der Gattung *A. tumefaciens, E. coli* und *B. subtilis.* Beispiele für eukaryontische Wirtszellen sind Hefezellen wie *Saccharomyces* oder *Schizosaccharomyces,* aber auch Zellen tierischen oder pflanzlichen Ursprungs.

Offenbart werden auch Proteine, die als tonoplastidärer Protonen/Saccharose-Antiporter funktionieren. Dieser Antiporter ist spezifisch für Saccharose. Vorzugsweise wird das Protein durch ein Nukleinsäuremolekül gemäß des ersten Aspekts kodiert.

Gemäß einer Ausführungsform ist das tonoplastidäre Protonen/Saccharose-Antiporter-Protein aus der Gruppe von Proteinen ausgewählt, die
a) eine Aminosäuresequenz gemäß SEQ ID NO: 1 aufweisen;
b) eine Aminosäuresequenz aufweisen, die eine Identität von mindestens 80% zu der Aminosäuresequenz gemäß SEQ ID NO: 1 hat;
c) ein Homolog, ein Analog oder ein Ortholog des Proteins gemäß SEQ ID NO: 1 sind.

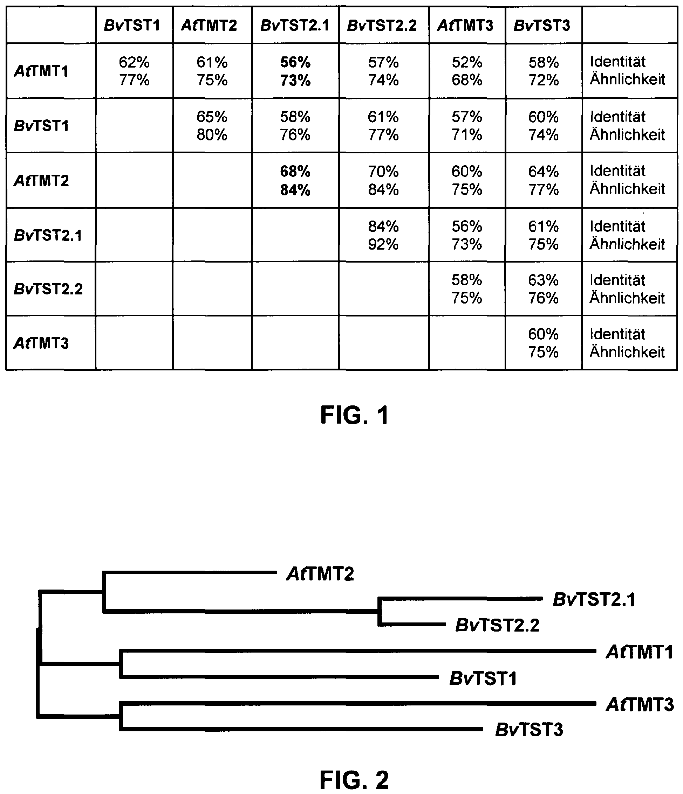

Das tonoplastidäre Protonen/Zucker-Antiporter-Protein gemäß SEQ ID NO: 1, auch als *Bv*TST2.1 bezeichnet, weist eine Aminosäuresequenz mit einer Länge von 735 Aminosäuren auf. Eine Hydrophobizitätsanalyse weist darauf hin, dass *Bv*TST2.1 offensichtlich 12 hydrophobe Transmembrandomänen und eine große, zentral lokalisierte hydrophile Schleife aufweist, welche die sechste und die siebente Transmembrandomäne miteinander verbindet. Die größte Sequenzidentität weist *Bv*TST2.1 zu dem tonoplastidären Monosaccharid Transporter Protein 2 von *Arabidopsis thaliana* (*At*TMT2) auf. Die Identität dieser beiden Aminosäuresequenzen liegt bei 68% und sie weisen unter Berücksichtigung von konservativen und semikonservativen Aminosäureaustauschen eine Sequenzähnlichkeit von 84% auf (Fig. 1).

Offenbart sind auch Proteine, die als tonoplastidärer Protonen/Zucker-Antiporter funktionieren. Vorzugsweise wird das Protein durch ein Nukleinsäuremolekül gemäß des ersten Aspekts kodiert.

Gemäß einer Ausführungsform ist das tonoplastidäre Protonen/Zucker-Antiporter-Protein aus der Gruppe von Proteinen ausgewählt, die
a) eine Aminosäuresequenz gemäß SEQ ID NO: 3, 5 oder 7 aufweisen;
b) eine Aminosäuresequenz aufweisen, die eine Identität von mindestens 80% zu der Aminosäuresequenz gemäß SEQ ID NO: 3, 5 oder 7 hat;
c) ein Homolog, ein Analog oder ein Ortholog des Proteins gemäß SEQ ID NO: 3, 5 oder 7 sind.

Das tonoplastidäre Protonen/Zucker-Antiporter-Protein gemäß SEQ ID NO: 3 wird auch als *Bv*TST1, gemäß SEQ ID NO: 5 auch als *Bv*TST2.2 und gemäß SEQ ID NO: 7 auch als *Bv*TST3 bezeichnet.

Da das in *Beta vulgaris* identifizierte tonoplastidäre Protonen/Saccharose Antiporter-Protein *Bv*TST2.1 wie auch die anderen tonoplastidären Protonen/Zucker Antiporter-Protein *Bv*TST1, *Bv*TST2.2 und *Bv*TST3 auch Sequenzidentitäten zu Transportproteinen von anderen Pflanzen aufweisen, umfassen die tonoplastidären Protonen/Zucker-Antiporter-Proteine, insbesondere die tonoplastidären Protonen/Saccharose-Antiporter-Proteine, auch Proteine, deren Aminosäuresequenz eine Identität von mindestens 80% zu der Aminosäuresequenz gemäß SEQ ID NO: 1, 3, 5 oder 7 hat, vorzugsweise von mindestens 85%, mindestens 90%, mindestens 95%, mindestens 96%, mindestens 97%, mindestens 98% oder mindestens 99%, ebenso wie Homologe, Analoge oder Orthologe davon. Hierbei ist unerheblich, in welcher Spezies diese Proteine natürlicherweise vorkommen oder ob es sich um nicht natürlich vorkommende Proteine handelt, die beispielsweise mittels molekulargenetischer Methoden erzeugt werden.

Gemäß einem weiteren Aspekt betrifft die Erfindung eine transgene Pflanzenzelle, die ein Nukleinsäuremolekül gemäß dem ersten Aspekt als Transgen, oder einen Vektor oder mobiles genetisches Element gemäß dem dritten Aspekt als Transgen umfasst, sowie eine transgene Pflanze oder Teile davon, die mindestens eine solche Pflanzenzelle umfassen. Insofern umfassen auch die transgene Pflanze oder Teile davon ein Nukleinsäuremolekül gemäß dem ersten Aspekt als Transgen, oder einen Vektor oder mobiles genetisches Element gemäß dem dritten Aspekt als Transgen.

Gemäß einem weiteren Aspekt betrifft die Erfindung Samen einer transgenen Pflanze gemäß dem vorhergehenden Aspekt, wobei der Samen und insbesondere mindestens eine embryonale Zelle des Samens ein Nukleinsäuremolekül gemäß dem ersten Aspekt als Transgen, oder einen Vektor oder mobiles genetisches Element gemäß dem dritten Aspekt umfasst.

In einer Ausführungsform handelt es sich bei der Pflanzenzelle um die Zelle einer monokotyledonen Pflanze. In einer anderen Ausführungsform handelt es sich bei der Pflanzenzelle um eine Zelle einer dikotyledonen Pflanze. Gemäß einer weiteren und/oder zusätzlichen Ausführungsform handelt es sich bei der Pflanzenzelle um Zellen einer Pflanze, die aus der Gruppe der Arten oder übergeordneten Gattungen ausgewählt ist, die *Beta vulgaris, Saccharum officinarum, Arenga saccharifera, Acer saccharum* und *Sorghum sp.* umfasst. Dementsprechend ist gemäß einer weiteren Ausführungsform die transgene Pflanze aus der Gruppe ausgewählt, die *Beta vulgaris, Saccharum officinarum, Arenga saccharifera, Acer saccharum* und *Sorghum sp.* umfasst. Gemäß einer weiteren Ausführungsform stammen die Teile einer transgene Pflanze oder der Samen einer transgenen Pflanze aus der Gruppe von Pflanzen, die *Beta vulgaris, Saccharum officinarum, Arenga saccharifera, Acer saccharum* und *Sorghum sp.* umfasst.

In einer zusätzlichen und/oder alternativen Ausführungsform weist die transgene Pflanzenzelle, die transgene Pflanze oder die Teile der transgenen Pflanze, bei dem es sich vorzugsweise um das Saccharosespeicherorgan der Pflanze handelt, eine höhere Saccharosekonzentration als die unter identischen Bedingungen kultivierte isogene Pflanzenzelle oder Pflanze auf. Ferner können Teile einer Pflanze verbunden sein mit oder abgetrennt sein von der gesamten intakten Pflanze. Solche Teile schließen beispielsweise Organe, Gewebe, Zellen und Samen der Pflanze ein.

Vorzugsweise beruht die höhere Saccharosekonzentration auf einer höheren Saccharosekonzentration in der pflanzlichen Vakuole, insbesondere in der Vakuole von mindestens einer Zelle des Saccharosespeicherorgans der Pflanze. Besonders bevorzugt weist eine Pflanze mit einer höheren Saccharosekonzentration auch einen gesteigerten Saccharose-Ertrag auf. Ertrag bedeutet hierbei die Ausbeute an Saccharose aus dem Saccharosespeicherorgan mit Bezug auf eine definierte Anbaufläche (z.B. ein Hektar) oder mit Bezug auf das Gewicht eines Saccharosespeicherorgans unter Berücksichtigung des Wasseranteils im Saccharosespeicherorgan (vorzugsweise erfolgt die Normierung auf Frischgewicht oder Trockengewicht).

Gemäß einem weiteren Aspekt betrifft die Erfindung ein Verfahren zur Herstellung von transgenen Pflanzen, wobei das Verfahren mindestens die folgenden Schritte umfasst:
(a) das Einbringen eines Nukleinsäuremoleküls gemäß dem ersten Aspekt und/oder eines Vektors oder mobilen genetischen Elements gemäß dem dritten Aspekt in mindestens eine Zelle einer Pflanze; und
(b) das Regenerieren der transgenen Pflanze aus der in Schritt (a) erhaltenen Pflanzenzelle.

Gemäß einer Ausführungsform ist die aus dem Verfahren hervorgehende transgene Pflanze in der Lage, Saccharose in den Vakuolen ihrer Zellen, vorzugsweise in den Vakuolen der Zellen ihres Saccharosespeicherorgans höher zu konzentrieren als eine isogene, unter identischen Bedingungen kultivierte Kontrollpflanze.

Im Sinne der vorliegenden Erfindung werden unter "isogenen Pflanzen bzw. Kontrollpflanzen" oder "isogenen Pflanzenzellen" diejenigen Pflanzen oder

Pflanzenzellen verstanden, die als Ausgangsmaterial zur Erzeugung der transgenen Pflanzen oder transgenen Pflanzenzellen verwendet wurden. Somit unterscheiden sich das Genom der transgenen Pflanzen und/oder Pflanzenzellen, insoweit es sich um gentechnisch veränderte Pflanzen oder Pflanzenzellen handelt, bis auf die gentechnisch übertragenen Gene und/oder eingebrachten Nukleotidsequenzen nicht voneinander.

Gemäß einer zusätzlichen und/oder alternativen Ausführungsform exprimiert oder überexprimiert die transgene Pflanze die für mindestens einen Protonen/Zucker-Antiporter-Protein kodierende Nukleotidsequenz in mindestens einer Zelle.

Das Einbringen des Nukleinsäuremoleküls, zum Beispiel auf dem Wege der Transformation, kann mit Techniken erfolgen, die dem Fachmann dem Grunde nach bekannt sind. Beispielsweise kann das Nukleinsäuremolekül durch Infektion eines Pflanzengewebes oder einer Pflanzenzelle mit *Agrobacterium tumefaciens,* welches die zu transferierende Nukleinsäuresequenz auf seinem in das pflanzliche Genom integrierbare Plasmid enthält, in die Pflanzenzellen eingebracht werden. Möglich ist auch ein Einbringen mittels biolistischem Transfer, bei dem die zu in die Pflanzenzelle einzubringende Nukleinsäure auf Goldpartikel oder Wolframpartikel, die anschließend mit hoher Geschwindigkeit in die Zellen geschossen werden, aufgebracht wird. Eine weitere dem Fachmann bekannte Möglichkeit, die Nukleinsäure in eine Pflanzenzelle einzubringen, besteht in der Protoplastentransformation, bei der den Protoplasten in Gegenwart der einzubringenden Nukleinsäuremoleküle entweder Polyethylenglykol hinzugefügt oder die Protoplasten einem kurzen Stromstoß ausgesetzt werden, so dass die Protoplastenmembran transient für die Nukleinsäuremoleküle durchlässig wird. Verfahren zur Regeneration von ganzen Pflanzen aus transformierten Gewebe oder Zellen sind dem Fachmann aus dem Stand der Technik ebenfalls bekannt.

Bevorzugt wird das Nukleinsäuremolekül gemäß dem ersten Aspekt, das rekombinanten Gen gemäß dem zweiten Aspekt und/oder der Vektor oder das mobile genetische Element gemäß dem dritten Aspekt stabil in das Genom der Zelle der Pflanze eingebracht. Das bedeutet, dass die transferierte Nukleinsäuresequenz stabil nach Regeneration einer Pflanze von dieser auf eine Nachkommenpflanze vererbt werden kann.

Vorzugsweise erfolgt die Transformation und die Regeneration von Zuckerrüben nach der von Lindsey beschriebenen Methode (Lindsey K. (1991) "Regeneration and transformation of sugar beet by Agrobacterium tumefaciens". Plant Tissue Culture Manual B7: 1-13, Kluwer Academic Publisher).

Die Transgenität der Pflanzen kann mittels Polymerase-Kettenreaktion unter Verwendung geeigneter Oligonukleotid-Primer überprüft werden. Nach der Regenerierung können die Transformanten zur Gewinnung von Saatgut im Gewächshaus angezogen und geselbstet werden.

In einer Ausführungsform handelt es sich bei den zu transformierenden Pflanzenzellen um Zellen monokotyledoner Pflanzen. In einer anderen Ausführungsform handelt es sich bei den zu transformierenden Pflanzenzellen um Zellen dikotyledoner Pflanzen. Gemäß einer weiteren und/oder zusätzlichen Ausführungsform handelt es sich bei den zu transformierenden Pflanzenzellen um Zellen einer Pflanze, die aus der Gruppe der Arten oder übergeordneten Gattungen ausgewählt ist, die *Beta vulgaris, Saccharum officinarum, Arenga saccharifera, Acer saccharum* und *Sorghum sp.* umfasst.

Gemäß einem weiteren Aspekt betrifft die Erfindung Verfahren zur Erhöhung der Saccharosekonzentration eines Saccharosespeicherorgans einer Pflanze durch Expression oder Überexpression eines tonoplastidären Protonen/Zucker-Antiporter-Proteins, insbesondere eines tonoplastidären Protonen/Saccharose-Antiporter-Proteins, in mindestens einer Zelle der Pflanze. Die Expression oder Überexpression kann durch genetische Modifikation von mindestens einer Zelle der Pflanze erfolgen und umfasst
(1) das Einbringen eines Nukleinsäuremoleküls gemäß dem ersten Aspekt, eines rekombinanten Gens gemäß dem zweiten Aspekt und/oder eines Vektors oder mobilen genetischen Elements gemäß dem dritten Aspekt in mindestens eine Zelle einer Pflanze, wodurch eine zusätzliche Expression oder eine Überexpression eines tonoplastidären Protonen/Zucker-Antiporter-Proteins bewirkt wird, oder
(2) das genetische Modifizieren eines endogenen regulatorischen Elements wie eines Promoters, welcher die Expression eines endogenen Gens, kodierend ein tonoplastidäres Protonen/Zucker-Antiporter-Protein, reguliert, beispielsweise durch Einfügen zusätzlicher cis-Elemente oder Enhancer, wodurch eine gesteigerte Expression des regulierten tonoplastidären Protonen/Zucker-Antiporter-Proteins bewirkt wird.

Durch die Expression oder Überexpression eines tonoplastidären Protonen/Zucker-Antiporter-Proteins, insbesondere eines tonoplastidären Protonen/Saccharose-Antiporter-Proteins, in mindestens einer Zelle der Pflanze wird der Import von Saccharose in die Vakuolen der genetisch modifizierten Zelle verbessert. Dadurch wird auch die Saccharosekonzentration in den Vakuolen dieser Zelle, verglichen mit einer isogenen Pflanzenzelle, erhöht.

Eine "Erhöhung der Saccharosekonzentration" oder eine "erhöhte Saccharosekonzentration" oder eine "höhere Saccharosekonzentration eines Saccharosespeicherorgans einer Pflanze" bedeutet eine Steigerung der durchschnittlichen Saccharosekonzentration, bezogen auf das Frischgewicht des Saccharosespeicherorgans im Vergleich mit einer unter identischen Bedingungen kultivierten, nicht-transgenen (isogenen) Kontrollpflanze von mindestens 0,2 %, 0,4 %, 0,6 %, 0,8 % oder 1 %, bevorzugt von mindestens 1,2 %, 1,4 %, 1,6 %, 1,8% oder 2 %, besonders bevorzugt von mindestens 2,5 %, 3 %, 3,5 %, 4 %, 4,5 %, 5 %, 6 %, 7 %, 8 %, oder 10%, und ganz besonders bevorzugt von mindestens 15 %.

Im Sinne der Erfindung wird unter dem Begriff "überexprimiert" verstanden, dass die Menge an tonoplastidärem Protonen/Zucker-Antiporter-Protein in einer Pflanze, Pflanzenzelle oder deren Tonoplasten höher als in der isogenen Pflanze, isogenen Pflanzenzelle oder deren Tonoplasten ist.

Gemäß einer Ausführungsform umfasst das Verfahren zur Erhöhung der Saccharosekonzentration eines Saccharosespeicherorgans einer Pflanze die Expression und/oder Überexpression der für ein tonoplastidäres Protonen/Zucker-Antiporter-Protein kodierenden Nukleotidsequenz eines Nukleinsäuremoleküls gemäß dem ersten Aspekt der Erfindung.

Zu diesem Zweck wird eine transgene Pflanze gemäß dem vorhergehend beschriebenen Verfahren hergestellt, wobei die Expression und/oder Überexpression des/der Protonen/Zucker-Antiporter-Proteine in der transgenen Pflanze wie oben beschrieben durch verschiedene genetische Modifikationen ermöglicht werden kann.

So kann ferner beispielsweise ein Konstrukt aus einem starken Promotor und einer Nukleotidsequenz gemäß dem ersten Aspekt der Erfindung in eine zu transformierende Pflanzenzelle eingebracht werden. Alternativ kann der endogene Promotor eines für ein tonoplastidäres Protonen/Zucker-Antiporter-Protein kodierenden Gens, insbesondere eines für ein tonoplastidäres Protonen/Saccharose-Antiporter-Protein kodierenden Gens, derart modifiziert werden, dass er in der transgenen Pflanze stärker aktiv ist als in der isogenen Kontrollpflanze. Mittel zur Modifikation eines endogenen Promotors können beispielsweise TALENs oder Zinkfinger-Nukleasen sein. Gemäß einer weiteren Alternative können zusätzliche Genkopien des endogenen für ein tonoplastidäres Protonen/Zucker-Antiporter-Protein kodierenden Gens, insbesondere des endogenen für ein tonoplastidäres Protonen/Saccharose-Antiporter-Protein kodierenden Gens, einschließlich seines natürlichen Promotors, in die Pflanzenzelle eingebracht werden.

In einer alternativen und/oder zusätzlichen Ausführungsform ist das tonoplastidäre Protonen/Saccharose-Antiporter-Protein aus der Gruppe ausgewählt, die BvTST2.1-Proteine, Homologe, Analoge und Orthologe davon umfasst.

Gemäß einem weiteren Aspekt betrifft die Erfindung Verfahren zum Identifizieren einer Pflanze, die geeignet ist, eine erhöhte Saccharosekonzentration in ihrem Saccharosespeicherogan zu erzeugen.

Gemäß einer Ausführungsform können die zu identifizierenden Pflanzen einer markergestützten Identifizierung unterworfen werden. Hierfür wird die DNA jeder zu untersuchenden Pflanze isoliert und entweder einer Polymerasekettenreaktion (PCR) mit geeigneten Oligonukleotid-Primern unterworfen, so dass aus der Analyse der Reaktionsprodukte der PCR, sei es über Gelchromatographie oder mittels Fluoreszenznachweis wie bei RT-PCR, diejeingen Pflanzen identifiziert werden können, die aufgrund ihrer genetischen Ausstattung geeignet sind, eine erhöhte Saccharosekonzentration in ihrem Saccharosespeicherogan zu erzeugen. Gemäß einer zusätzlichen und/oder alternativen Ausführungsform kann die genetische Ausstattung der zu identifizierenden Pflanzen auch mittels eines Restriktionslängepolymorphismus erfolgen, bei der die isolierte DNA mit verschiedenen Restiktionsendonukleasen hydrolysiert wird, die Restriktionsfragmente gelchromatographisch aufgetrennt, geblottet und mit einer geeigneten Sonde hybridisiert werden. Geeignete exemplarische Oligonukleotide für eine Identifikation von transgenen Pflanzen, die geeignet sind, eine erhöhte Saccharosekonzentration in ihrem Saccharosespeicherorgan zu erzeugen, weil sie die Nukleotidsequenz gemäß SEQ ID NO: 2 exprimieren oder überexprimieren, können aus der Gruppe von Oligonukleotiden, umfassend SEQ ID NO: 15 bis SEQ ID NO: 26, entnommen werden. Dem Fachmann ist bekannt wie er geeignete Oligonukleotide auch für Homologe, Analoge oder Orthologe der SEQ ID NO: 2 bereitstellen kann.

Gemäß einer zusätzlichen und/oder alternativen Ausführungsform erfolgt die Identifikation der Pflanzen, die geeignet sind, eine erhöhte Saccharosekonzentration in ihrem Saccharosespeicherogan zu erzeugen, nicht anhand ihrer genetischen Ausstattung, sondern anhand der Expression ihrer tonoplastidären Protonen/Saccharose-Antiporter-Proteine. Dies kann beispielsweise auf Ebene der mRNA erfolgen, indem die Menge an mRNA der für die tonoplastidären Protonen/Zucker-Antiporter-Proteine kodierenden Desoxyribonukleotidsequenzen, insbesondere der für die tonoplastidären Protonen/Saccharose-Antiporter-Proteine kodierenden Desoxyribonukleotidsequenzen, bestimmt wird, beispielsweise durch "real-time quantitative PCR". Die Bestimmung einer größeren Menge an mRNA, kodierend für mindestens ein oben beschriebenes tonoplastidäres Protonen/Zucker-Antiporter-Protein in einer Pflanze, in einem Pflanzengewebe oder einer Pflanzenzelle, insbesondere in einem Gewebe oder einer Zelle des Saccharosespeicherorgans der Pflanze, im Verhältnis zu einer Vergleichspflanze derselben Spezies oder einem Teil davon oder im Verhältnis zu einem anderen Pflanzengewebe oder einer Pflanzenzelle derselben Pflanze, welche nicht Teil des Saccharosespeicherorgans der Pflanze sind, gilt als Nachweis für die Eignung einer Pflanze eine erhöhte Saccharosekonzentration in ihrem Saccharosespeicherogan zu erzeugen.

Eine Identifikation der Pflanzen, die geeignet sind, eine erhöhte Saccharosekonzentration in ihrem Saccharosespeicherogan zu erzeugen, kann auch durch den quantitativen Nachweis der Menge an tonoplastidären Protonen/Zucker-Antiporter-Protein, insbesondere an tonoplastidären Protonen/Saccharose-Antiporter-Protein, in einem Pflanzenteil erfolgen. Dafür wird ein sogenannter Western-Blot verwendet, bei dem die elektrophoretisch aufgetrennten Proteine des Pflanzenteils, vorzugsweise der Vakuolen, besonders bevorzugt der Vakuolenmembran dieses Teils mit einem für ein oder mehrere oben beschriebene tonoplastidäre Protonen/Zucker-Antiporter-Proteine spezifischen Antikörper inkubiert werden. Mittels eines sekundären Antikörpers, der den für ein oder mehrere oben beschriebene tonoplastidäre Protonen/Zucker-Antiporter-Proteine spezifischen Antikörper bindet, und der eine nachweisbare Markierung aufweist, kann die Menge an tonoplastidären Protonen/Zucker-Antiporter-Protein, insbesondere an tonoplastidären Protonen/Saccharose-Antiporter-Protein, in dem Pflanzenteil bestimmt und die Pflanzen identifiziert werden, die geeignet sind, eine erhöhte Saccharosekonzentration in ihrem Saccharosespeicherogan zu erzeugen. Die Bestimmung einer größeren Menge von mindestens einem tonoplastidären Protonen/Saccharose-Antiporter-Protein in einer Pflanze, in einem Pflanzenteil oder einer Pflanzenzelle, insbesondere in einem Gewebe oder einer Zelle des Saccharosespeicherorgans der Pflanze, im Verhältnis zu einer Vergleichspflanze derselben Spezies oder einem Teil davon oder im Verhältnis zu einem anderen Pflanzengewebe oder einer Pflanzenzelle derselben Pflanze, welche nicht Teil des Saccharosespeicherorgans der Pflanze sind, gilt als Nachweis für die Eignung einer Pflanze eine erhöhte Saccharosekonzentration in ihrem Saccharosespeicherogan zu erzeugen.

Offenbart sind auch die mit dem vorgenannten Verfahren identifizierten Pflanzen, die geeignet sind, eine erhöhte Saccharosekonzentration in ihrem Saccharosespeicherorgan zu erzeugen.

Gemäß einem weiteren Aspekt betrifft die Erfindung Oligonukleotide, die zur Verwendung als molekulare Marker für den diagnostischen Nachweis eines Nukleinsäuremoleküls gemäß dem ersten Aspekt geeignet sind.

Gemäß einer Ausführungsform ist mindestens eines der geeigneten Oligonukleotide aus der Gruppe ausgewählt, die die Oligonukleotide gemäß SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25 und SEQ ID NO: 26 umfasst. Diese können als molekulare Marker für den diagnostischen Nachweis eines Nukleinsäuremoleküls mit einer Nukleotidsequenz gemäß SEQ ID NO: 2 verwendet werden.

Weiterhin offenbart ist ein Antikörper die diagnostisch für ein Protein ist, das als tonoplastidärer Protonen/Zucker-Antiporter, vorzugweise als tonoplastidärer Protonen/Saccharose-Antiporter, funktioniert.

In einer Ausführungsform ist der diagnostische Antikörper ein monoklonaler Antikörper. In einer alternativen Ausführungsform ist der diagnostische Antikörper Teil eines polyklonalen Antiserums.

In einer zusätzlichen und/oder alternativen Ausführungsform ist der diagnostische Antikörper beziehungsweise das polyklonale Antiserum spezifisch für ein bestimmtes tonoplastidäres Protonen/Zucker-Antiporter-Protein wie ein tonoplastidäres Protonen/Saccharose-Antiporter-Protein. Vorzugsweise erkennt und bindet der diagnostische Antikörper ein Epitop auf der Schleife zwischen der sechsten und der siebten Transmembrandomäne eines Protonen/Saccharose-Antiporter-Proteins

Gemäß einem weiteren Aspekt betrifft die Erfindung die Verwendung eines tonoplastidären Protonen/Zucker-Antiporter-Proteins zur Erhöhung der Saccharosekonzentration eines Saccharosespeicherorgans einer Pflanze.

Gemäß einer Ausführungsform umfasst die Verwendung eines tonoplastidären Protonen/Zucker-Antiporter-Proteins zur Erhöhung der Saccharosekonzentration eines Saccharosespeicherorgans einer Pflanze die Erhöhung der Saccharosekonzentration durch Expression oder Überexpression eines Nukleinsäuremoleküls, welches für das tonoplastidäre Protonen/Zucker-Antiporter-Protein kodiert. Vorzugsweise umfasst das Nukleinsäuremolekül
i. ein Nukleinsäuremolekül mit einer Nukleotidsequenz gemäß SEQ ID NO: 2, 4, 6, 8, 10, 12 oder 14, oder mit einer Nukleotidsequenz, die eine Identität von mindestens 80% zu einer der Nukleotidsequenzen gemäß SEQ ID NO: 2, 4, 6, 8, 10, 12 oder 14 aufweist;
ii. ein Nukleinsäuremolekül mit einer Nukleotidsequenz, die komplementär zu einer der Nukleotidsequenzen gemäß i. ist;
iii. ein Nukleinsäuremolekül, das mit einem der Nukleinsäuremoleküle gemäß i. oder ii. hybridisiert; oder
iv. ein Nukleinsäuremolekül, das ein Polypeptid mit einer Aminosäuresequenz gemäß SEQ ID NO: 1, 3, 5, 7, 9, 11 oder 13 kodiert, oder das ein Polypeptid mit einer Aminosäuresequenz kodiert, die eine Identität von mindestens 80% zu einer der Aminosäuresequenzen gemäß SEQ ID NO: 1, 3, 5, 7, 9, 11 oder 13 aufweist.

Das Nukleinsäuremolekül gemäß SEQ ID NO: 2 kodiert für den tonoplastidären Protonen/Zucker-Antiporter TST2.1 von *Beta vulgaris* mit der Aminosäuresequenz gemäß SEQ ID NO: 1.

Das Nukleinsäuremolekül gemäß SEQ ID NO: 4 kodiert für den tonoplastidären Protonen/Zucker-Antiporter TST1 von *Beta vulgaris* mit der Aminosäuresequenz gemäß SEQ ID NO: 3.

Das Nukleinsäuremolekül gemäß SEQ ID NO: 6 kodiert für den tonoplastidären Protonen/Zucker-Antiporter TST2.2 von *Beta vulgaris* mit der Aminosäuresequenz gemäß SEQ ID NO: 5.

Das Nukleinsäuremolekül gemäß SEQ ID NO: 8 kodiert für den tonoplastidären Protonen/Zucker-Antiporter TST3 von *Beta vulgaris* mit der Aminosäuresequenz gemäß SEQ ID NO: 7.

Das Nukleinsäuremolekül gemäß SEQ ID NO: 10 kodiert für den tonoplastidären Protonen/Zucker-Antiporter TMT1 von *Arabidopsis thaliana* mit der Aminosäuresequenz gemäß SEQ ID NO: 9.

Das Nukleinsäuremolekül gemäß SEQ ID NO: 12 kodiert für den tonoplastidären Protonen/Zucker-Antiporter TMT2 von *Arabidopsis thaliana* mit der Aminosäuresequenz gemäß SEQ ID NO: 11.

Das Nukleinsäuremolekül gemäß SEQ ID NO: 14 kodiert für den tonoplastidären Protonen/Zucker-Antiporter TMT3 von *Arabidopsis thaliana* mit der Aminosäuresequenz gemäß SEQ ID NO: 13.

Durch die Expression und/oder Überexpression mindestens einer der unter i. bis iv. genannten Nukleotidsequenzen in einer Pflanze nach deren Einbringen in mindestens eine Zelle der Pflanze kann die Menge an Protonen/Zucker-Antiporter-Protein in der Vakuolenmembran dieser Pflanze erhöht werden, insbesondere in den Membranen der Vakuolen der Saccharosespeicherorgane dieser Pflanze, so dass mehr Saccharose in die Vakuolen der Pflanze transportiert werden kann und die Saccharosekonzentration in dem Saccharosespeicherorgan der Pflanze, verglichen mit einer unter identischen Bedingungen kultivierten isogenen Kontrollpflanze, erhöht ist. Dadurch lässt sich der Saccharose-Ertrag pro Pflanze, pro Saccharosespeicherorgan und/oder pro Anbaufläche steigern.

Die vorliegende Erfindung wird nachfolgend anhand von Ausführungsbeispielen veranschaulicht, wobei die Ausführungsbeispiele nur zur Erläuterung dienen, aber die vorliegende Erfindung nicht einschränken. Die vorliegende Erfindung wird ausschließlich durch die Ansprüche definiert. Der Ausdruck "ein" oder "eine" ist nicht als die Anzahl spezifizierend zu verstehen.

Die Ausführungsbeispiele zeigen deutlich, dass das TST2.1 von *Beta vulgaris* das tonoplastidäre Membranprotein ist, welches als Protonen/Zucker-Antiporter hochspezifisch Saccharose in die Vakuole einer Pflanzenzelle importieren kann.

### Beispiel 1: Pflanzenmaterial und Wachstumsbedingungen

Für die nachfolgenden Versuche wurden Zuckerrüben der Sorten "Belladonna KWS" und "Brigadier" verwendet. Das Saatgut für die Sorte "Belladonna KWS" wurde von der KWS Saat AG, Einbeck, DE zur Verfügung gestellt, das Saatgut für Rüben der Sorte "Brigadier" wurden im örtlichen Saatenhandel käuflich erworben.

Ferner wurden Pflanzen und Pflanzenzellen von *Nicotiana benthamiana* und *Arabidopsis thaliana* verwendet. Die Pflanzen wuchsen in Wachstumskammern auf dem Standardsubstrat ED-73 der Firma Einheitserde- und Humuswerke Gebr. Patzer GmbH & Co. KG bei einem Hell-Dunkel-Zyklus von 10 Stunden Licht und 14 Stunden Dunkelheit, 22 °C und 125 µmol quanta m⁻²s⁻¹.

Die *Arabidopsis Attst*1-2 T-DNA Doppel-Genknockout-Mutante wurde im Stand der Technik beschrieben (Wormit, A. et al. (2006) "Molecular identification and physiological characterization of a novel monosaccharide transporter from Arabidopsis involved in vacuolar sugar transport", Plant Cell 18, 3476-3490). Für die Wachstumsversuche mit 2-Deoxyglucose wurden oberflächensterilisierte *Arabidopsis-*Samen auf halb konzentrierten Murashige und Skoog (½MS) Agarplatten wie beschrieben ausgesät, (Reiser, J. et al. (2004) "Molecular physiological analysis of the two plastidic ATP/ADP transporters from Arabidopsis", Plant Physiol. 136: 3524-3536). Die Selektion der pUBQ:*Bv*TST2.1-GFP und 35S:*Bv*TST1 überexprimierenden Pflanzen erfolgte auf ½MS-Agarplatten, die entweder 50 µg/ml Hygromycin oder 40 µg/ml Kanamycin enthielten.

### Beispiel 2: Quantitative Bestimmung von Zuckern in Geweben der Zuckerrübe

Pfahlwurzelgewebe der Zuckerrüben wurde mit einem Gemüsehobel geerntet, sofort in flüssigem Stickstoff eingefroren und bis zur quantitativen Zuckerbestimmung bei -80 °C gelagert. Für die Bestimmung des Zuckergehalts wurde das Pflanzengewebe in flüssigem Stickstoff gemörsert und 50 µg gemahlenes Gewebe zweimal für 20 Minuten bei 80 °C mit 80%-igem Ethanol extrahiert. Die Überstände wurden vereinigt und mit einer SpeedVac (Firma Eppendorf, Hamburg, DE) eingedampft. Die getrockneten Zucker wurden in Wasser gelöst und mittels eines NADP-gekoppelten enzymatischen Tests in einem Mikrotiterplatten-Lesegerät wie beschreiben quantifiziert (Bergmeyer, H. U. and Bernt, E. (1974) "Methods of Enzymatic Analysis", vol.3, Bergmeyer,H.U. ed., Verlag-Chemie New York, pp.1176-117; Lee, Y.C. (1972) "α-Mannosidase, β-glucosidase, and β-galactosidase from sweet almond emulsion". Methods Enzymol. 28: 699-702).

### Beispiel 3: Genexpressionsanalyse

Die relative Akkumulation von mRNA erfolgte durch Northern-Blot-Analysen wie beschrieben (Jung, B. et al. (2011) "Arabidopsis nucleoside hydrolases involved in intracellular and extracellular degradation of purines". Plant J. 65: 703-711). Quantitative RT-PCR wurde durchgeführt wie früher beschrieben (Leroch M. et al. (2005) "Identification and characterization of a novel plastidic adenine nucleotide uniporter from Solanum tuberosum". J. Biol. Chem. 280: 17992-18000). Die genspezifischen Primer, die verwendet wurden, sind in Tabelle 1 aufgeführt:

**Tabelle 1: Genspezifische Primer für die Amplifikation der für BvTST1 und BvTST2.1 kodierenden Nukleotidsequenzen sowie für die quantitative PCR zur Expressionsanalyse der 4 paralogen TST-Gene aus Beta vulgaris.**

| Bezeichnung | Nukleotidsequenz | SEQ ID NO: |
|---|---|---|
| BvTST1 GWfw | | 15 |
| BvTST1GW rev | | 16 |
| BvTST2.1fw_XhoI | CTCGAGATGAGTGCAGCAGTATTAG | 17 |
| BvTST2.1 rev_XbaI | TCTAGAGTGGCTTGCTTGTCTTGCACC | 18 |
| qPCRfwTST1 | GCTGTTGCTATGAGGCTCATGGA | 19 |
| qPCRrevTST1 | CCTTAGCGGCTTCTAACTGTTTAGG | 20 |
| qPCRfwTST2.1 | AAAGATGAACACCACTGTGTATG | 21 |
| qPCRrevTST2.1 | GTCATCAGTGGCTTGCTTGTCTTG | 22 |
| qPCRfwTST2.2 | AAAGATGAGCACTACTGTGCACG | 23 |
| qPCRrevTST2.2 | TCAGTTGTCCTTGTCTTCAGAAGG | 24 |
| qPCRfwTST3 | TCTACTTCTGCTGCTTTGTCATGG | 25 |
| qPCRrevTST3 | TCAGCTTCAGCTTGCCTTGCA C | 26 |
| Bvef1α_fw | CCACATTGCTGTCAAGTTTGCTG | 27 |
| Bvef1α_rev | TGGTAACCTTGGCACCGGTTG | 28 |

### Beispiel 4: Isolierung von Vakuolen und Tonoplastenmembran aus Pfahlwurzelgewebe

Vakuolen wurden nach dem Verfahren gemäß Leigh und Branton (Leigh, R. A. and Branton, D. (1976). "Isolation of Vacuoles from Root Storage Tissue of Beta vulgaris", L. Plant Physiol. 58: 656-662) mit den folgenden Veränderungen durchgeführt: Pfahlwurzelgewebe wurde mit einem Gemüsehobel in Scheiben von 0,1 bis 0,2 mm Dicke geschnitten, die sofort in einem Sammelmedium (1M Sorbitol, 1mM DTT, 5 mM EDTA, 50 mM Tris-HCl, pH, 7,6) bei Raumtemperatur inkubiert wurden. Nachfolgend wurden die dünnen Scheiben des Pfahlwurzelgewebes mit einer Rasierklinge in dem Sammelmedium (1 M Sorbitol, 1 mM DTT, 5 mM EDTA, 50 mM Tris-HCl, pH 7,6) zerkleinert, durch ein Edelstahlsieb (100 µm Maschenweite) filtriert und durch Zentrifugation (2.000 x g, 20 min., 4°C) sedimentiert. Das Sediment wurde in Sammelmedium mit 30% Nycodenz (Axis-Shield GmbH, Heidelberg, DE) resuspendiert und in 17 ml Zentrifugationsröhrchen (Beckmann UltraClear) überführt. In der nachfolgenden Ausschwingzentrifugation (1.500 x g, 15 min., 8°C) bildete das Nycodenz einen Dichtegradienten aus und die Vakuolen schwammen auf der oberen Phase des Dichtegradienten.

Die Membranen der Vakuolen wurden wie im Stand der Technik beschrieben isoliert (Schulze W.X. et al. (2012) "Cold acclimation induce changes in Arabidopsis tonoplast protein abundance and activity and alters phosphorylation of tonoplast monosaccharide transporters", Plant. J. 69: 529-541). Die Aktivität von α -Mannosidase in mit Ultraschall behandelten Vakuolen erfolgte wie anderweitig beschrieben (Boller, T. and Kende, H. (1979) "Hydrolytic enzymes in the central vacuole of plant cells". Plant Physiol. 63: 1123-1132; Lee, Y.C. (1972) "α-Mannosidase, β-glucosidase, and β-galactosidase from sweet almond emulsion". Methods Enzymol. 28: 699-702)

### Beispiel 5: Flüssigchromatographie und Tandem-Massenspektrometrie

Die Sedimente isolierter Tonoplasten-Membranen von 2 oder 5 Monate alten Pflanzen wurden in Puffer (4% SDS, 50 mM NH₄HCO₃) in einer Konzentration von 1 µg/ml aufgenommen. Die aufgenommenen Proteine wurden über Nacht bei - 20°C in 80% Aceton ausgefällt und weiter bearbeitet wie von Mühlhaus beschrieben (Mühlhaus, T. et al. (2011) "Quantitative shotgun proteomics using a uniform 15N-labeled standard to monitor proteome dynamics in time course experiments reveals new insights into the heat stress response of Chlamydomonas reinhardtii", Mol. Cell. Proteomics 10: M110 004739). Die extrahierten Peptide wurden in 200 µl Puffer (2% Acetonitril, 0,4% Essigsäure) resuspendiert.

Proben zu je 3 µl der extrahierten Peptide wurden der Flüssigchromatographie-Tandem-Massenspektrometrie (LC-MS/MS Analyse) zugeführt. Die chromatographische Trennung erfolgte auf einer nanoAquity UPLC (Waters, Eschborn, DE) mittels einer "Symmetry C18 trap column (5 mm Partikelgröße, 180 µm x 20 mm Säulenmaß) und einer BEH 130 C18-Säule (1,7 µm Partikelgröße, 75 mm x 150 mm Säulenmaße). Eluiert wurde mit einem doppelten Gradienten, zunächst von 100% Puffer A (0,4% Essigsäure, 1% 2-Propanol, 2% Acetonitril) auf 40 % Puffer B (0,4% Essigsäure, 1% 2-Propanol, 90% Acetonitril) innerhalb von 2 oder 3 Stunden, anschließend auf 90% Puffer B innerhalb von 5 min. und abschließend 15 min mit 90% Puffer B. Die Säule wurde am Ende für 15 min. mit 100% Puffer A wieder equilibriert. Das Hybrid LTQ XL-Orbitrap Massenspektrometer (ThermoScientific, Hamburg, DE) arbeitete im datenabhängigen Modus mit einem Zyklus einer vollständigen Abtastung des Massenspektrums 300 - 1500 m/z (Orbitrap) bei einer eingestellten Auflösung von 60.000 bei 400 m/z, gefolgt von sieben aufeinanderfolgenden datenabhängigen MS² Abtastungen (LTQ) der intensivsten Ionen. Einzeln geladene Ionen wurden von der MS² Analyse ausgeschlossen und die Ausgangsionen für die MS² Analyse wurden für 20 s auf eine Ausschlussliste gesetzt. Jede Probe wurde dreifach analysiert.

Proteine wurden mit Hilfe der MaxQuant Software und der Andromeda Suchmaschine (Cox, J. and Mann, M. (2008) "MaxQuant enables high peptide identification rates, individualized p.p.b.-ränge mass accuracies and proteomewide protein quantification". Nat. Biotechnol. 26: 1367-72) in einer im Hause eines der Erfinder erstellten Datenbank für Zuckerrübenproteine identifiziert.

### Beispiel 6: Nukleinsäurekonstrukte

Komplementäre DNA (cDNA) von *Beta vulgaris* wurde durch reverse Transkription von aus Pfahlwurzeln oder Blättern isolierter RNA hergestellt. Alle Polymerase Kettenreaktionen (PCR) wurden mit der Phusion-HF DNA Polymerase (Thermo Scientific) durchgeführt.

Das pUBQ:*Bv*TST1-GFP Fusionskonstrukt wurde unter Verwendung des Vektors pUBC-GFP-Dest (Grefen et al. (2010) "A ubiquitin-10 promoter-based vector set for fluorescent protein tagging facilitates temporal stability and native protein distribution in transient and stable expression studies", Plant J. 64: 355-365) hergestellt. Dafür wurde die cDNA von *Bv*TST1 amplifiziert und das Stopcodon mittels PCR unter Verwendung der *Bv*TST1 Primer, die die attB1 und attB2-Stellen enthalten, entfernt. Das Amplifikationsprodukt wurde über eine BP-Reaktion in pDONRZEO (Invitrogen, Heidelberg, DE) kloniert, gefolgt von einer LR-Reaktion in pUBC-GFP-Dest.

Das pUBQ:*Bv*TST2.1-GFP Konstrukt wurde wie folgt hergestellt: Der gesamte offene Leserahmen des *Bv*TST2.1-Gens wurde mit den Primern *Bv*TST2.1fw_XhoI/*Bv*TST2.1rev_XbaI amplifiziert. Das erhaltene PCR-Produkt wurde mit XhoI und XbaI verdaut und in den mit XhoI und SpeI geöffneten Vektor pUBC-cGFP-Dest (Grefen et al. (2010)) ligiert. Das so erzeugte Konstrukt enthält das bar-Gen, das in transformierten Pflanzen zu einer Basta-Resistenz führt. Anschließend wurde die vollständige für *Bv*TST2.1-GFP kodierende Nukleotidsequenz mit XhoI/PstI aus diesem Konstrukt herausgeschnitten und in einen entsprechend mit XhoI und PstI geöffneten Vektor pUBN-nYFP-Dest inseriert, der eine Hygromycinresistenz in transformeirten Pflanzen vermittelt. Ein Verdau von pUBN-nYFP-Dest mit XhoI/PstI führte zu einem vollständigen Entfernen der nYFP-Sequenz und der "Gateway"-Eigenschaften des Zielvektors, so dass dieser für die Transformation der *At*tst1-2 Doppel-Genkonckout-Mutanten mittels Agrobacterien geeignet sind (Clough S.J., Bent, A.F. (1998) "Floral dip: a simplified method for Agrobacterium-mediated transformation of Arabidopsis thaliana". Plant J. 16: 735-743). Die Nukleotidsequenzen aller erzeugten Genkonstrukte wurden mittels Sequenanalyse überprüft.

### Beispiel 7: Patch-Clamp Untersuchungen von Vakuolen transformierter Nicotiana benthamina-Pflanzen

Für die transiente Überexpression von an ihren C-terminalen Enden mit dem grün fluoreszierenden Protein (GFP) markierten Zuckertransport-Proteinen (*Bv*TST1-GFP und *Bv*TST2.1-GFP) oder nur mit GFP unter der Kontrolle des Ubiquitin-Promotors (pUBQ10) in Mesophyllzellen von *N. benthamiana* wurde die das Latz et al (2007) beschriebene Verfahren der Agroinfiltration bei 5 bis 7 Wochen alten Pflanzen verwendet (Latz et al. (2007) "In planta AKT2 subunits constitute a pHand Ca2+-sensitive inward rectifying K+ channel", Planta, 225: 1179-1191). Abweichend von dem im Stand der Technik beschriebenen Verfahren wurde der *Agrobacterium tumefaciens* Stamm GV3101 als Träger der für das Gen 19K kodierenden Nukleotidsequenz sowie für die entsprechenden Zuckertransportprotein/GFP-Konstrukte verwendet. Die Bakterien wurden über Nacht in 5 ml YEB Medium kultiviert, bei 8.000 x g für 1 min bei Raumtemperatur abzentrifugiert und 2-mal mit Agromix gewaschen (Latz et al. (2007)). Die Bakterienzellen wurden in 3 ml Agromix resuspendiert und für 2 bis 3 Stunden bei 28°C im Dunkeln gehalten. Für die Infiltration wurde 1 ml der Suspension mit den 19K enthaltenden Agrobakterien mit 1 ml der Suspension an Agrobakterien, die pUBQ:*Bv*TST1-GFP, pUBQ:*Bv*TST2.1-GFP oder pUBQ:GFP enthalten, gemischt und mit 2 ml Agromix versetzt.

Zwei Tage nach der Agroinfiltration wurden die Protoplasten der Mesophyllzellen isoliert, im Wesentlichen wie von Beyhl et al. beschrieben (Beyhl, D. et al. (2009) "The fou2 mutation in the major vacuolar cation channel TPC1 confers tolerance to inhibitory luminal calcium", Plant J. 58: 715-723). Nach der Enzyminkubation von Blattscheiben für 1 Stunde wurden die freigesetzten Protoplasten mit 500 mM Sorbitol und 1 mM CaCl₂ gewaschen. Die Vakuolen wurden direkt in den Patch-Clamp-Kammern aus den Protoplasten freigesetzt, indem sie einem Lysepuffer mit einer Osmolarität von 280 mOsmol x kg⁻¹ (10 mM EGTA, 10 mM Hepes/Tris, pH 7,4; Osmolarität mit D-Sorbitol eingestellt) ausgesetzt wurden. Makroskopische Ströme wurden in der "whole-vacuolar"-Konfiguration gemessen (Beyhl, D. et al. (2009) "The fou2 mutation in the major vacuolar cation channel TPC1 confers tolerance to inhibitory luminal calcium". Plant J. 58: 715-723); und bei 100 Hz tiefpassgefiltert. Das Bad und die Pipettenlösung waren bis auf den pH-Wert in Bezug auf ihre Zusammensetzungen identisch (100 mM KCl, 2 mM MgCl₂, 1mM CaCl₂, 450-500 Osmol x kg⁻¹, eingestellt mit D-Sorbitol). Der pH-Wert des Bades wurde auf 7,4 (Hepes/Tris) und der pH-Wert der Pipettenlösung auf 5,5 (Mes/Tris) eingestellt. Um einen zuckerinduzierten Protonenfluss zu messen, wurden Glucose oder Saccharose zur cytoplasmatischen Seite der Vakuolenmembran gegeben, jeweils in einer Endkonzentration von 50 mM.

### Beispiel 8: Analyse des Membranproteoms der Vakuolen aus Zellen der Pfahlwurzel der Zuckerrübe

Um die Proteinausstattung der Vakuolenmembran von Pfahlwurzelzellen der Zuckerrübe zu analysieren, wurden die Vakuolen der Pfahlwurzelzellen von fünf Monate alten Zuckerrüben (*Beta vulgaris*) der Sorte "Belladonna KWS" isoliert und die Vakuolenmembran mittels Hochgeschwindigkeitszentrifugation angereichert. Die hydrophoben Membranproteine wurden mit Aceton aus der mehrfach gewaschenen Tonoplastenfraktion ausgefällt, nachfolgend in einer Harnstofflösung (8 M Harnstoff) resuspendiert und vor der LC-MS/MS-Analyse einem tryptischen Verdau unterzogen.

Insgesamt wurden etwa 400 unterschiedliche Proteine in jeder der angereicherten Tonoplastenpräparationen identifiziert. Eines dieser Proteine, nachfolgend BvTST2.1 genannt (SEQ ID NO: 1), war in allen unabhängig voneinander durchgeführten Präparationen in großer Menge vorhanden, wies die Signatur eines Zuckertransporters auf ([LIVMSTAG] - [LIVMFSAG] - (SH) - (RDE) - [LIVMSA] - [DE] - (TD) - [LIVMFYWA] - G - R - [RK] - x (4,6) - [GSTA]; Prosite Pattern PS00216, http://prosite.expasy.org/) und hatte die höchste Ähnlichkeit zum vakuolären Monosaccharid-Transporter TMT2 aus *Arabidopsis thaliana* (Fig. 1)

### Beispiel 9: Gene für tonoplastidäre Zuckertransportproteine im Zuckerrübengenom

Beim Durchsuchen des Genoms von *B. vulgaris* konnten 4 paraloge Gene identifiziert werden, die für tonoplastidäre Zuckertransportproteine kodieren. Die phylogenetische Analyse (Fig. 2) zeigte, dass die Zuckertransporter *Bv*TST1 und *Bv*TST3 am nächsten mit den orthologen Genen *At*TMT1 bzw. *At*TMT3 von *Arabidopsis* verwandt sind, während *Bv*TST2.1 und *Bv*TST2.2, ein sehr ähnliches Paar von Genen, die größte Sequenzähnlichkeit zum Arabidopsis Ortholog *At*TMT2 aufweisen (Fig. 1). Die Aminosäuresequenz von *Bv*TST2.1 stimmt zu etwa 68 % mit der von *At*TMT2 überein und die Ähnlichkeit liegt bei 84% (Fig. 1).

### Beispiel 10: Subzelluläre Lokalisation von BvTST2.1

Die subzelluläre Lokalisation von *Bv*TST2.1 wurde in stabil mit pUBQ:*Bv*TST2.1-GFP transformierten *Attst*1-2 Doppel-Genknockout-Mutanten untersucht.

Die Isolierung von Protoplasten aus Blattmesophyllzellen und die Freisetzung von Vakuolen erfolgte nach einem bekannten Verfahren (Yoo, S.D. et al. (2007) "Arabidopsis mesophyll protoplasts: a versatile cell system for transient gene expression analysis", Nat Protocol 1565-1572).

Ein konfokales Laserscanning Mikroskop (Leica TCS SP5, Leica Microsystems, Wetzlar, DE) wurde für fluoreszenzmikroskopische Aufnahmen verwendet. Alle Aufnahmen wurden mit einem Leica HCX PL APO 63x/1.20w motCORR CS Objektiv gemacht. Die Bildbearbeitung erfolgte mit der Leica Application-Suite Advanced Fluorescence Lite Software.

Nach der Klonierung der vollständigen *Bv*TST2.1 mRNA wurde die subzelluläre Lokalisation des Proteins bestimmt, indem ein *Bv*TST2.1-GFP Fusionsprotein stabil in *Arabidopsis* exprimiert wurde. Die grüne Fluoreszenz, die in Mesophyllzellen der Blätter von *Bv*TS2.1-GFP stabil exprimierenden *Arabidopsis-*Mutanten beobachtet wurde, zeigte an, dass das Fusionsprotein in der Membran der Vakuolen, die die Chloroplasten eng umgaben, lokalisiert war.

Ein enzymatischer Verdau des Mesophyllgewebes von *Bv*TST2.1-GFP exprimierenden Pflanzen führte zu einzelnen intakten Protoplasten. Die nachfolgende hyposomotische Behandlung dieser Protoplasten führte zur Freisetzung stabiler, grün fluoreszierender Vakuolen, wodurch die Lokalisation von *Bv*TST2.1-GFP im Tonoplasten bestätigt wurde.

### Beispiel 11: Korrelation der BvTST2.1 Expression und der SaccharoseKonzentration in Pfahlwurzeln der Zuckerrübe

Um eine mögliche Korrelation zwischen der Expression von *Bv*TST2.1 in den Pfahlwurzeln der Zuckerrübe und der Saccharose-Konzentration der Zuckerrübe zu eruieren, wurden die Expression des *Bv*TST2.1-Gens den Zuckerrüben-Sorten "Belladonna KWS" und "Brigadier" bestimmt.

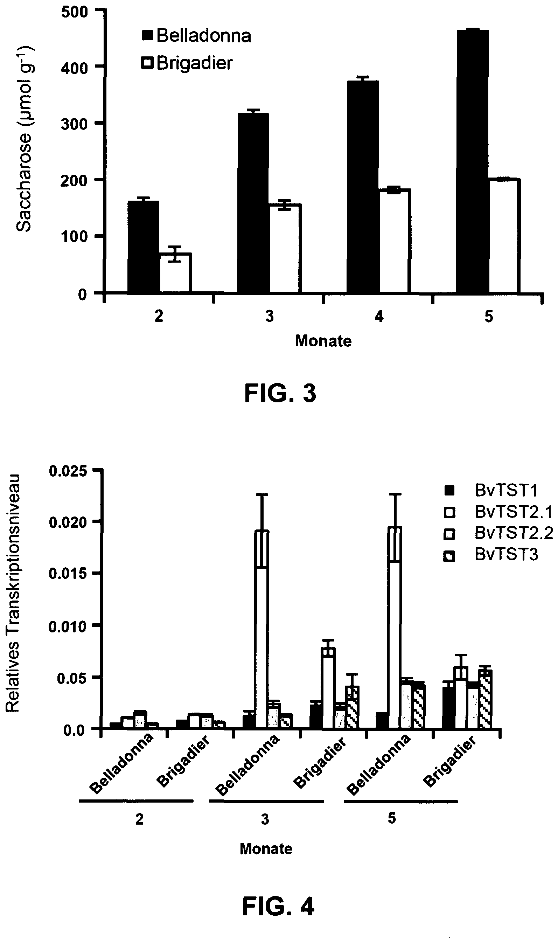

Die Sorte "Belladonna KWS" ist bekannt als eine Zuckerrüben-Sorte, die eine hohen Saccharose-Konzentration aufweist und bereits zwei Monate nach dem Auspflanzen eine Saccharose-Konzentration von etwa 160 µmol x g⁻¹ Frischgewicht in den Pfahlwurzeln aufweist (Fig. 3). Diese hohe Saccharose-Konzentration stieg während der folgenden drei Entwicklungsmonate an und erreichte etwa 450 µmol x g⁻¹ Frischgewicht. Diese entspricht, bezogen auf die zwei Monate alten Pfahlwurzeln, einen Anstieg um das Dreifache.

Im Unterschied dazu enthielten die Pfahlwurzeln der Sorte "Brigadier" weniger als 70 µmol Saccharose je Frischgewicht nach zweimonatigem Wachstum, und sie akkumulierten nur etwa 195 µmol Saccharose je g Frischgewicht in den folgenden drei Monaten (Fig. 3).

Bei dem Vergleich der Saccharose-Konzentration von Blättern und Pfahlwurzeln konnte ein etwa 30-fach höherer Saccharose-Gehalt in den Pfahlwurzeln, verglichen mit den Blättern, nachgewiesen werden, während die GlucoseKonzentration in den Blättern etwa um den Faktor 80 über dem in den Pfahlwurzeln lag.

Die Unterschiede bei der Saccharose-Akkumulation zwischen den unterschiedlichen Zuckerrüben-Sorten spiegelten sich auch in der Menge der für *Bv*TST2.1 kodierenden mRNA wieder (Fig. 4). Sowohl in den Pfahlwurzeln der Sorte "Belladonna KWS" wie auch in der Sorte "Brigadier" waren die mRNA-Mengen für alle vier paralogen Zuckertransporter nach zwei Wachstumsmonaten niedrig.

Nach einem weiteren Wachstums- und Entwicklungsmonat war die mRNA-Menge für *Bv*TST2.1 in beiden Sorten deutlich höher als die Mengen der für *Bv*TMT1, *Bv*TMT2.2 und *Bv*TMT3 kodierenden mRNAs. Zudem war die *Bv*TST2.1 mRNA-Menge in den Pfahlwurzeln der Sorte "Belladonna KWS" etwa 2,6-fach höher als in den Pfahlwurzeln der Sorte "Brigadier" (Fig. 4).

Während zwei weiterer Wachstumsmonate, änderte sich die *Bv*TST2.1 mRNA-Menge in beiden Sorten nicht wesentlich, verglichen mit der Menge nach 3 Monaten Wachstum, so dass auch nach einer fünfmonatigen Wachstums- und Entwicklungsphase die mRNA-Menge für *Bv*TST2.1 in Pfahlwurzeln der Sorte "Belladonna KWS" nach wie vor um das etwa 2,6-fach höher als in den Pfahlwurzeln der Sorte "Brigadier" war.

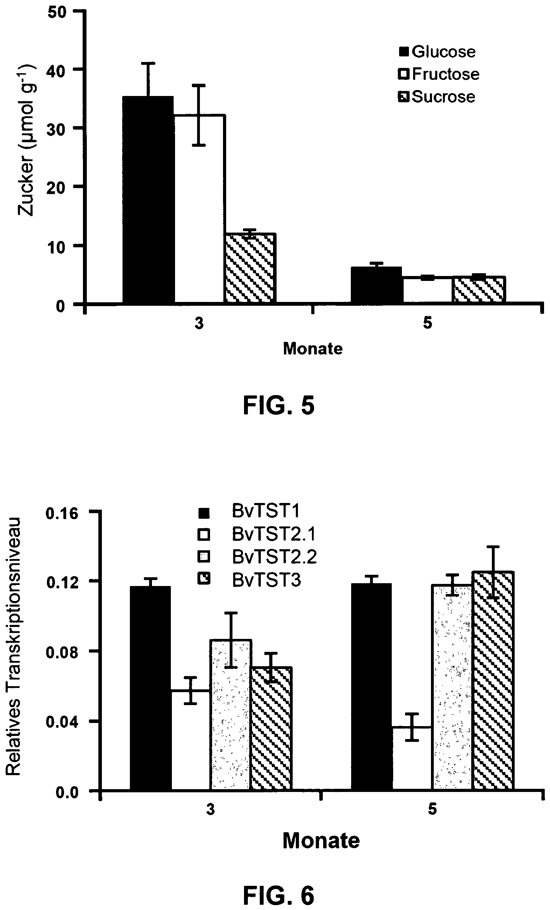

Um weitere Hinweise auf die Bedeutung des *Bv*TST2.1-Proteins für die Saccharose-Speicherung zu erhalten, wurden die Konzentrationen an Glucose, Fructose und Saccharose in Blättern von drei und fünf Monate alten Zuckerrüben der Sorte "Belladonna KWS" bestimmt (Fig. 5) und mit den mRNA-Mengen der vier TST-Paralogen (Fig. 6) verglichen. Im Unterschied zu den Pfahlwurzeln, in denen der Glucose- und der Fructosegehalt sehr niedrig war, akkumulierten diese beiden Monosaccharide in den Blättern. In den Blättern der drei Monate alten Zuckerrüben betrug die Konzentration an Glucose und Fructose zwischen 33 und 35 µmol/g Frischgewicht, während die Konzentration an Saccharose unter 15 µmol/g Frischgewicht lag. Nach fünf Monaten Wachstum betrug die Konzentration jedes der drei Zucker zwischen 6 und 9 µmol/g Frischgewicht (Fig. 5).

Bemerkenswert war, dass die mRNA-Menge für *Bv*TST2.1 in den Blättern durchweg niedriger war als die mRNA-Menge für *Bv*TMT1, *Bv*TMT2.2 und *Bv*TMT3, während die mRNA-Menge für *Bv*TST2.1 in der Pfahlwurzel immer höher war als die mRNA-Menge für die anderen Isoformen (Fig. 6).

### Beispiel 12: BvTST2.1-vermittelter Tonoplastentransport von Saccharose

Um die Transportfunktion von *Bv*TST2.1 nachzuweisen, wurde die "Patch-Clamp"-Technologie an isolierten Vakuolen angewendet. Dazu wurde ein *Bv*TST2.1-GFP Fusionsprotein transient in Mesophylzellen von *Nicotiana benthaminana* exprimiert. Intakte Vakuolen transformierter Protoplasten konnten nach milder hypoosmotischer Lyse durch ihre Grünfärbung identifiziert werden.

Um den physiologischen Protonengradienten über den Tonoplasten der isolierten Vakuolen nachzubilden, wurde das Medium in der Pipette, das den luminalen Inhalt der Vakuole repräsentiert, auf ein pH-Wert von 5,5 gepuffert, während das Medium in der Kammer (=Bad), das das Cytsol repräsentiert, auf pH 7,5 eingestellt wurde. Wenn Saccharose zu dem "cytosolischen" Medium zugegeben wurde, reagierten die Vakuolen mit einem stark abwärts gerichteten Ausschlag des Stromflusses. Die Zugabe von Saccharose in das die isolierten Vakuolen umgebende Medium führte zu einem einwärts gerichteten Strom, der für einen Protonen-Antiport des Saccharosetransports spricht.

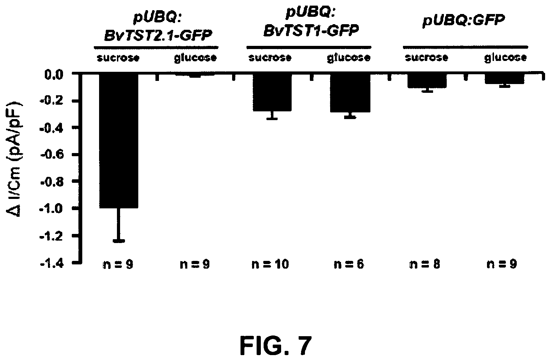

In Abwesenheit von *Bv*TST2.1 zeigten isolierte Vakuolen von *N. benthaminana* keine signifikante Saccharose/Protonentransport-Aktivität. Im Unterschied dazu führte die Zugabe von Saccharose in das Kammermedium bei *Bv*TST2.1 aufweisenden Vakuolen zu einem einwärts gerichteten Stromfluss in einer Größe von fast -1 pA/pF (Figur 7). Diese Ströme repräsentieren den biologischen Fingerabdruck eines von Protonen getriebenen Saccharoseimports über die *Bv*TST2.1-GFP enthaltende Vakuolenmembran und ist ein klares Zeichen dafür, dass *Bv*TST2.1 den Export von Protonen entlang des Protonengradienten über der Membran mit einem Import von Saccharose gegen den bestehenden Saccharose-Gradienten koppelt. Die letztgenannte Funktion ist biochemische Voraussetzung, damit die Zuckerrübe hohe Mengen Saccharose in den Vakuolen ihrer Pfahlwurzeln akkumulieren kann.

Bemerkenswert ist, dass *Bv*TST2.1 keinen Glucose-vermittelten Export von Protonen ermöglicht. Im Unterschied zu *Bv*TST2.1 vermittelt die Isoform *Bv*TST1 sowohl einen Saccharose bedingten wie auch einen Glucose bedingten Stromfluss in der Größenordnung von etwa -03, pA/pF (Fig. 7; Tabelle 2).

**Tabelle 2:**

| Stromdichte | | | |
|---|---|---|---|
| [Δ l/Cm (pA/pF)] | | | |
| | Saccharose | Glucose | Nettoverhältnis sac/glc |
| *BvTST1-GFP* | -0.28 ± 0.06 | -0.29 ± 0.04 | 0.81 |
| *BvTST2.1-GFP* | -1.03 ± 0.29 | -0.018 ± 0.005 | ∞ |
| *GFP* (control) | -0.11 ± 0.04 | -0.08 ± 0.03 | |

Zuckerinduzierte Änderungen in der Stromdichte einzelner Vakuolen.

Diese Daten verdeutlichen die Spezifität von *Bv*TST2.1 für Saccharose.

### Beispiel 13: Saccharosespezifität von BvTST2.1 in vivo

Um die hohe Substratspezifität von *Bv*TST2.1 in lebenden Pflanzenzellen zu analysieren wurden *At*TMT Doppel-Gen-Konockout Mutanten, die keines der beiden Wesentlichen tonoplastidären Monosaccharid Transporterproteine aufweisen, mit entweder einem PUBQ:*Bv*TST2.1-GFP Konstrukt oder einem pUBQ:*Bv*TST1 Konstrukt transformiert. Die Transformanden wuchsen in Gegenwart des toxischen Glucose-Analogons 2-Deoxyglucose. In Kontrollversuchen ohne 2-Deoxyglucose wiesen alle Pflanzenlinien ein vergleichbares Wachstum auf. In Gegenwart von 2-Deoxyglucose entwickelten sich die tst1-2 Doppel-Gen-Knockout-Mutanten nicht richtig, während Wildtyp-Pflanzen und die Linien, die *Bv*TST1 exprimierten, ein wesentlich besseres Wachstum zeigten. Die Wildtyp-Pflanzen und die *Bv*TST1 exprimierende Doppel-Gen-Knockout-Mutante wuchsen in Gegenwart von 2-Deoxyglucose vermutlich besser, weil 2-Deoxyglucose zur Entgiftung in die Vakuolen transportiert werden konnte. Dazu ist die Doppel-Gen-Knockout-Mutante nicht in der Lage. Diejenigen Doppel-Gen-Kockout-Pflanzen die *Bv*TST2.1 exprimierten, konnten den Wachstumsstopp der *Attst*1-2 Doppel-Gen-Knockout-Mutante in Gegenwart von 2-Deoxyglucose nicht kompensieren, obwohl das *Bv*TST2.1-GFP-Fusionsprotein in den Vakuolenmembranen vorhanden war.

Die bemerkenswerte Sensitivität der *At*tst1-2::*Bv*TST2.1-GFP Pflanzen gegenüber 2-Deoxyglucose in vivo steht in Einklang mit den elektrophysiologischen Daten und der Saccharosespezifität von *Bv*TST2.1, die an den isolierten Vakuolen gewonnen wurde.

## Patentansprüche

1. Ein Vektor oder mobiles genetisches Element, umfassend ein Nukleinsäuremolekül, welches ein tonoplastidäres Protonen/Zucker-Antiporter-Protein kodiert, **dadurch gekennzeichnet, dass** das tonoplastidäre Protonen/Zucker-Antiporter-Protein spezifisch für Saccharose ist,
wobei die Spezifität des tonoplastidären Protonen/Zucker-Antiporter-Proteins für Saccharose gegenüber einem Monosaccharid mindestens 5-fach höher ist und wobei das Nukleinsäuremolekül ein Nukleinsäuremolekül umfasst, ausgewählt aus der Gruppe:
a) ein Nukleinsäuremolekül mit einer Nukleotidsequenz gemäß SEQ ID NO: 2, oder ein Nukleinsäuremolekül mit einer Nukleotidsequenz, die eine Identität von mindestens 80% zu der Nukleotidsequenz gemäß SEQ ID NO: 2 aufweist;
b) ein Nukleinsäuremolekül mit einer Nukleotidsequenz, die komplementär zu einer der Nukleotidsequenzen gemäß a) ist;
c) ein Nukleinsäuremoleküle mit einer Nukleotidsequenz, die ein Polypeptid mit einer Aminosäuresequenz gemäß SEQ ID NO: 1 kodiert, oder ein Nukleinsäuremolekül mit einer Nukleotidsequenz, die ein Polypeptid kodiert, dessen Aminosäuresequenz eine Identität von mindestens 80% zu SEQ ID NO: 1 aufweist.

2. Eine eukaryontische oder prokaryontische Wirtszelle, umfassend den Vektor oder das mobile genetische Element gemäß Anspruch 1.

3. Eine transgene Pflanzenzelle, umfassend ein Nukleinsäuremolekül, kodierend für ein tonoplastidäres Protonen/Zucker-Antiporter-Protein wie in Anspruch 1 definiert, als Transgen, oder den Vektor oder das mobile genetische Element gemäß Anspruch 1.

4. Eine transgene Pflanze oder ein Teil davon, umfassend mindestens eine transgene Pflanzenzelle gemäß Anspruch 3.

5. Ein Samen von der transgenen Pflanze gemäß Anspruch 4, wobei der Samen ein Nukleinsäuremolekül, kodierend für ein tonoplastidäres Protonen/Zucker-Antiporter-Protein, wie in Anspruch 1 definiert, als Transgen, oder den Vektor oder das mobile genetische Element gemäß Anspruch 1 aufweist.

6. Ein Verfahren zur Herstellung einer transgenen Pflanze, wobei das Verfahren die folgenden Schritte umfasst:
(a) Einbringen eines Nukleinsäuremoleküls, kodierend für ein tonoplastidäres Protonen/Zucker-Antiporter-Protein, wie in Anspruch 1 definiert, oder des Vektors oder mobilen genetischen Elements gemäß Anspruch 1 in mindestens eine Zelle einer Pflanze, und
(b) Regenerieren einer transgenen Pflanze aus der in Schritt a) erhaltenen Pflanzenzelle.

7. Ein Verfahren zur Erhöhung der Saccharosekonzentration eines Saccharosespeicherorgans einer Pflanze durch Überexpression eines Nukleinsäuremoleküls, wie in Anspruch 1 definiert, welches für das tonoplastidäre Protonen/Zucker-Antiporter Protein kodiert in mindestens einer Zelle der Pflanze.

8. Das Verfahren nach Anspruch 7, wobei die Überexpression durch genetische Modifizierung eines endogenen regulatorischen Elements erreicht wird, wobei das endogene regulatorische Element operativ verknüpft ist mit der für das Protein kodierenden Nukleinsäure.

9. Ein Verfahren zur Identifizierung einer Pflanze, die geeignet ist, eine erhöhte Saccharosekonzentration in ihrem Saccharosespeicherorgan zu erzeugen, umfassend das Nachweisen eines Nukleinsäuremoleküls, kodierend für ein tonoplastidäres Protonen/Zucker-Antiporter-Protein, wie in Anspruch 1 definiert.

10. Ein Oligonukleotid, geeignet zur Verwendung als molekularer Marker, welcher diagnostisch für den Nachweis eines Nukleinsäuremoleküls, kodierend für ein tonoplastidäres Protonen/Zucker-Antiporter-Protein, wie in Anspruch 1 definiert, ist, wobei das Oligonukleotid ausgewählt ist aus der folgenden Gruppe: SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25 und SEQ ID NO: 26.

11. Die Verwendung eines tonoplastidären Protonen/Zucker-Antiporter-Proteins zur Erhöhung der Saccharosekonzentration eines Saccharosespeicherorgans einer Pflanze durch Überexpression eines Nukleinsäuremoleküls, welches für das tonoplastidäre Protonen/Zucker-Antiporter-Protein kodiert, und wobei das Nukleinsäuremolekül Folgendes umfasst:
i. ein Nukleinsäuremolekül mit einer Nukleotidsequenz gemäß SEQ ID NO: 2, 4, 6, 8, 10, 12 oder 14 oder mit einer Nukleotidsequenz, die eine Identität von mindestens 80% zu einer der Nukleotidsequenzen gemäß SEQ ID NO: 2, 4, 6, 8, 10, 12 oder 14 aufweist;
ii. ein Nukleinsäuremolekül mit einer Nukleotidsequenz, die komplementär zu einer der Nukleotidsequenzen gemäß i. ist; oder
iii. ein Nukleinsäuremolekül, das ein Polypeptid mit einer Aminosäuresequenz gemäß SEQ ID NO: 1, 3, 5, 7, 9, 11 oder 13 kodiert, oder das ein Polypeptid mit einer Aminosäuresequenz kodiert, die eine Identität von mindestens 80% zu einer der Aminosäuresequenzen gemäß SEQ ID NO: 1, 3, 5, 7, 9, 11 oder 13 aufweist.

12. Die Verwendung nach Anspruch 11, wobei die Überexpression durch genetische Modifizierung eines endogenen regulatorischen Elements erreicht wird.

## Claims

1. A vector or mobile genetic element, comprising a nucleic acid molecule which encodes a tonoplast proton/sugar antiporter protein, **characterized in that** the tonoplast proton/sugar antiporter protein is specific for saccharose,
wherein the specificity of the tonoplast proton/sugar antiporter protein for saccharose is at least five times higher than for a monosaccharide and
wherein the nucleic acid molecule comprises a nucleic acid molecule selected from the group consisting of:
a) a nucleic acid molecule having a nucleotide sequence in accordance with SEQ ID NO: 2 or a nucleic acid molecule which has a nucleotide sequence which has at least 80 % identity with the nucleotide sequence in accordance with SEQ ID NO: 2;
b) a nucleic acid molecule having a nucleotide sequence which is complementary to one of the nucleotide sequences according to a);
c) a nucleic acid molecule having a nucleotide sequence which encodes a polypeptide with an amino acid sequence in accordance with SEQ ID NO: 1, or a nucleic acid molecule having a nucleotide sequence which encodes a polypeptide the amino acid sequence of which has at least 80 % identity with SEQ ID NO: 1.

2. A eukaryotic or prokaryotic host cell comprising the vector or the mobile genetic element as claimed in claim 1.

3. A transgenic plant cell comprising a nucleic acid molecule which encodes a tonoplast proton/sugar antiporter protein as defined in claim 1, as a transgene, or the vector or the mobile genetic element as claimed in claim 1.

4. A transgenic plant or a portion thereof, comprising at least one transgenic plant cell as claimed in claim 3.

5. A seed from the transgenic plant as claimed in claim 4, wherein the seed comprises a nucleic acid molecule which encodes a tonoplast proton/sugar antiporter protein as defined in claim 1, as a transgene, or the vector or the mobile genetic element as claimed in claim 1.

6. A method for producing a transgenic plant, wherein the method comprises the following steps:
(a) incorporating a nucleic acid molecule which encodes a tonoplast proton/sugar antiporter protein as defined in claim 1, as a transgene, or the vector or mobile genetic element as claimed in claim 1, into at least one cell of a plant, and
(b) regenerating a transgenic plant from the plant cell obtained in step a).

7. A method for increasing the saccharose concentration of a saccharose storage organ of a plant by overexpression of a nucleic acid molecule as defined in claim 1 which encodes the tonoplast proton/sugar antiporter protein in at least one cell of the plant.

8. The method as claimed in claim 7, wherein the overexpression is obtained by genetic modification of an endogenous regulatory element, wherein the endogenous regulatory element is operatively linked to the nucleic acid encoding the protein.

9. A method for identifying a plant which is suitable for producing an increased saccharose concentration in its saccharose storage organ, comprising detecting a nucleic acid molecule which encodes a tonoplast proton/sugar antiporter protein as defined in claim 1.

10. An oligonucleotide suitable for use as a molecular marker, which is diagnostic for the detection of a nucleic acid molecule which encodes a tonoplast proton/sugar antiporter protein as defined in claim 1, wherein the oligonucleotide is selected from the following group: SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25 and SEQ ID NO: 26.

11. The use of a tonoplast proton/sugar antiporter protein for increasing the saccharose concentration of a saccharose storage organ of a plant by overexpression of a nucleic acid molecule which encodes the tonoplast proton/sugar antiporter protein, and wherein the nucleic acid molecule comprises the following:
i. a nucleic acid molecule having a nucleotide sequence in accordance with SEQ ID NO: 2, 4, 6, 8, 10, 12 or 14 or having a nucleotide sequence which has at least 80 % identity with one of the nucleotide sequences in accordance with SEQ ID NO: 2, 4, 6, 8, 10, 12 or 14;
ii. a nucleic acid molecule having a nucleotide sequence which is complementary to one of the nucleotide sequences according to i.; or
iii. a nucleic acid molecule which encodes a polypeptide having an amino acid sequence in accordance with SEQ ID NO: 1, 3, 5, 7, 9, 11 or 13, or which encodes a polypeptide having an amino acid sequence which has at least 80 % identity with one of the amino acid sequences in accordance with SEQ ID NO: 1, 3, 5, 7, 9, 11 or 13.

12. The use as claimed in claim 11, wherein the overexpression is obtained by means of genetic modification of an endogenous regulatory element.

## Revendications

1. Un vecteur ou élément génétique mobile, comprenant une molécule d'acide nucléique, laquelle code une protéine antiporteuse tonoplaste protons/sucre, **caractérisé en ce que** la protéine antiporteuse tonoplaste protons/sucre est spécifique pour le saccharose,
la spécificité de la protéine antiporteuse tonoplaste protons/sucre pour le saccharose étant au moins 5 fois plus élevée par rapport à un monosaccharide, et
la molécule d'acide nucléique comprenant une molécule d'acide nucléique sélectionnée dans le groupe comprenant :
a) une molécule d'acide nucléique avec une séquence nucléotidique selon SEQ ID NO : 2, ou une molécule d'acide nucléique avec une séquence nucléotidique qui fait preuve d'une identité d'au moins 80 % avec la séquence nucléotidique selon SEQ ID NO : 2 ;
b) une molécule d'acide nucléique avec une séquence nucléotidique, qui est complémentaire à l'une des séquences nucléotidiques selon a) ;
c) une molécule d'acide nucléique avec une séquence nucléotidique qui code un polypeptide avec une séquence en aminoacides selon SEQ ID NO : 1, ou une molécule d'acide nucléique avec une séquence nucléotidique qui code un polypeptide dont la séquence en aminoacides fait preuve d'une identité d'au moins 80 % avec SEQ ID NO : 1.

2. Une cellule hôte eucaryote ou procaryote comprenant le vecteur ou l'élément génétique mobile selon la revendication 1.

3. Une cellule végétale transgénique, comprenant une molécule d'acide nucléique, codant pour une protéine antiporteuse tonoplaste protons/sucre, telle que définie dans la revendication 1, en tant que transgène, ou le vecteur ou l'élément génétique mobile selon la revendication 1.

4. Un végétal transgénique ou une partie de celui-ci, comprenant au moins une cellule végétale transgénique selon la revendication 3.

5. Une semence du végétal transgénique selon la revendication 4, la semence comportant une molécule d'acide nucléique, codant pour une protéine antiporteuse tonoplaste protons/sucre, telle que définie dans la revendication 1, en tant que transgène, ou le vecteur ou l'élément génétique mobile selon la revendication 1.

6. Un procédé, destiné à produire un végétal transgénique, le procédé comprenant les étapes suivantes, consistant à :
(a) introduire une molécule d'acide nucléique, codant pour une protéine antiporteuse tonoplaste protons/sucre, telle que définie dans la revendication 1, en tant que transgène, ou le vecteur ou l'élément génétique mobile selon la revendication 1 dans au moins une cellule ou un végétal,
(b) régénérer un végétal transgénique à partir de la cellule végétale obtenue dans l'étape a).

7. Un procédé, destiné à élever la concentration en saccharose d'un organe de stockage de saccharose d'un végétal, par surexpression d'une molécule d'acide nucléique, codant pour la protéine antiporteuse tonoplaste protons/sucre dans au moins une cellule du végétal.

8. Le procédé selon la revendication 7, la surexpression étant obtenue par modification génétique d'un élément régulateur endogène, l'élément régulateur endogène étant fonctionnellement lié avec l'acide nucléique codant pour la protéine.

9. Un procédé, destiné à identifier un végétal, qui est apte à générer une concentration élevée en saccharose dans son organe de stockage de saccharose, comprenant la détection d'une molécule d'acide nucléique, codant pour une protéine antiporteuse tonoplaste protons/sucre, telle que définie dans la revendication 1.

10. Un oligonucléotide, apte à être utilisé comme marqueur moléculaire, lequel est défini du point de vue diagnostic pour déceler une molécule d'acide nucléique, codant pour une protéine antiporteuse tonoplaste protons/sucre, telle que définie dans la revendication 1, l'oligonucléotide étant sélectionné dans le groupe suivant : SEQ ID NO : 15, SEQ ID NO : 16, SEQ ID NO : 17, SEQ ID NO : 18, SEQ ID NO : 19, SEQ ID NO : 20, SEQ ID NO : 21, SEQ ID NO : 22, SEQ ID NO : 23, SEQ ID NO : 24, SEQ ID NO : 25 et SEQ ID NO : 26.

11. L'utilisation d'une protéine antiporteuse tonoplaste protons/sucre pour élever la concentration en saccharose d'un organe de stockage de saccharose d'un végétal, par surexpression d'une molécule d'acide nucléique, codant pour la protéine antiporteuse tonoplaste protons/sucre, et la molécule d'acide nucléique comprenant ce qui suit :
i. une molécule d'acide nucléique avec une séquence nucléotidique selon SEQ ID NO : 2, 4, 6, 8, 10, 12 ou 14 ou avec une séquence nucléotidique, faisant preuve d'une identité d'au moins 80 % avec l'une des séquences nucléotidiques selon SEQ ID NO : 2, 4, 6, 8, 10, 12 ou 14 ;
ii. une molécule d'acide nucléique avec une séquence nucléotidique, qui est complémentaire à l'une des séquences nucléotidiques selon i. ; ou
iii. une molécule d'acide nucléique, codant un polypeptide avec une séquence en aminoacides selon SEQ ID NO : 1, 3, 5, 7, 9, 11 ou 13, ou codant un polypeptide avec une séquence en aminoacides faisant preuve d'une identité d'au moins 80 % avec l'une des séquences nucléotidiques selon SEQ ID NO : 1, 3, 5, 7, 9, 11 ou 13.

12. L'utilisation selon la revendication 11, la surexpression étant obtenue par modification génétique d'un élément régulateur endogène.


### SEQUENCE LISTING

<110> KWS SAAT AG
   Südzucker AG Mannheim/Ochsenfurt
   Friedrich-Alexander-Universität Erlangen-Nürnberg
   Technische Universität Kaiserslautern
   Universität zu Köln
   Julius-Maximilians-Universität würzburg
<120> Tonoplastidäre Protonen/Zucker-Antiporter-Proteine und deren Verwendung zur Erhöhung der Saccharosekonzentration eines Saccharosespeicherorgans von Pflanzen
<130> KWS0218PCT
<150> DE102014005337
   <151> 2014-04-11
<160> 28
<170> PatentIn version 3.5
<210> 1
   <211> 735
   <212> PRT
   <213> Beta vulgaris
<400> 1
   Met Ser Ala Ala Val Leu Val Ala Ile Ala Ala Thr Val Gly Asp Leu
   1               5                   10                  15
   Leu Tyr Gly Trp Asp Asn Ala Thr Ile Ala Gly Ala Val Leu Tyr Ile
               20                  25                  30
   Lys Lys Glu Phe Asn Leu Glu Ser Ser Pro Thr Leu Glu Gly Leu Ile
           35                  40                  45
   Val Ala Thr Ser Ile Ile Gly Ala Thr Leu Ile Thr Thr Cys Ser Gly
       50                  55                  60
   Pro Ile Ala Asp Arg Leu Gly Arg Arg Pro Met Met Ile Ile Ser Ser
   65                  70                  75                  80
   Val Cys Phe Phe Val Ser Ala Leu Ile Met Leu Trp Ser Pro Asn Val
                   85                  90                  95
   Tyr Val Leu Leu Phe Gly Arg Leu Leu Asp Gly Phe Gly Ser Gly Leu
               100                 105                 110
   Ala Val Thr Leu Gly Pro Leu Tyr Ile Ser Glu Thr Ala Pro Thr Asp
           115                 120                 125
   Ile Arg Gly Ser Leu Asn Thr Leu Pro Gln Phe Thr Gly Ser Gly Gly
       130                 135                 140
   Met Phe Leu Ala Tyr Cys Met Val Phe Gly Met Ser Leu Met Glu Thr
   145                 150                 155                 160
   Pro Ser Trp Arg Leu Met Leu Gly Ile Leu Phe Val Pro Ser Thr Val
   165 170 175
   Tyr Phe Leu Leu Thr Val Phe Phe Leu Pro Glu Ser Pro Arg Trp Leu
   180 185 190
   Val Ser Lys Gly Arg Met Asn Glu Ala Lys Lys Val Leu Gln Trp Leu
   195 200 205
   Arg Gly Arg Glu Asp Val Phe Ala Glu Met Ala Leu Leu Val Glu Gly
   210 215 220
   Leu Arg Val Gly Gly Asp Thr Ser Ile Glu Glu Tyr Leu Ile Glu Pro
   225 230 235 240
   Asp Ala Gly Leu Ala Glu Asp Gln Asp Pro Met Thr Val Lys Asp Gln
   245 250 255
   Val Arg Leu Tyr Gly Ser Glu Ala Gly Cys Ser Trp Val Ala Arg Pro
   260 265 270
   Val Thr Gly Gln Ser Met Leu Gly Ile Ala Ser Arg Gln Gly Ser Met
   275 280 285
   Gln Ser Pro Ser Val Pro Leu Met Asp Pro Leu Val Thr Leu Phe Gly
   290 295 300
   Ser Val His Glu Lys Leu Pro Glu Gln Gly Ser Met Leu Ser Val Ile
   305 310 315 320
   Phe Pro Thr Phe Gly Ser Met Phe Ser Met Gly Gly Lys Glu Pro Lys
   325 330 335
   Asn Glu Glu Trp Asp Asp Glu Asn Thr Ile Gly Asp Asp Asp Asp Tyr
   340 345 350
   Gly His Asp Asp Glu Asp Tyr Ala Gly Asp Ala Asp Glu Asp Asp Asn
   355 360 365
   Leu Arg Ser Ser Leu Ile Ser Arg Gln Asp Thr Gly Pro Asp Lys Ala
   370 375 380
   Met Val Ala Pro Thr Ser Gly Ser Met Phe Ser Met Lys His Ser Ser
   385 390 395 400
   Trp Leu Gln Gly Ser Glu Ala Ser Gly Ile Gly Gly Gly Trp Gln Leu
   405 410 415
   Ala Trp Lys Trp Ser Glu Arg Glu Gly Leu Asp Gly Thr Lys Glu Gly
   420 425 430
   Gly Phe Lys Arg Leu Tyr Leu His Gln Glu Gly Asp Ala Gly Ser Lys
           435                     440                     445
   Arg Gly Ser Val Ile Ser Leu Ala Gly Gly Glu Val Ile Gly Asp Asn
       450                     455                     460
   Glu Tyr Val Lys Ala Ala Ala Leu Val Ser Gln Pro Ala Leu Tyr Ser
   465                     470                     475                     480
   Arg Asp Phe Met Asp Arg Asp Ser Ile Gly Pro Ala Met Val His Pro
                   485                     490                     495
   Ser Glu Ala Ser Ala Lys Arg Pro Ser Trp Arg Asp Phe Leu Glu Pro
               500                     505                     510
   Gly Val Arg Arg Ala Leu Val Val Gly Val Gly Leu Gln Leu Leu Gln
           515                     520                     525
   Gln Phe Ala Gly Ile Asn Gly Val Leu Tyr Tyr Thr Pro Gln Ile Leu
       530                     535                     540
   Glu Gln Ala Gly Val Gly Asp Leu Leu Ser His Met Gly Ile Gly Ala
   545                     550                     555                     560
   Ser Ser Ala Leu Leu Leu Ile Ser Ala Leu Thr Thr Leu Leu Met Leu
                   565                     570                     575
   Pro Ala Ile Ala Val Ala Met Arg Leu Met Asp Leu Ser Gly Arg Arg
               580                     585                     590
   Thr Leu Leu Leu Thr Thr Ile Pro Val Leu Phe Leu Ser Leu Val Val
           595                     600                     605
   Leu Ile Leu Ala Asn Val Ile Lys Met Asn Thr Thr Val Tyr Ala Val
       610                     615                     620
   Val Ser Thr Val Ala Val Val Leu Tyr Phe Cys Phe Phe Val Met Gly
   625                     630                     635                     640
   Phe Gly Pro Ile Pro Asn Ile Leu Cys Ala Glu Ile Phe Pro Thr Lys
                   645                     650                     655
   Ile Arg Gly Val Cys Ile Ala Ile Cys Ala Leu Thr Phe Trp Ile Cys
               660                     665                     670
   Asp Ile Ile Val Thr Tyr Thr Leu Pro Met Met Leu Lys Ala Val Gly
           675                     680                     685
   Leu Ala Gly Leu Phe Gly Phe Tyr Ala Val Val Ile Leu Ile Ala Trp
       690                     695                     700
   Ile Phe Ile Phe Leu Lys Val Pro Glu Thr Lys Gly Met Pro Leu Glu
   705 710 715 720
   Val Ile Thr Glu Phe Phe Ala Leu Gly Ala Arg Gln Ala Ser His
   725 730 735
<210> 2
   <211> 2208
   <212> DNA
   <213> Beta vulgaris
<400> 2
   atgagtgcag cagtattagt tgcaattgct gcaacagttg gagatttgct gtatggatgg 60
   gataatgcta ctattgctgg ggctgtatta tatattaaga aagagttcaa cttggagagt 120
   tctccaacct tggaagggtt aattgtggcc acatcaataa ttggagccac tcttattaca 180
   acatgttctg gaccgattgc agatcgtctt ggtcgtcgcc ctatgatgat aatttcctca 240
   gtttgtttct ttgttagtgc cttaataatg ttgtggtctc ccaatgttta tgttttactc 300
   ttcggtcggc tattagatgg atttggaagt ggtttggcag tcactcttgg tcctctttat 360
   atatcagaga ccgctccaac tgatataaga ggctcactga acacacttcc tcagtttact 420
   ggttctggtg gaatgttcct cgcatactgc atggttttcg ggatgtcatt gatggaaaca 480
   cctagctgga gattaatgct tgggattctt tttgttccat ctactgttta ttttctatta 540
   actgtattct tcttacctga gtctcctcgc tggcttgtta gcaaaggacg gatgaatgag 600
   gctaaaaagg ttcttcaatg gttgcgaggc agggaagatg tctttgctga gatggctctc 660
   cttgttgagg gtcttagagt tggaggtgat acatcaatag aggaatactt gattgagcca 720
   gatgctggac tcgctgagga tcaagatccg atgactgtca aagatcaggt taggctgtat 780
   gggtccgaag caggctgctc ctgggttgcc agaccagtca ctggtcagag tatgctgggt 840
   attgcatctc ggcagggaag catgcagagt cctagtgttc ctttaatgga tccccttgta 900
   actctttttg gtagtgtaca tgaaaagctt ccagaacaag gaagtatgct tagtgtcata 960
   ttcccaactt ttggtagtat gtttagtatg ggagggaaag agcccaaaaa tgaagagtgg 1020
   gatgatgaaa atactattgg ggatgatgat gattatggtc atgacgatga agattatgca 1080
   ggtgatgctg atgaagatga caatttacgt agttcactta tatctcgtca ggatacaggt 1140
   ccagacaaag ccatggttgc tcctacttca ggtagcatgt tcagcatgaa gcatagtagt 1200
   tggttacaag gaagcgaagc tagtggtatt ggtggtggtt ggcagttagc ttggaaatgg 1260
   agcgagagag aaggcttgga tggtacgaag gaaggaggat tcaaaagact ttatctacat 1320
   caggaaggtg atgctggatc taaacgaggc tctgttattt ctcttgctgg tggtgaggtt 1380
   attggcgaca atgagtatgt gaaggctgct gcactagtaa gtcaacctgc cctttattcg 1440
   agggatttca tggatcggga tagtattggt ccagctatgg ttcacccttc cgaggcttct 1500
   gcaaaaaggc ctagttggag ggatttttta gagcctggtg tcaggcgtgc attagttgtt 1560
   ggtgtcggac ttcaacttct tcaacagttc gctgggataa atggcgttct gtattatact 1620
   cctcaaatac tagagcaagc tggggtgggt gatcttcttt cgcatatggg tataggcgct 1680
   tcctctgcat tgttactcat cagtgcactc acaactcttt tgatgcttcc tgctatagct 1740
   gttgcaatga ggcttatgga tctttctggg agaaggactt tgctactaac cacaattccg 1800
   gtgttgttct tatcgctcgt tgtcttaata ctcgcaaatg tcataaagat gaacaccact 1860
   gtgtatgcag tggtctccac agtcgcggta gttctctact tctgcttctt tgtgatgggg 1920
   tttgggccta tcccaaatat cctatgtgca gaaattttcc caaccaagat tcgtggagtc 1980
   tgtattgcta tttgtgcact tactttctgg atctgtgata tcatagtcac ctacacactc 2040
   cctatgatgc ttaaagctgt tggacttgct ggtctctttg gcttctatgc tgttgtgatt 2100
   ttaattgcat ggatatttat atttttgaag gttcctgaaa ccaagggcat gccccttgag 2160
   gtaatcactg agttctttgc tctcggtgca agacaagcaa gccactga 2208
<210> 3
   <211> 735
   <212> PRT
   <213> Beta vulgaris
<400> 3
   Met Lys Gly Ala Val Leu Val Ala Leu Ala Ala Thr Ile Gly Asn Phe
   1               5                   10                  15
   Leu Gln Gly Trp Asp Asn Ala Thr Ile Ala Gly Ser Ile Leu Tyr Ile
               20                  25                  30
   Lys Lys Glu Leu Glu Leu Ser Thr Ala Met Glu Gly Leu Val Val Ala
           35                  40                  45
   Met Ser Leu Ile Gly Ala Thr Val Ile Thr Thr Cys Ser Gly Ala Val
       50                  55                  60
   Ser Asp Ala Val Gly Arg Arg Pro Leu Leu Met Leu Ser Ala Ser Leu
   65                  70                  75                  80
   Tyr Phe Ile Gly Ser Leu Val Met Leu Trp Ser Pro Asn Val Tyr Ile
                   85                  90                  95
   Leu Leu Leu Ala Arg Leu Leu Asp Gly Phe Gly Ile Gly Leu Ala Val
               100                 105                 110
   Thr Leu Val Pro Val Tyr Ile Ser Glu Thr Ser Pro Pro Glu Ile Arg
           115                 120                 125
   Gly Leu Leu Asn Thr Leu Pro Gln Phe Thr Gly Ser Gly Gly Met Phe
       130                 135                 140
   Leu Ser Tyr Cys Met Ile Phe Gly Met Ser Leu Met Ala Ser Pro Ser
   145                 150                 155                 160
   Trp Arg Ile Met Leu Gly Val Leu Gly Ile Pro Ser Val Phe Tyr Leu
                   165                 170                 175
   Leu Phe Ala Phe Phe Tyr Leu Pro Glu Ser Pro Arg Trp Leu Val Ser
               180                 185                 190
   Lys Gly Arg Met Ser Glu Ala Lys Lys Val Leu Lys Arg Leu Arg Gly
           195                 200                 205
   Thr Glu Asp Val Ser Gly Glu Leu Ser Leu Leu Val Glu Gly Leu Gly
       210                 215                 220
   Val Gly Gly Glu Thr Ser Ile Glu Glu Tyr Ile Val Glu Pro Ala Glu
   225                 230                 235                 240
   Glu Leu Ala Gly Gly Thr Glu Lys Gly Lys Val Lys Leu Tyr Gly Ala
                   245                 250                 255
   Ala Glu Gly Leu Ser Trp Ile Ala Lys Pro Val Thr Gly Gln Ser Ala
               260                 265                 270
   Val Gly Leu Val Ser Arg His Gly Ser Met Val Gly Leu Val Asp Pro
           275                 280                 285
   Leu Val Thr Leu Phe Gly Ser Val His Glu Lys Leu Pro Glu Gln Gly
       290                 295                 300
   Asn Met Arg Ser Ala Leu Phe Pro Ser Ile Gly Ser Met Leu Ser Thr
   305                 310                 315                 320
   Ala Asp Ala His Val His Arg Asp Gln Trp Asp Glu Glu Asn Gln Asp
                   325                 330                 335
   Val Asp Glu Asp Asp Glu Pro Ile Ala Asp Pro Ala Gly Gly Glu Asp
               340                 345                 350
   Phe Asp Asp Asn Asp Leu His Ala Pro Leu Ile Ser Arg Gln Thr Thr
           355                 360                 365
   Ser Met Glu Lys Asp Met Gly Leu Pro Pro Val Ser His Gly Thr Val
       370                 375                 380
   Met Ser Met Gly Tyr His Gly Ser Leu Phe Gln Gly Ala Gly Glu Thr
   385                 390                 395                 400
   Ile Thr Thr Thr Gly Ile Gly Gly Gly Trp Gln Leu Ala Trp Thr Leu
                   405                 410                 415
   Asp Glu Lys Glu Ala Glu Asp Gly Lys Lys Ser Lys Asp Phe Lys Arg
               420                 425                 430
   Ile Tyr Leu His Gln Asp Gly Gly Pro Ala Ser Met Arg Gly Ser Leu
           435                 440                 445
   Leu Ser Leu Pro Gly Gly Asp Phe Pro Gly Asp Gly Asp Cys Val Gln
       450                 455                 460
   Ala Ser Ala Leu Val Ser Asn Pro Ala Leu Tyr Ser Lys Glu Val Leu
   465                 470                 475                 480
   Gly Gln Ser Pro Ile Gly Pro Ala Met Val His Pro Ala Glu Ile Ala
                   485                 490                 495
   Ser Gln Gly Pro Thr Trp Lys Ala Leu Leu Asp Pro Gly Val Lys Arg
               500                 505                 510
   Ala Leu Ile Val Gly Val Gly Ile Gln Met Leu Gln Gln Phe Ala Gly
           515                 520                 525
   Ile Asn Gly Val Leu Tyr Tyr Thr Pro Gln Ile Leu Glu Glu Ala Gly
       530                 535                 540
   Val Glu Val Leu Leu Ser Asp Leu Gly Ile Ser Ser Thr Ser Ala Ser
   545                 550                 555                 560
   Phe Leu Ile Ser Ala Leu Thr Thr Phe Leu Met Leu Pro Cys Ile Ala
                   565                 570                 575
   Val Ala Met Arg Leu Met Asp Ile Ser Gly Arg Arg Ser Leu Leu Leu
               580                 585                 590
   Ala Thr Ile Pro Val Leu Ile Ala Ser Leu Val Ile Leu Val Ile Ser
           595                 600                 605
   Cys Thr Val Ser Met Gly Ser Val Ile His Ala Val Ile Ser Ile Ile
       610                 615                 620
   Cys Val Ile Val Tyr Phe Cys Thr Phe Val Met Ala Tyr Gly Pro Ile
   625                 630                 635                 640
   Pro Asn Ile Leu Cys Ser Glu Ile Phe Pro Thr Arg Val Arg Gly Val
                   645                 650                 655
   Cys Ile Ala Ile Cys Gly Leu Val Phe Trp Ile Cys Asp Ile Ile Val
               660                 665                 670
   Thr Tyr Ser Leu Pro Val Met Leu Asn Ser Ile Gly Leu Gly Gly Ile
           675                 680                 685
   Phe Ala Ile Tyr Ala Val Val Cys Val Ile Ser Leu Val Phe Val Tyr
       690                 695                 700
   Leu Lys Val Pro Glu Thr Lys Gly Met Pro Leu Glu Val Ile Thr Glu
   705 710 715 720
   Phe Phe Ser Val Asp Pro Lys Gln Leu Glu Ala Ala Lys Ala Glu
   725 730 735
<210> 4
   <211> 2208
   <212> DNA
   <213> Beta vulgaris
<400> 4
   atgaagggtg ctgtgcttgt ggctttggct gctacaattg gtaattttct acagggttgg      60
   gacaatgcaa caattgctgg gtctattctc tacatcaaga aagaacttga actatccacc     120
   gccatggagg gacttgtcgt ggcaatgtca cttattggag ctacggttat cacaacgtgc     180
   tcgggggccg tatcagatgc tgttggtcga cgtcctttgc tgatgctctc ggcctcactc     240
   tactttattg gcagtttggt gatgttatgg tcacctaatg tctatatttt gcttttagcc     300
   aggttgttgg atggttttgg aatagggctg gctgtgaccc ttgttcctgt ttatatatca     360
   gagacttccc ctccagagat taggggatta ctaaatacac ttcctcaatt cactggctct     420
   ggtggaatgt tcttatcata ctgcatgatc tttggaatgt cactcatggc atctcctagc     480
   tggagaataa tgcttggtgt tcttgggatc ccttctgttt tttatctttt atttgcattc     540
   ttctacttgc ctgaatcccc gcggtggctt gtgagcaaag gaaggatgtc tgaagcaaag     600
   aaggttttga aaagattacg tggcactgaa gatgtctcag gtgaattgtc tttgctagtt     660
   gaagggcttg gtgtcggggg tgaaacttca atagaagagt acattgtaga accagcagaa     720
   gagctagcag gtggtactga gaaaggcaaa gtaaagctat acggagcagc agaaggcctt     780
   tcttggattg caaaacctgt cactggacag agtgctgttg gtcttgtatc ccgtcatgga     840
   agcatggtgg gcctagttga tcctctcgtg actctgtttg gaagcgtcca tgaaaagctt     900
   cctgaacaag gaaacatgag aagtgcgctt ttcccaagta ttggcagcat gttaagcacg     960
   gcagatgctc atgttcacag agatcaatgg gatgaagaaa accaggatgt tgatgaagac    1020
   gatgagccaa tcgctgatcc tgcaggaggg gaggattttg atgataatga cttgcatgct    1080
   ccattgattt cacgtcaaac aacaagcatg gagaaagaca tgggtcttcc tcctgtctct    1140
   catggtactg ttatgagcat gggataccat ggcagtcttt ttcaaggtgc tggggaaact    1200
   attactacta caggaattgg cggtggttgg caattggcgt ggactttaga tgagaaagaa    1260
   gctgaagatg gaaagaaatc taaagacttc aaaaggattt acttgcatca ggatggcggg    1320
   ccggcttcta tgcgtggatc acttctatca cttcctggtg gtgatttccc tggagatgga    1380
   gattgtgttc aggcttctgc tcttgtaagt aatcctgcac tttattcgaa ggaggttctg    1440
   ggtcaaagtc ctattggtcc tgcgatggtt catccagctg aaattgcttc ccaaggacca    1500
   acctggaagg ctctccttga tccaggagtc aagcgtgcat tgattgttgg agttggaatt    1560
   cagatgcttc aacagttcgc tggtataaat ggcgttctct actatacccc acaaattcta    1620
   gaagaggcag gagtagaagt tcttctatct gatctaggga tcagctctac atctgcctca 1680
   tttcttatca gtgcattaac gaccttctta atgctgccct gtatcgctgt tgctatgagg 1740
   ctcatggata tctctggtag aaggtcactg ttgcttgcta caattcctgt gttgatcgcc 1800
   tcattggtta tcctagtaat cagctgcact gttagcatgg gtagtgtaat tcatgctgtc 1860
   atctcaatca tctgtgttat tgtctacttc tgcacatttg ttatggctta cggacccata 1920
   ccgaatattt tatgttccga gatcttccct actcgagtcc gtggtgtttg tattgctata 1980
   tgtggcttgg ttttctggat atgcgacatt attgtcactt actccttgcc cgtcatgctc 2040
   aattctattg gtttgggagg catcttcgca atatatgctg tggtttgtgt catctctttg 2100
   gtgtttgtct acctcaaagt cccagaaaca aagggtatgc ccttagaagt tatcacagag 2160
   ttcttttcag ttgatcctaa acagttagaa gccgctaagg cggagtag 2208
<210> 5
   <211> 739
   <212> PRT
   <213> Beta vulgaris
<400> 5
   Met Ser Gly Ala Val Leu Val Ala Ile Ala Ala Ala Val Gly Asn Leu
   1 5 10 15
   Leu Gln Gly Trp Asp Asn Ala Thr Ile Ala Gly Ala Val Leu Tyr Ile
   20 25 30
   Lys Lys Glu Phe Asn Leu Glu Gly Ala Pro Thr Met Glu Gly Leu Ile
   35 40 45
   Val Ala Met Ser Leu Ile Gly Ala Thr Ile Ile Thr Thr Cys Ser Gly
   50 55 60
   Pro Val Ser Asp Arg Phe Gly Arg Arg Pro Met Met Ile Ile Ser Ser
   65 70 75 80
   Ile Cys Phe Phe Phe Ser Ala Leu Ile Met Leu Trp Ser Pro Asn Val
   85 90 95
   Tyr Val Leu Leu Leu Gly Arg Leu Leu Asp Gly Phe Gly Ser Gly Leu
   100 105 110
   Ala Val Thr Leu Val Pro Leu Tyr Ile Ser Glu Thr Ala Pro Thr Asp
   115 120 125
   Ile Arg Gly Ser Leu Asn Thr Leu Pro Gln Phe Thr Gly Ser Gly Gly
   130 135 140
   Met Phe Val Ala Tyr Cys Met Val Phe Gly Met Ser Leu Met Glu Lys
   145 150 155 160
   Pro Ser Trp Arg Leu Met Leu Gly Ile Leu Val Val Pro Ser Ala Leu
   165 170 175
   Tyr Phe Ala Leu Thr Val Phe Phe Leu Pro Glu Ser Pro Arg Trp Leu
   180 185 190
   Val Ser Lys Gly Arg Met Asn Glu Ala Lys Lys Val Leu Gln Arg Leu
   195 200 205
   Arg Gly Arg Glu Asp Val Ser Ala Glu Met Ala Leu Leu Val Glu Gly
   210 215 220
   Leu Gly Val Gly Gly Asp Ile Ser Ile Glu Glu Tyr Leu Ile Glu Pro
   225 230 235 240
   Asp Val Gly Ile Ser Glu Glu Tyr Asp Pro Met Ala Ala Lys Asp Gln
   245 250 255
   Ile Lys Leu Tyr Gly Ser Asp Ala Gly His Ser Trp Val Ala Arg Pro
   260 265 270
   Val Thr Gly Gln Ser Met Leu Gly Leu Ala Ser Arg Gln Gly Ser Ile
   275 280 285
   Gln Asn Pro Ser Val Pro Leu Met Asp Pro Leu Val Thr Leu Phe Gly
   290 295 300
   Ser Val His Glu Lys Leu Pro Glu Gln Gly Ser Met Arg Ser Ile Ile
   305 310 315 320
   Phe Pro Thr Phe Gly Ser Met Phe Ser Met Gly Gly Lys Asp Pro Arg
   325 330 335
   Asn Glu Glu Trp Asp Glu Glu Asn Leu His Gly Asp Asp Asp Asp Tyr
   340 345 350
   Ala His Asn Asp Asp Asp Asn Asp Asp Tyr Ala Glu Asp Asp Asp Asn
   355 360 365
   Leu His Ser Pro Leu Ile Ser Arg Gln Ala Thr Gly Thr Asp Lys Ala
   370 375 380
   Met Val Ala Pro Val Ser Gly Ser Met Phe Ser Met Lys Pro Ser Gly
   385 390 395 400
   Leu Ile Gln Gly Thr Glu Ala Ser Gly Ile Gly Gly Gly Trp Gln Leu
   405 410 415
   Ala Trp Gln Trp Ser Glu Lys Glu Gly Ala Asp Gly Arg Lys Glu Gly
   420 425 430
   Gly Phe Lys Arg Leu Tyr Leu His Gln Glu Gly Asp Met Val Ser Lys
           435                 440                 445
   Arg Gly Ser Val Ile Ser Leu Pro Gly Gly Asp Val Thr Gly Glu Thr
       450                 455                 460
   Glu Tyr Met Lys Ala Ala Ala Leu Val Ser Gln Pro Ala Leu Tyr Ser
   465                 470                 475                 480
   Arg Glu Leu Met Asn Gln His Thr Ile Gly Pro Ala Met Val His Pro
                   485                 490                 495
   Ser Glu Thr Ala Ala Lys Gly Ser Ser Trp Arg Asp Leu Leu Glu Pro
               500                 505                 510
   Gly Val Arg Arg Ala Leu Ile Val Gly Val Gly Leu Gln Leu Leu Gln
           515                 520                 525
   Gln Phe Ser Gly Ile Asn Gly Val Leu Tyr Tyr Thr Pro Gln Ile Leu
       530                 535                 540
   Glu Gln Ala Gly Val Gly Asp Leu Leu Ser Asn Met Gly Ile Gly Ala
   545                 550                 555                 560
   Ser Ser Ala Ser Leu Leu Ile Ser Ala Leu Thr Thr Leu Leu Met Leu
                   565                 570                 575
   Pro Ser Ile Ala Val Ala Met Arg Leu Met Asp Ile Ser Gly Arg Arg
               580                 585                 590
   Thr Leu Leu Leu Thr Thr Ile Pro Val Leu Phe Leu Ser Leu Val Val
           595                 600                 605
   Leu Ile Leu Gly Asn Ile Ile Lys Met Ser Thr Thr Val His Ala Val
       610                 615                 620
   Ile Ser Thr Val Ser Val Val Leu Tyr Phe Cys Phe Phe Val Met Gly
   625                 630                 635                 640
   Phe Gly Pro Ile Pro Asn Ile Leu Cys Ala Glu Ile Phe Pro Thr Arg
                   645                 650                 655
   Ile Arg Gly Val Cys Ile Ala Ile Cys Ala Leu Thr Phe Trp Ile Gly
               660                 665                 670
   Asp Ile Ile Val Thr Asp Thr Leu Pro Ile Met Leu Asn Ala Val Gly
           675                 680                 685
   Leu Ala Gly Val Phe Gly Phe Tyr Ala Val Val Ser Val Ile Ala Trp
       690                 695                 700
   Ile Phe Ile Phe Leu Lys Val Pro Glu Thr Lys Gly Met Pro Leu Glu
   705                 710                 715                 720
   Val Ile Thr Glu Phe Phe Ala Leu Gly Ala Arg Gln Pro Ser Glu Asp
                   725                 730                 735
   Lys Asp Asn
<210> 6
   <211> 2220
   <212> DNA
   <213> Beta vulgaris
<400> 6
   atgagtggag cggtattagt tgcaattgct gcggcagttg ggaatttact acaaggatgg      60
   gataatgcta ctattgctgg ggctgtgttg tatattaaga aagaattcaa cttggagggc     120
   gcgccaacca tggaaggctt aattgtggcc atgtcactta ttggagccac tattatcaca     180
   acatgctctg gaccagtttc agatcgcttt gggcgtcgcc ctatgatgat aatctcctct     240
   atatgtttct tttttagtgc cctaataatg ttgtggtctc ccaatgttta tgtcctactc     300
   ttgggtcgat tattagatgg atttggaagt ggtttggctg tcactcttgt tcctctttat     360
   atatcagaga cagctccaac tgatataagg ggttcattga atacacttcc tcaatttact     420
   ggttcgggtg gaatgtttgt tgcgtactgt atggtgtttg ggatgtcttt aatggaaaaa     480
   cctagctgga gattgatgct tgggattctt gttgttccat ctgctcttta ttttgcatta     540
   actgtattct tcttacccga gtctcctcga tggcttgtta gtaaaggacg aatgaatgag     600
   gccaaaaagg ttcttcaacg attgcggggc agggaagatg tctctgctga gatggctctg     660
   cttgttgagg gtcttggagt tggaggtgac atatcaatag aagaatactt aattgagcca     720
   gatgttggca tcagcgagga atatgatccg atggctgcca aagatcagat taaattatat     780
   gggtcagatg caggccactc ttgggttgcc agaccagtca ccggccagag tatgctgggc     840
   cttgcatctc gtcagggaag cattcagaac cctagtgttc ccttaatgga ccccctggtc     900
   acgcttttcg gtagtgtaca tgagaagctt ccagaacaag gaagcatgcg aagtattatt     960
   ttcccgactt ttggtagtat gtttagtatg ggaggcaaag accctagaaa tgaagagtgg    1020
   gatgaggaga atcttcatgg ggatgatgat gattatgccc ataatgatga tgacaatgat    1080
   gattatgctg aagatgatga caatttacat agtccactta tatctcgtca ggctacaggt    1140
   acagacaaag ctatggttgc tccagtttca ggtagcatgt tcagcatgaa acctagtggt    1200
   ttaatacaag gaactgaagc tagtggaatt ggtggtggtt ggcagctggc ttggcaatgg    1260
   agtgagaaag aaggtgcaga tgggaggaag gagggaggat tcaaaagact ttacttacat    1320
   caggaaggtg atatggtatc taaacgagga tctgtaatct ctcttcctgg cggtgatgtt    1380
   actggagaga cagagtatat gaaggctgct gcactagtga gtcaacctgc cctctattcg    1440
   agggagttga tgaatcagca tactattgga ccagctatgg ttcatccttc tgagactgct    1500
   gcaaaagggt ctagctggag ggacctttta gaacctggtg tcaggcgtgc attgattgtt 1560
   ggtgtcgggc tccaacttct tcagcagttt tctggtataa atggggttct gtattacact 1620
   cctcaaatac tagagcaagc tggggttggt gatcttcttt caaacatggg tattggcgct 1680
   tcctctgcat cattgctcat cagtgcactc acaactcttt tgatgcttcc ttccatagct 1740
   gttgcaatga ggcttatgga tatttccggg aggaggactt tgctgcttac cacaattccg 1800
   gtgttgttcc tttcgctcgt cgtcctaata ctcggaaata tcataaagat gagcactact 1860
   gtgcacgcag tgatctcaac agtctccgta gttctctact tctgcttctt tgtgatgggc 1920
   tttggcccaa tcccaaatat cctatgcgcg gaaattttcc caaccaggat tcgtggtgtc 1980
   tgcattgcta tttgtgcact taccttttgg atcggagata ttatagttac tgacacactt 2040
   cctataatgc tcaatgctgt tggacttgct ggtgtctttg gcttctatgc tgtcgttagt 2100
   gtaattgctt ggatttttat ctttctaaag gttcccgaaa ccaagggcat gcctctcgag 2160
   gtcattactg agttctttgc tctcggggca agacaacctt ctgaagacaa ggacaactga 2220
<210> 7
   <211> 728
   <212> PRT
   <213> Beta vulgaris
<400> 7
   Met Arg Gly Ala Val Phe Ala Ala Leu Ala Ala Thr Met Gly Asn Leu
   1               5                   10                  15
   Leu Gln Gly Trp Asp Asn Ala Thr Ile Ala Gly Ala Val Ile Tyr Ile
               20                  25                  30
   Lys Arg Glu Phe Ser Leu Glu Thr Gln Pro Thr Leu Glu Gly Leu Ile
           35                  40                  45
   Val Ala Met Ser Leu Ile Gly Ala Thr Val Ile Thr Thr Phe Ser Gly
       50                  55                  60
   Pro Val Ser Asp Ser Val Gly Arg Arg Pro Met Leu Ile Ile Ser Ser
   65                  70                  75                  80
   Ile Leu Tyr Phe Leu Gly Gly Leu Val Met Leu Trp Ser Pro Asn Val
                   85                  90                  95
   Tyr Val Leu Leu Leu Gly Arg Leu Leu Asp Gly Phe Gly Val Gly Leu
               100                 105                 110
   Ala Val Thr Leu Val Pro Val Tyr Ile Ser Glu Thr Ala Pro Pro Glu
           115                 120                 125
   Ile Arg Gly Gln Leu Asn Thr Leu Pro Gln Phe Thr Gly Ser Gly Gly
       130                 135                 140
   Met Phe Leu Ser Tyr Cys Met Val Phe Gly Met Ser Leu Met Glu Ala
   145                 150                 155                 160
   Pro Arg Trp Arg Leu Met Leu Gly Val Ile Ser Ile Pro Ser Leu Leu
                   165                 170                 175
   Tyr Leu Gly Leu Met Val Phe Tyr Leu Pro Glu Ser Pro Arg Trp Leu
               180                 185                 190
   Val Ser Lys Gly Lys Met His Glu Ala Lys Lys Val Leu Gln Lys Leu
           195                 200                 205
   Arg Gly Arg Glu Asp Val Thr Gly Glu Met Ala Leu Leu Ile Glu Gly
       210                 215                 220
   Leu Gly Thr Gly Lys Asn Thr Ser Ile Glu Glu Tyr Val Ile Gly Pro
   225                 230                 235                 240
   Ala Asn Asp Glu Glu Ala Thr Thr Asp Lys Asp Gln Ile Lys Leu Tyr
                   245                 250                 255
   Gly Ala Glu Gln Gly Gln Ser Trp Ile Ala Lys Pro Val Arg Gly Gln
               260                 265                 270
   Ser Thr Leu Gly Met Val Ser Arg Tyr Gly Ser Met Ala Gln Gln Gly
           275                 280                 285
   Ser Met Ala Asn Met Met Asp Pro Leu Val Thr Leu Phe Gly Ser Val
       290                 295                 300
   His Glu Lys Leu Pro Gln Ser Gly Ser Met Arg Ser Ala Ile Phe Pro
   305                 310                 315                 320
   Asn Phe Gly Ser Met Phe Ser Thr Ala Ala Asp Asp His Val Lys His
                   325                 330                 335
   Val Asn Trp Glu Val Glu Ser Arg Asp Glu Asp Ser Ser Ser Asp Val
               340                 345                 350
   Gly His Asp Asp Ser Asp Asp Asn Leu Arg Ser Pro Leu Leu Ser Pro
           355                 360                 365
   His Ala Pro Gly Ala Glu Lys Asp Ala Val Pro Pro Leu Asn Gly Asn
       370                 375                 380
   Ser Met Leu Met Gln Ser Gly Glu Leu Val Asn Ser Thr Gly Ile Gly
   385                 390                 395                 400
   Gly Gly Trp Gln Leu Ala Tyr Lys Lys Ala Glu Asp Gly Gly Glu Leu
                   405                 410                 415
   Lys Arg Val Tyr Leu His Gln Glu Pro Gly Met Gly Ser Met Arg Gly
               420                 425                 430
   Ser Met Arg Gly Ser Met Arg Gly Ser Val Leu Ser Leu His Pro Ser
           435                 440                 445
   Asp Ile Pro Glu Gly Gln Leu Val Pro Ala Ala Gly Leu Val Ser Gln
       450                 455                 460
   Ser Thr Leu Gln Ile Lys Asp Phe Lys Gly Glu Ser Pro Phe Glu Gly
   465                 470                 475                 480
   Gly Asp Ile Arg Pro Ser Ala Ala Ala Thr Lys Gly Pro Ser Trp Arg
                   485                 490                 495
   Glu Leu Leu Glu Pro Gly Val Lys Arg Ala Leu Leu Val Gly Met Gly
               500                 505                 510
   Met Gln Ile Leu Gln Gln Phe Ser Gly Ile Asn Gly Val Leu Tyr Tyr
           515                 520                 525
   Thr Pro Gln Ile Leu Ser Gln Ala Gly Val Asp Val Leu Leu Ser Glu
       530                 535                 540
   Leu Gly Ile Gly Ser Asp Ser Ala Ser Leu Leu Ile Ser Gly Leu Thr
   545                 550                 555                 560
   Thr Leu Leu Met Leu Pro Ser Ile Gly Leu Ala Met Arg Leu Met Asp
                   565                 570                 575
   Ile Ser Gly Arg Arg Phe Leu Leu Leu Asn Thr Leu Pro Val Leu Ile
               580                 585                 590
   Gly Ser Leu Ile Ile Leu Val Leu Ser Asn Val Ile Glu Met Gly Thr
           595                 600                 605
   Val Leu His Ala Thr Leu Ser Thr Ile Ser Val Val Val Tyr Phe Cys
       610                 615                 620
   Cys Phe Val Met Gly Phe Gly Pro Ile Pro Asn Ile Leu Cys Ser Glu
   625                 630                 635                 640
   Ile Phe Pro Thr Arg Val Arg Gly Leu Cys Ile Ala Ile Cys Ser Leu
                   645                 650                 655
   Thr Phe Trp Phe Gly Asp Ile Ile Val Thr Tyr Ser Leu Pro Ala Leu
               660                 665                 670
   Leu Ser Ser Ile Gly Leu Ala Gly Val Phe Gly Ile Tyr Ala Val Val
           675                 680                 685
   Cys Ile Val Ser Trp Phe Phe Val Tyr Phe Met Val Pro Glu Thr Lys
       690                 695                 700
   Gly Met Pro Leu Glu Val Ile Ser Glu Phe Phe Asn Val Gly Ala Arg
   705                 710                 715                 720
   Gln Ala Glu Ala Glu Lys Asn Met
                   725
<210> 8
   <211> 2187
   <212> DNA
   <213> Beta vulgaris
<400> 8
   atgagaggag ctgtatttgc agcacttgct gccacaatgg gtaacttgtt gcaagggtgg 60
   gataatgcca ccatagcagg agctgttata tacatcaaga gggaattcag cctcgaaaca 120
   caaccaacct tggaggggct aattgtggcc atgtcgctta ttggggccac agtgatcaca 180
   accttctcag gtcccgtttc agattcagtt gggcgtcgtc caatgctaat catctcatca 240
   atattatact ttcttggtgg cttggttatg ctatggtctc ctaatgtata tgtcttgctc 300
   ttgggaaggc ttttggatgg ttttggtgtt ggtctagcag ttactcttgt gccagtatat 360
   atatcagaga ctgcaccccc agaaatcaga ggacagttga ataccctccc acagttcact 420
   ggctcaggag ggatgttctt gtcttactgt atggtatttg gcatgtcttt gatggaagca 480
   cctagatgga gattgatgct cggtgttatt tcaatcccgt cacttttgta tcttggattg 540
   atggtgtttt atttgcctga gtctcctagg tggctcgtca gcaaaggaaa gatgcatgag 600
   gctaagaaag tcttacaaaa attgcgcggc agggaagatg tcactggtga gatggcattg 660
   cttatagaag ggcttggaac tgggaaaaat acatccatcg aagagtatgt gataggccca 720
   gcaaatgatg aagaagccac cacagataaa gatcaaatca agctatatgg tgctgagcaa 780
   ggccaatctt ggatagccaa accagtcaga ggtcaaagca cgcttggcat ggtttctcgt 840
   tatggaagca tggctcagca gggaagtatg gcaaacatga tggatcctct cgtcactttg 900
   tttggtagtg ttcatgaaaa gcttccccaa tcagggagca tgcggagtgc aatattccct 960
   aactttggga gcatgttcag tactgctgct gatgaccatg ttaaacatgt aaattgggag 1020
   gtggagagcc gagatgagga ctcctcatct gatgttggcc atgatgactc tgatgataat 1080
   ctgaggagtc cactgctttc acctcatgcc cctggagcag aaaaggatgc agttcctcca 1140
   ttaaatggca acagcatgct gatgcaaagt ggtgaattag tcaatagtac aggtataggt 1200
   ggaggttggc agttagcgta caagaaagca gaagatggtg gtgaactaaa aagggtttat 1260
   ctccatcaag aaccaggaat ggggtctatg cgtggatcta tgcgtgggtc tatgcgtggg 1320
   tctgtccttt cactgcatcc ttctgatatt cctgaaggtc agcttgttcc agctgctggt 1380
   cttgtaagcc agtccaccct tcaaatcaag gatttcaagg gagaatctcc ttttgaggga 1440
   ggtgatatac gaccttctgc agctgctaca aaagggccaa gctggagaga gcttcttgaa 1500
   ccaggggtta agcgtgcatt gttggttgga atgggaatgc agatacttca acagttctct 1560
   gggatcaatg gagttctcta ctacacccct caaattcttt cacaagcagg agtggacgtt 1620
   ctcctatcag aattagggat tggttcagac tccgcttctc ttcttataag tggtttgacg 1680
   acattgttga tgcttcctag cataggcctt gcaatgaggc tgatggatat ctctgggaga 1740
   aggtttcttt tactaaacac acttcccgtc ttgataggat ctctcattat acttgtactt 1800
   tccaatgtta tcgagatggg aaccgtctta cacgcgacat tatctactat cagtgttgta 1860
   gtctacttct gctgctttgt catgggtttt ggccccattc caaatatcct ctgctctgaa 1920
   atcttcccta ctcgtgtccg tggcctttgc attgccatat gttctcttac cttctggttt 1980
   ggagatatca ttgtcacgta ctctctccca gctttgctct cctctatagg gctcgccgga 2040
   gtatttggca tctatgccgt ggtttgcatc gtctcttggt tctttgttta tttcatggta 2100
   cctgaaacaa agggcatgcc ccttgaagtt atcagtgagt tcttcaatgt gggtgcaagg 2160
   caagctgaag ctgagaaaaa tatgtga 2187
<210> 9
   <211> 734
   <212> PRT
   <213> Arabidopsis thaliana
<400> 9
   Met Lys Gly Ala Thr Leu Val Ala Leu Ala Ala Thr Ile Gly Asn Phe
   1 5 10 15
   Leu Gln Gly Trp Asp Asn Ala Thr Ile Ala Gly Ala Met Val Tyr Ile
   20 25 30
   Asn Lys Asp Leu Asn Leu Pro Thr Ser Val Gln Gly Leu Val Val Ala
   35 40 45
   Met Ser Leu Ile Gly Ala Thr Val Ile Thr Thr Cys Ser Gly Pro Ile
   50 55 60
   Ser Asp Trp Leu Gly Arg Arg Pro Met Leu Ile Leu Ser Ser Val Met
   65 70 75 80
   Tyr Phe Val Cys Gly Leu Ile Met Leu Trp Ser Pro Asn Val Tyr Val
   85 90 95
   Leu Cys Phe Ala Arg Leu Leu Asn Gly Phe Gly Ala Gly Leu Ala Val
   100 105 110
   Thr Leu Val Pro Val Tyr Ile Ser Glu Thr Ala Pro Pro Glu Ile Arg
   115 120 125
   Gly Gln Leu Asn Thr Leu Pro Gln Phe Leu Gly Ser Gly Gly Met Phe
   130 135 140
   Leu Ser Tyr Cys Met Val Phe Thr Met Ser Leu Ser Asp Ser Pro Ser
   145 150 155 160
   Trp Arg Ala Met Leu Gly Val Leu Ser Ile Pro Ser Leu Leu Tyr Leu
   165 170 175
   Phe Leu Thr Val Phe Tyr Leu Pro Glu Ser Pro Arg Trp Leu Val Ser
   180 185 190
   Lys Gly Arg Met Asp Glu Ala Lys Arg Val Leu Gln Gln Leu Cys Gly
   195 200 205
   Arg Glu Asp Val Thr Asp Glu Met Ala Leu Leu Val Glu Gly Leu Asp
   210 215 220
   Ile Gly Gly Glu Lys Thr Met Glu Asp Leu Leu Val Thr Leu Glu Asp
   225 230 235 240
   His Glu Gly Asp Asp Thr Leu Glu Thr Val Asp Glu Asp Gly Gln Met
   245 250 255
   Arg Leu Tyr Gly Thr His Glu Asn Gln Ser Tyr Leu Ala Arg Pro Val
   260 265 270
   Pro Glu Gln Asn Ser Ser Leu Gly Leu Arg Ser Arg His Gly Ser Leu
   275 280 285
   Ala Asn Gln Ser Met Ile Leu Lys Asp Pro Leu Val Asn Leu Phe Gly
   290 295 300
   Ser Leu His Glu Lys Met Pro Glu Ala Gly Gly Asn Thr Arg Ser Gly
   305 310 315 320
   Ile Phe Pro His Phe Gly Ser Met Phe Ser Thr Thr Ala Asp Ala Pro
   325 330 335
   His Gly Lys Pro Ala His Trp Glu Lys Asp Ile Glu Ser His Tyr Asn
   340 345 350
   Lys Asp Asn Asp Asp Tyr Ala Thr Asp Asp Gly Ala Gly Asp Asp Asp
   355 360 365
   Asp Ser Asp Asn Asp Leu Arg Ser Pro Leu Met Ser Arg Gln Thr Thr
   370 375 380
   Ser Met Asp Lys Asp Met Ile Pro His Pro Thr Ser Gly Ser Thr Leu
   385 390 395 400
   Ser Met Arg Arg His Ser Thr Leu Met Gln Gly Asn Gly Glu Ser Ser
   405 410 415
   Met Gly Ile Gly Gly Gly Trp His Met Gly Tyr Arg Tyr Glu Asn Asp
               420                 425                 430
   Glu Tyr Lys Arg Tyr Tyr Leu Lys Glu Asp Gly Ala Glu Ser Arg Arg
           435                 440                 445
   Gly Ser Ile Ile Ser Ile Pro Gly Gly Pro Asp Gly Gly Gly Ser Tyr
       450                 455                 460
   Ile His Ala Ser Ala Leu Val Ser Arg Ser Val Leu Gly Pro Lys Ser
   465                 470                 475                 480
   Val His Gly Ser Ala Met Val Pro Pro Glu Lys Ile Ala Ala Ser Gly
                   485                 490                 495
   Pro Leu Trp Ser Ala Leu Leu Glu Pro Gly Val Lys Arg Ala Leu Val
               500                 505                 510
   Val Gly Val Gly Ile Gln Ile Leu Gln Gln Phe Ser Gly Ile Asn Gly
           515                 520                 525
   Val Leu Tyr Tyr Thr Pro Gln Ile Leu Glu Arg Ala Gly Val Asp Ile
       530                 535                 540
   Leu Leu Ser Ser Leu Gly Leu Ser Ser Ile Ser Ala Ser Phe Leu Ile
   545                 550                 555                 560
   Ser Gly Leu Thr Thr Leu Leu Met Leu Pro Ala Ile Val Val Ala Met
                   565                 570                 575
   Arg Leu Met Asp Val Ser Gly Arg Arg Ser Leu Leu Leu Trp Thr Ile
               580                 585                 590
   Pro Val Leu Ile Val Ser Leu Val Val Leu Val Ile Ser Glu Leu Ile
           595                 600                 605
   His Ile Ser Lys Val Val Asn Ala Ala Leu Ser Thr Gly Cys Val Val
       610                 615                 620
   Leu Tyr Phe Cys Phe Phe Val Met Gly Tyr Gly Pro Ile Pro Asn Ile
   625                 630                 635                 640
   Leu Cys Ser Glu Ile Phe Pro Thr Arg Val Arg Gly Leu Cys Ile Ala
                   645                 650                 655
   Ile Cys Ala Met Val Phe Trp Ile Gly Asp Ile Ile Val Thr Tyr Ser
               660                 665                 670
   Leu Pro Val Leu Leu Ser Ser Ile Gly Leu Val Gly Val Phe Ser Ile
           675                 680                 685
   Tyr Ala Ala Val Cys Val Ile Ser Trp Ile Phe Val Tyr Met Lys Val
       690                 695                 700
   Pro Glu Thr Lys Gly Met Pro Leu Glu Val Ile Thr Asp Tyr Phe Ala
   705                 710                 715                 720
   Phe Gly Ala Gln Ala Gln Ala Ser Ala Pro Ser Lys Asp Ile
                   725                 730
<210> 10
   <211> 1698
   <212> DNA
   <213> Arabidopsis thaliana
<400> 10
   atggcggtta agcacggcgt cgacggcgac ggcagcgaaa tcgaatcgag aactctggcg 60
   gtagatcgaa aaagcggttt ctgcgaatca acatcgatat tctacagcaa acgtgagcca 120
   atggctctcc cgccaaacca atttctcgac gtcacgtctt tcattgcatc acagcctcat 180
   cgcggcaaaa ccgtattcgt cgacgccgta accggtcgtc gactaagctt ccctgagctc 240
   tggctcggtg tcgaaagagt cgcgggttgt ctttacgcat taggtgtacg caaaggaaat 300
   gtcgtcatta tactctctcc aaactcaatc ctcttcccga tcgtctccct ctccgtaatg 360
   tcactcggcg caatcataac caccgctaat ccgatcaaca cgtccgacga aatctccaaa 420
   cagataggcg attcgcgtcc tgttctcgcc ttcaccacat gcaaactcgt ctccaaactc 480
   gccgccgcgt cgaattttaa tctcccggtg gttctcatgg acgactacca cgttccttcg 540
   caaagttacg gtgaccgcgt gaaactagtc gggaggttag agacgatgat tgaaacagaa 600
   ccgagtgagt cacgagttaa gcaacgagtc aaccaggacg acacggcggc tctgttatac 660
   tcatcaggta cgacggggac gagtaaagga gtaatgctga gtcaccgtaa cctaatcgca 720
   ttggtacaag cataccgggc ccggttcggt ttagagcagc gaaccatttg cacaatccca 780
   atgtgtcaca tcttcggatt cggtggtttc gcgacggggc taatcgcgtt aggatggaca 840
   atcgttgttc ttcctaaatt cgacatggct aagcttctct cggcggtgga gactcatcgt 900
   tcttcgtacc tttctcttgt accgccgatt gtagtagcta tggttaacgg agcaaatgag 960
   attaattcca agtatgatct gagctcgttg cacactgtgg tggctggagg agctccgttg 1020
   agtagagagg tgacggagaa gttcgttgag aattatccca aggttaagat cctacaaggg 1080
   tatggtttga ctgagtcaac ggctatagct gcttctatgt ttaataaaga ggagactaag 1140
   aggtatggag cttctggctt actggctccg aatgtggaag gtaagattgt ggatccggat 1200
   acgggtcggg ttttgggtgt gaatcaaacg ggtgagctgt ggattcgaag tcctactgtg 1260
   atgaaaggtt atttcaagaa taaagaagct actgcttcta ccattgattc agaaggatgg 1320
   ttgaaaactg gagatttgtg ttacattgac ggtgatgggt ttgtctttgt tgttgataga 1380
   ttaaaggagc tcatcaaatg caatggttat caggttgctc cagctgaact agaggcattg 1440
   ttgcttgctc atccagagat tgctgatgca gcagtaatac ccatccctga catgaaagct 1500
   gggcaatatc caatggcata tatcgtaaga aaagttggaa gtaacttatc cgagagcgaa 1560
   atcatgggat ttgtcgcaaa acaggtatca ccgtacaaga agattcgcaa agtcacattt 1620
   ttggcttcaa tccccaaaaa tccttcgggc aagattttaa gaagagaact tacaaagctc 1680
   acaacttcaa aactctag 1698
<210> 11
   <211> 739
   <212> PRT
   <213> Arabidopsis thaliana
<400> 11
   Met Ser Gly Ala Val Leu Val Ala Ile Ala Ala Ala Val Gly Asn Leu
   1 5 10 15
   Leu Gln Gly Trp Asp Asn Ala Thr Ile Ala Gly Ala Val Leu Tyr Ile
   20 25 30
   Lys Lys Glu Phe Asn Leu Glu Ser Asn Pro Ser Val Glu Gly Leu Ile
   35 40 45
   Val Ala Met Ser Leu Ile Gly Ala Thr Leu Ile Thr Thr Cys Ser Gly
   50 55 60
   Gly Val Ala Asp Trp Leu Gly Arg Arg Pro Met Leu Ile Leu Ser Ser
   65 70 75 80
   Ile Leu Tyr Phe Val Gly Ser Leu Val Met Leu Trp Ser Pro Asn Val
   85 90 95
   Tyr Val Leu Leu Leu Gly Arg Leu Leu Asp Gly Phe Gly Val Gly Leu
   100 105 110
   Val Val Thr Leu Val Pro Ile Tyr Ile Ser Glu Thr Ala Pro Pro Glu
   115 120 125
   Ile Arg Gly Leu Leu Asn Thr Leu Pro Gln Phe Thr Gly Ser Gly Gly
   130 135 140
   Met Phe Leu Ser Tyr Cys Met Val Phe Gly Met Ser Leu Met Pro Ser
   145 150 155 160
   Pro Ser Trp Arg Leu Met Leu Gly Val Leu Phe Ile Pro Ser Leu Val
   165 170 175
   Phe Phe Phe Leu Thr Val Phe Phe Leu Pro Glu Ser Pro Arg Trp Leu
   180 185 190
   Val Ser Lys Gly Arg Met Leu Glu Ala Lys Arg Val Leu Gln Arg Leu
   195 200 205
   Arg Gly Arg Glu Asp Val Ser Gly Glu Met Ala Leu Leu Val Glu Gly
       210                 215                 220
   Leu Gly Ile Gly Gly Glu Thr Thr Ile Glu Glu Tyr Ile Ile Gly Pro
   225                 230                 235                 240
   Ala Asp Glu Val Thr Asp Asp His Asp Ile Ala Val Asp Lys Asp Gln
                   245                 250                 255
   Ile Lys Leu Tyr Gly Ala Glu Glu Gly Leu Ser Trp Val Ala Arg Pro
               260                 265                 270
   Val Lys Gly Gly Ser Thr Met Ser Val Leu Ser Arg His Gly Ser Thr
           275                 280                 285
   Met Ser Arg Arg Gln Gly Ser Leu Ile Asp Pro Leu Val Thr Leu Phe
       290                 295                 300
   Gly Ser Val His Glu Lys Met Pro Asp Thr Gly Ser Met Arg Ser Ala
   305                 310                 315                 320
   Leu Phe Pro His Phe Gly Ser Met Phe Ser Val Gly Gly Asn Gln Pro
                   325                 330                 335
   Arg His Glu Asp Trp Asp Glu Glu Asn Leu Val Gly Glu Gly Glu Asp
               340                 345                 350
   Tyr Pro Ser Asp His Gly Asp Asp Ser Glu Asp Asp Leu His Ser Pro
           355                 360                 365
   Leu Ile Ser Arg Gln Thr Thr Ser Met Glu Lys Asp Met Pro His Thr
       370                 375                 380
   Ala His Gly Thr Leu Ser Thr Phe Arg His Gly Ser Gln Val Gln Gly
   385                 390                 395                 400
   Ala Gln Gly Glu Gly Ala Gly Ser Met Gly Ile Gly Gly Gly Trp Gln
                   405                 410                 415
   Val Ala Trp Lys Trp Thr Glu Arg Glu Asp Glu Ser Gly Gln Lys Glu
               420                 425                 430
   Gly Gly Phe Lys Arg Ile Tyr Leu His Gln Glu Gly Phe Pro Gly Ser
           435                 440                 445
   Arg Arg Gly Ser Ile Val Ser Leu Pro Gly Gly Asp Gly Thr Gly Glu
       450                 455                 460
   Ala Asp Phe Val Gln Ala Ser Ala Leu Val Ser Gln Pro Ala Leu Tyr
   465                 470                 475                 480
   Ser Lys Asp Leu Leu Lys Glu His Thr Ile Gly Pro Ala Met Val His
   485 490 495
   Pro Ser Glu Thr Thr Lys Gly Ser Ile Trp His Asp Leu His Asp Pro
   500 505 510
   Gly Val Lys Arg Ala Leu Val Val Gly Val Gly Leu Gln Ile Leu Gln
   515 520 525
   Gln Phe Ser Gly Ile Asn Gly Val Leu Tyr Tyr Thr Pro Gln Ile Leu
   530 535 540
   Glu Gln Ala Gly Val Gly Ile Leu Leu Ser Asn Met Gly Ile Ser Ser
   545 550 555 560
   Ser Ser Ala Ser Leu Leu Ile Ser Ala Leu Thr Thr Phe Val Met Leu
   565 570 575
   Pro Ala Ile Ala Val Ala Met Arg Leu Met Asp Leu Ser Gly Arg Arg
   580 585 590
   Thr Leu Leu Leu Thr Thr Ile Pro Ile Leu Ile Ala Ser Leu Leu Val
   595 600 605
   Leu Val Ile Ser Asn Leu Val His Met Asn Ser Ile Val His Ala Val
   610 615 620
   Leu Ser Thr Val Ser Val Val Leu Tyr Phe Cys Phe Phe Val Met Gly
   625 630 635 640
   Phe Gly Pro Ala Pro Asn Ile Leu Cys Ser Glu Ile Phe Pro Thr Arg
   645 650 655
   Val Arg Gly Ile Cys Ile Ala Ile Cys Ala Leu Thr Phe Trp Ile Cys
   660 665 670
   Asp Ile Ile Val Thr Tyr Ser Leu Pro Val Leu Leu Lys Ser Ile Gly
   675 680 685
   Leu Ala Gly Val Phe Gly Met Tyr Ala Ile Val Cys Cys Ile Ser Trp
   690 695 700
   Val Phe Val Phe Ile Lys Val Pro Glu Thr Lys Gly Met Pro Leu Glu
   705 710 715 720
   Val Ile Thr Glu Phe Phe Ser Val Gly Ala Arg Gln Ala Glu Ala Ala
   725 730 735
   Lys Asn Glu
<210> 12
   <211> 2220
   <212> DNA
   <213> Arabidopsis thaliana
<400> 12
   atgagtggag ctgtgcttgt tgctattgct gctgctgttg gcaacttgtt acaaggatgg      60
   gataacgcaa ctattgcagg agctgtgttg tacataaaaa aggagtttaa tttggagagt     120
   aatccatcag tggaaggtct aattgtggcg atgtcactta ttggtgctac tctgattaca     180
   acatgctctg gaggggtagc tgattggctt ggtcgccgtc ccatgctaat attgtcctca     240
   attctctact ttgttggttc tctagtaatg ctatggtctc cgaatgttta tgtgttgctc     300
   ttaggaaggt tgttagatgg atttggggtt ggtcttgtgg tcacacttgt tcctatttat     360
   atatctgaga ctgcaccacc tgagattagg ggactgttga atacgctacc gcagttcact     420
   ggctctggag ggatgttctt atcttactgt atggttttcg gaatgtcgtt gatgccatca     480
   cctagctgga gattgatgct tggtgtcctt ttcatccctt cccttgtctt tttcttcctc     540
   acggtcttct tcttgcccga gtccccaagg tggctcgtga gcaaaggtcg aatgcttgaa     600
   gcaaagcggg ttcttcagag actgcgtggt cgcgaagatg tgtctggtga gatggctttg     660
   ttggttgagg gtcttggaat tggaggtgaa acaaccatag aggaatatat aattggtccc     720
   gcggatgaag ttactgatga tcatgatata gctgtggata aggatcaaat taagttatat     780
   ggtgcagaag aagggctgag ttgggttgct aggccagtca aaggaggaag cactatgagt     840
   gttttgtctc gccatggaag tacaatgagc aggaggcaag gctcattgat tgatcctctt     900
   gtcacactgt ttgggagcgt tcacgagaag atgccggaca ctggaagcat gaggagtgcc     960
   ttgttcccac attttgggag tatgttcagt gttggaggga atcaaccaag acatgaagat    1020
   tgggatgaag agaatcttgt tggagaaggt gaggattatc catccgacca tggagatgat    1080
   tctgaagatg atcttcattc tccgttgatc tcacgtcaaa cgacaagcat ggagaaagac    1140
   atgcctcaca ctgctcatgg aactctttct accttcagac atggaagtca agtgcaggga    1200
   gctcaagggg aaggagcggg tagtatgggg attggaggtg gatggcaagt ggcatggaaa    1260
   tggacggaaa gagaagatga atcgggacag aaagaaggtg ggtttaaacg gatatacttg    1320
   catcaagaag gtttcccagg atctcgacgt ggctcaattg tttcattgcc tggtggtgat    1380
   ggaaccggtg aggcagattt tgtacaagcg tctgctttgg ttagccaacc agctctttat    1440
   tccaaagacc ttctcaaaga acatacaatt ggtcctgcta tggtacatcc atccgaaaca    1500
   actaaagggt caatttggca tgatcttcat gatcctggag tcaagcgtgc attagtcgta    1560
   ggagttggac ttcaaatact tcagcagttc tcaggcatca acggagttct ttactacaca    1620
   ccgcaaatcc ttgagcaggc gggtgtcggg atcctactat cgaacatggg gattagttct    1680
   tcctcagcat ccttacttat aagtgcattg acaacctttg tgatgttacc tgcaatagct    1740
   gttgcaatga ggctcatgga tctttctggt cgaaggacct tgcttctcac cacgatacca    1800
   atcctgatag catctctatt ggttttagta atctcaaatc ttgttcacat gaacagcatt    1860
   gtgcacgcgg tcttatcaac cgtaagcgtt gtgctctact tctgcttctt cgtgatgggt      1920
   ttcggtcctg ctccaaacat cctctgttca gagatttttc caactcgagt ccgcggaatc      1980
   tgcatcgcca tctgcgcact caccttctgg atctgtgaca taatcgtcac ttacagtctc      2040
   cccgtgctgc tcaaatccat tggactagct ggtgtgtttg gaatgtacgc aatcgtatgt      2100
   tgcatttcat gggtctttgt gttcattaaa gtcccggaaa ctaaaggcat gccacttgaa      2160
   gtcatcacag agttcttttc tgttggagct agacaagctg aagctgctaa aaacgagtga      2220
<210> 13
   <211> 737
   <212> PRT
   <213> Arabidopsis thaliana
<400> 13
   Met Arg Ser Val Val Leu Val Ala Leu Ala Ala Ala Ile Gly Asn Met
   1               5                   10                  15
   Leu Gln Gly Trp Asp Asn Ala Thr Ile Ala Gly Ala Val Ile Tyr Ile
               20                  25                  30
   Lys Lys Glu Phe His Leu Glu Lys Glu Pro Lys Ile Glu Gly Leu Ile
           35                  40                  45
   Val Ala Met Ser Leu Ile Gly Ala Thr Leu Ile Thr Thr Phe Ser Gly
       50                  55                  60
   Pro Val Ser Asp Lys Val Gly Arg Arg Ser Met Leu Ile Leu Ser Ser
   65                  70                  75                  80
   Val Leu Tyr Phe Leu Ser Ser Ile Val Met Phe Trp Ser Pro Asn Val
                   85                  90                  95
   Tyr Val Leu Leu Phe Ala Arg Leu Leu Asp Gly Phe Gly Ile Gly Leu
               100                 105                 110
   Ala Val Thr Leu Val Pro Ile Tyr Ile Ser Glu Thr Ala Pro Ser Glu
           115                 120                 125
   Ile Arg Gly Leu Leu Asn Thr Phe Pro Gln Phe Cys Gly Ser Gly Gly
       130                 135                 140
   Met Phe Leu Ser Tyr Cys Leu Val Phe Gly Met Ser Leu Gln Glu Ser
   145                 150                 155                 160
   Pro Ser Trp Arg Leu Met Leu Gly Val Leu Ser Ile Pro Ser Ile Ala
                   165                 170                 175
   Tyr Phe Val Leu Ala Ala Phe Phe Leu Pro Glu Ser Pro Arg Trp Leu
               180                 185                 190
   Val Ser Lys Gly Arg Met Asp Glu Ala Arg Gln Val Leu Gln Arg Leu
           195                 200                 205
   Arg Gly Arg Glu Asp Val Ser Gly Glu Leu Ala Leu Leu Val Glu Gly
       210                 215                 220
   Leu Gly Val Gly Lys Asp Thr Ser Ile Glu Glu Tyr Val Ile Gly Pro
   225                 230                 235                 240
   Asp Asn Glu Glu Asn Glu Gly Gly Asn Glu Leu Pro Arg Lys Asp Gln
                   245                 250                 255
   Ile Lys Leu Tyr Gly Pro Glu Asp Gly Gln Ser Trp Met Ala Lys Pro
               260                 265                 270
   Val Lys Gly Gln Ser Ser Leu Ala Leu Ala Ser Arg Gln Gly Ser Met
           275                 280                 285
   Leu Pro Arg Gly Gly Ser Leu Met Asp Pro Leu Val Thr Leu Phe Gly
       290                 295                 300
   Ser Ile His Glu Asn Leu Pro Ser Glu Asn Met Asn Ala Ser Ser Arg
   305                 310                 315                 320
   Ser Met Leu Phe Pro Asn Met Gly Ser Ile Leu Gly Met Met Gly Arg
                   325                 330                 335
   Gln Glu Ser Gln Trp Asp Pro Glu Arg Asn Asn Glu Asp Ser Ser Asp
               340                 345                 350
   Gln Asp Glu Asn Leu Asn Ser Pro Leu Leu Ser Pro Gln Thr Thr Glu
           355                 360                 365
   Pro Asp Asp Tyr His Gln Arg Thr Val Gly Thr Met His Arg Arg Gln
       370                 375                 380
   Ser Ser Leu Phe Met Ala Asn Val Gly Glu Thr Ala Thr Ala Thr Ser
   385                 390                 395                 400
   Ile Gly Gly Gly Trp Gln Leu Ala Trp Lys Tyr Asn Asp Lys Val Gly
                   405                 410                 415
   Ala Asp Gly Lys Arg Val Asn Gly Gly Leu Gln Arg Met Tyr Ile His
               420                 425                 430
   Glu Glu Thr Ala Asn Asn Asn Thr Asn Asn Ile Pro Phe Ser Arg Arg
           435                 440                 445
   Gly Ser Leu Leu Ser Phe His Pro Glu Gly Asp Gly His Asp Gln Val
       450                 455                 460
   Asn Gly Tyr Val Gln Ala Ala Ala Leu Val Ser Gln Ala Ser Met Met
   465                 470                 475                 480
   Pro Gly Gly Lys Gly Glu Thr Ala Met Leu Pro Lys Glu Val Lys Asp
                   485                 490                 495
   Gly Pro Gly Trp Arg Glu Leu Lys Glu Pro Gly Val Lys Arg Ala Leu
               500                 505                 510
   Met Val Gly Val Gly Leu Gln Ile Leu Gln Gln Phe Ala Gly Ile Asn
           515                 520                 525
   Gly Val Met Tyr Tyr Thr Pro Gln Ile Leu Glu Glu Thr Gly Val Ser
       530                 535                 540
   Ser Leu Leu Thr Asn Leu Gly Ile Ser Ala Glu Ser Ala Ser Leu Leu
   545                 550                 555                 560
   Ile Ser Ala Leu Thr Thr Leu Leu Met Leu Pro Cys Ile Leu Val Ser
                   565                 570                 575
   Met Arg Leu Met Asp Val Thr Gly Arg Arg Ser Leu Met Leu Ser Thr
               580                 585                 590
   Ile Pro Ile Leu Ile Leu Ser Leu Val Thr Leu Val Ile Gly Ser Leu
           595                 600                 605
   Val Asn Leu Gly Gly Ser Ile Asn Ala Leu Ile Ser Thr Ala Ser Val
       610                 615                 620
   Thr Val Tyr Leu Ser Cys Phe Val Met Gly Phe Gly Ala Ile Pro Asn
   625                 630                 635                 640
   Ile Leu Cys Ser Glu Ile Phe Pro Thr Ser Val Arg Gly Leu Cys Ile
                   645                 650                 655
   Thr Ile Cys Ala Leu Thr Phe Trp Ile Cys Asp Ile Ile Val Thr Tyr
               660                 665                 670
   Thr Leu Pro Val Met Leu Lys Ser Ile Gly Ile Ala Gly Val Phe Gly
           675                 680                 685
   Ile Tyr Ala Ile Val Cys Ala Val Ala Trp Val Phe Val Tyr Leu Lys
       690                 695                 700
   Val Pro Glu Thr Lys Gly Met Pro Leu Glu Val Ile Ser Glu Phe Phe
   705                 710                 715                 720
   Ser Val Gly Ala Lys Gln Gln Asp Ala Ala Ala Ser Phe Leu Ser Asp
                   725                 730                 735
   Gly
<210> 14
   <211> 2190
   <212> DNA
   <213> Arabidopsis thaliana
<400> 14
   atgaggagtg tagtgcttgt tgctttagcc gctgcgatag ggaatatgtt gcagggctgg 60
   gacaatgcca ccattgcagg agctgtaatt tacattaaga aagaatttca tttggagaaa 120
   gaaccaaaga tagaaggact gatcgtggca atgtctctca ttggagccac tttgatcaca 180
   actttctctg gtcctgtctc tgacaaagta ggaaggcgat caatgcttat actctcctct 240
   gttctctatt ttctgagtag cattgttatg ttttggtctc ccaatgtcta cgttctcctt 300
   ttcgcaaggc ttcttgatgg ttttgggatc ggtttagctg tcactctcgt cccaatctac 360
   atctctgaga ccgcaccttc tgagatcaga ggattactca acactttccc gcagttttgt 420
   ggatccggtg ggatgttttt gtcgtattgt cttgtgtttg ggatgtcgct tcaagaatca 480
   ccaagctgga ggctgatgct tggtgttttg tcaattccgt ccattgccta ctttgtactt 540
   gcggctttct tcttgcctga atctccaagg tggcttgtca gcaaaggccg tatggatgaa 600
   gctagacagg ttctgcagag actccgtggc agagaagatg tttcaggcga gcttgctctg 660
   ctggttgaag ggcttggggt gggaaaagac acgtcgatag aagaatatgt gattggacca 720
   gacaacgagg aaaacgaggg tggaaatgaa ctgccgagga aagatcagat aaaactatat 780
   ggtccagagg atggacagtc atggatggct aagccagtga aaggacagag ttctctcgca 840
   ttggcttccc gacaaggcag catgttaccg cgtggcggat ccctcatgga cccacttgtc 900
   actctctttg gtagcattca tgagaatctc ccttccgaga acatgaacgc atcatcccgc 960
   agcatgctct tccccaatat gggaagtata ctgggaatga tgggaaggca ggagtcccag 1020
   tgggatccag agagaaacaa tgaagatagt tctgaccaag atgaaaactt aaacagtcct 1080
   ctgctttctc cgcaaaccac tgagccggat gactaccacc agcggaccgt tggtaccatg 1140
   cataggcgac agagcagctt gtttatggca aacgtgggtg agacagcgac ggctacaagc 1200
   ataggcggtg gatggcaatt ggcgtggaag tataatgaca aggttggtgc agatggtaag 1260
   agagtcaatg gagggttaca gagaatgtat attcacgagg aaaccgccaa caacaacacc 1320
   aacaacattc ctttttcgcg acgtggatca cttctctcct tccacccaga gggtgatggt 1380
   catgatcagg tgaatggata cgttcaggct gctgcacttg tgagccaagc ttcgatgatg 1440
   ccaggaggta aaggcgagac cgcaatgttg ccaaaggagg ttaaggatgg tccaggctgg 1500
   agggagctga aagaaccagg ggttaagaga gctttgatgg ttggagttgg gcttcagata 1560
   ctgcaacagt ttgcaggaat aaatggagtg atgtattata cacctcaaat attggaagaa 1620
   acaggtgtgt caagtctttt gacaaacctt ggaataagtg cagagtctgc atcgcttctc 1680
   ataagcgcct taaccacact cttgatgctt ccctgcattc ttgtctccat gaggtctctg 1740
   atgctttcga ctatccccat tctaatactg tcgctggtaa cactggtgat aggaagctta 1800
   gtgaatcttg gaggttcaat aaacgcgttg atatcgacag caagtgttac ggtgtaccta 1860
   agctgtttcg tgatgggttt tggggcaatt ccaaacatcc tctgctcaga gatattccct 1920
   acttctgtgc gcggtctctg catcaccata tgtgccctca ctttctggat ctgtgacata 1980
   atcgtcactt acactctccc agtcatgctc aaatccattg gcatcgcagg agtctttggc 2040
   atttatgcaa tcgtctgtgc tgtcgcgtgg gtttttgtgt acctcaaggt accagagaca 2100
   aagggaatgc cccttgaagt tatctctgag ttcttctccg tcggtgcaaa acagcaagac 2160
   gctgcagctt catttctctc tgatggatga 2190
<210> 15
   <211> 49
   <212> DNA
   <213> Artificial Sequence
<220>
   <223> 5'-Primer für die PCR-Amplification der für Beta vulgaris Tonoplast Zucker Transportprotein 1-kodierenden Nukleotidsequenz
<400> 15
   ggggacaagt ttgtacaaaa aagcaggctt aatgaagggt gctgtgctt 49
<210> 16
   <211> 48
   <212> DNA
   <213> Artificial Sequence
<220>
   <223> 3'-Primer für die PCR-Amplification der für Beta vulgaris Tonoplast Zucker Transportprotein 1-kodierenden Nukleotidsequenz
<400> 16
   ggggaccact ttgtacaaga aagctgggta ctccgcctta gcggcttc 48
<210> 17
   <211> 25
   <212> DNA
   <213> Artificial Sequence
<220>
   <223> 5'-Primer für die PCR-Amplification der für Beta vulgaris Tonoplast zucker Transportprotein 2.1-kodierenden Nukleotidsequenz
<400> 17
   ctcgagatga gtgcagcagt attag 25
<210> 18
   <211> 27
   <212> DNA
   <213> Artificial Sequence
<220>
   <223> 3'-Primer für die PCR-Amplification der für Beta vulgaris Tonoplast Zucker Transportprotein 2.1-kodierenden Nukleotidsequenz
<400> 18
   tctagagtgg cttgcttgtc ttgcacc 27
<210> 19
   <211> 23
   <212> DNA
   <213> Artificial Sequence
<220>
   <223> 5'-Primer für die quantitative PCR-Reaktion zur Amplifikation eines Fragments eines Nukleinsäuremoleküls, das für das Beta vulgaris Tonoplastidäres zucker Transportprotein 1 kodiert
<400> 19
   gctgttgcta tgaggctcat gga 23
<210> 20
   <211> 25
   <212> DNA
   <213> Artificial Sequence
<220>
   <223> 3'-Primer für die quantitative PCR-Reaktion zur Amplifikation eines Fragments eines Nukleinsäuremoleküls, das für das Beta vulgaris Tonoplastidäres Zucker Transportprotein 1 kodiert
<400> 20
   ccttagcggc ttctaactgt ttagg 25
<210> 21
   <211> 23
   <212> DNA
   <213> Artificial Sequence
<220>
   <223> 5'-Primer für die quantitative PCR-Reaktion zur Amplifikation eines Fragments eines Nukleinsäuremoleküls, das für das Beta vulgaris Tonoplastidäres Zucker Transportprotein 2.1 kodiert
<400> 21
   aaagatgaac accactgtgt atg 23
<210> 22
   <211> 24
   <212> DNA
   <213> Artificial Sequence
<220>
   <223> 3'-Primer für die quantitative PCR-Reaktion zur Amplifikation eines Fragments eines Nukleinsäuremoleküls, das für das Beta vulgaris Tonoplastidäres Zucker Transportprotein 2.1 kodiert
<400> 22
   gtcatcagtg gcttgcttgt cttg 24
<210> 23
   <211> 23
   <212> DNA
   <213> Artificial Sequence
<220>
   <223> 5'-Primer für die quantitative PCR-Reaktion zur Amplifikation eines Fragments eines Nukleinsäuremoleküls, das für das Beta vulgaris Tonoplastidäres Zucker Transportprotein 2.2 kodiert
<400> 23
   aaagatgagc actactgtgc acg 23
<210> 24
   <211> 24
   <212> DNA
   <213> Artificial Sequence
<220>
   <223> 3'-Primer für die quantitative PCR-Reaktion zur Amplifikation eines Fragments eines Nukleinsäuremoleküls, das für das Beta vulgaris Tonoplastidäres Zucker Transportprotein 2.2 kodiert
<400> 24
   tcagttgtcc ttgtcttcag aagg 24
<210> 25
   <211> 24
   <212> DNA
   <213> Artificial Sequence
<220>
   <223> 5'-Primer für die quantitative PCR-Reaktion zur Amplifikation eines Fragments eines Nukleinsäuremoleküls, das für das Beta vulgaris Tonoplastidäres Zucker Transportprotein 3 kodiert
<400> 25
   tctacttctg ctgctttgtc atgg 24
<210> 26
   <211> 22
   <212> DNA
   <213> Artificial Sequence
<220>
   <223> 3'-Primer für die quantitative PCR-Reaktion zur Amplifikation eines Fragments eines Nukleinsäuremoleküls, das für das Beta vulgaris Tonoplastidäres Zucker Transportprotein 3 kodiert
<400> 26
   tcagcttcag cttgccttgc ac 22
<210> 27
   <211> 23
   <212> DNA
   <213> Artificial Sequence
<220>
   <223> 5'-Primer für die quantitative PCR-Reaktion zur Amplifikation eines Fragments eines Nukleinsäuremoleküls, das für den Beta vulgaris Elongationsfaktor 1 alpha 1 kodiert
<400> 27
   ccacattgct gtcaagtttg ctg 23
<210> 28
   <211> 21
   <212> DNA
   <213> Artificial Sequence
<220>
   <223> 3'-Primer für die quantitative PCR-Reaktion zur Amplifikation eines Fragments eines Nukleinsäuremoleküls, das für den Beta vulgaris Elongationsfaktor 1 alpha 1 kodiert
<400> 28
   tggtaacctt ggcaccggtt g 21